# EUROPEAN PATENT SPECIFICATION

(11) **EP 1 351 961 B1**
(45) Date of publication and mention of the grant of the patent: **27.07.2005**
(21) Application number: 02717304.6
(22) Date of filing: 02.01.2002
(51) Int. Cl.: C07D 487/04, A61K 31/407, A61K 31/55

(54) **SUBSTITUTED INDOLINES AS 5-HT RECEPTOR LIGANDS**
SUBSTITUIERTE INDOLINE ALS 5-HT-REZEPTOR LIGANDEN
INDOLINES SUBSTITUEES UTILES EN TANT QUE LIGANDS DU RECEPTEUR 5-HT

(30) Priority: 08.01.2001 US 260364 P; 02.10.2001 US 326504 P
(43) Date of publication of application: 15.10.2003
(73) Proprietor: Pharmacia & Upjohn Company LLC, Kalamazoo, MI 49001 (US)
(72) Inventor: JACOBSEN, Susan, Fox, Richland, MI 49083 (US); MERCHANT, Kalpana, M., Portage, MI 49024 (US); JACOBSEN, E., Jon, Richland, MI 49083 (US)
(74) Representative: Perry, Robert Edward
(86) International application number: PCT/US2002/000477
(87) International publication number: WO 2002/060903

(56) References cited:
- EP-A- 0 466 548
- WO-A-00/64899
- WO-A-01/05793
- RICHARD A. GLENNON ET AL.: "2-Substituted Tryptamines........." JOURNAL OF MEDICINAL CHEMISTRY., vol. 43, no. 5, 2000, pages 1011-1018, XP002201555 AMERICAN CHEMICAL SOCIETY., US ISSN: 0022-2623

## Description

### FIELD OF THE INVENTION

The present invention relates to novel indoline derivatives, and more specifically, relates to substituted indoline compounds of formula I described herein below. These compounds of the present invention are 5-HT receptor ligands and are useful for treating diseases wherein modulation of 5-HT activity is desired.

### BACKGROUND OF THE INVENTION

Serotonin has been implicated in a number of diseases and conditions that originate in the central nervous system. These include diseases and conditions related to sleeping, eating, perceiving pain, controlling body temperature, controlling blood pressure, depression, anxiety, schizophrenia, and other bodily states. Serotonin also plays an important role in peripheral systems, such as the gastrointestinal system, where it has been found to mediate a variety of contractile, secretory, and electrophysiologic effects.

As a result of the broad distribution of serotonin within the body, there is a tremendous interest in drugs that affect serotonergic systems. In particular, agonists, partial agonists and antagonists are of interest for the treatment of a wide range of disorders, including anxiety; depression, hypertension, migraine, obesity, compulsive disorders, schizophrenia, autism, neurodegenerative disorders (e.g. Alzheimer's disease, Parkinsonism, and Huntington's chorea), and chemotherapy-induced vomiting.

The major classes of serotonin receptors (5-HT₁₋₇) contain fourteen to eighteen separate receptors that have been formally classified. See Glennon, et al., Neuroscience and Behavioral Reviews, 1990, 14, 35; and D. Hoyer, et al. Pharmacol. Rev. 1994, 46, 157-203.

There is currently a need for pharmaceutical agents that are useful to treat diseases and conditions that are associated with 5-HT receptors. In particular, there is a need for agents that can selectively bind to individual receptor sub-types (e.g. receptor-specific agonists or antagonists); such agents would be useful as pharmaceutical agents, or would be useful to facilitate the study of the 5-HT receptor family, or to aid in the identification of other compounds that selectively bind to the specific 5-HT receptors.

For example, The 5-HT₆ receptor was identified in 1993 (Monsma et al. Mol. Pharmacol. 1993, 43, 320-327 and Ruat, M. et al. Biochem. Biophys. Res. Com. 1993, 193, 269-276). Several antidepressants and atypical antipsychotics bind to the 5-HT₆ receptor with high affinity and this binding may be a factor in their profile of activities (Roth et al. J. Pharm. Exp. Therapeut. 1994, 268, 1403-1410; Sleight et al. Exp. Opin. Ther. Patents 1998, 8, 1217-1224; Bourson et al. Brit. J. Pharm. 1998, 125, 1562-1566; Boess et al. Mol. Pharmacol. 1998, 54, 577-583; Sleight et al. Brit. J. Pharmacol. 1998, 124, 556-562). In addition, the 5-HT₆ receptor has been linked to generalized stress and anxiety states (Yoshioka et al. Life Sciences 1998, 17/18, 1473-1477). Together these studies and observations suggest that compounds that antagonize the 5-HT₆ receptor will be useful in treating disorders of the central nervous system.

Generally, compounds of the present invention are 5-HT ligands. In particular, they can selectively bind to the 5-HT₆ receptor (e.g. receptor-specific agonists or antagonists). Thus, they are useful for treating diseases wherein modulation of 5-HT activity, specifically 5-HT₆ activity, is desired. Therefore, the compounds of this invention are useful for the treatment of diseases or disorders of the central nervous system. More specifically, for the treatment of psychosis, paraphrenia, psychotic depression, mania, schizophrenia, schizophreniform disorders, anxiety, migraine headache, drug addiction, convulsive disorders, personality disorders, post-traumatic stress syndrome, alcoholism, panic attacks, obsessive-compulsive disorders, and sleep disorders. The compounds of this invention are also useful to treat psychotic, affective, vegetative, and psychomotor symptoms of schizophrenia and the extrapyramidal motor side effects of other antipsychotic drugs. This last action will allow higher doses of antipsychotics to be used and thus greater antipsychotic efficacy to be obtained as a result of a reduction in side effects. The compounds of this invention are also useful in the modulation of eating behavior and thus are useful in treating excess weight and associated morbidity and mortality.

### DESCRIPTION OF THE STATE OF ART

US Patent 3,652,588 discloses 6-alkyl-1,2,3,4,5,6-hcxahydroazepino[4,5-b]indoles which were useful for tranquilizing and sedating mammals to suppress hunger in mammals. This document discloses that there can be substitution at the 9-position. However, those substituents are limited to hydrogen, alkyl, alkoxy and halogen.

US Patent 3,839,357 discloses 6-benzyl-1,2,3,4,5,6-hexahydroazepino[4,5-b]indoles which were useful for tranquilizing mammals. This document discloses that there can be substitution at the 9-position. However, those substituents are limited to hydrogen, alkyl, alkoxy and halogen.

US Patent 3,676,558 discloses 6-alkyl-1,2,3,4,5,6-hexahydroazepino[4,5-b]indoles which were useful to suppress hunger in mammals. This document discloses that there can be substitution at the 9-position. However, it is limited to hydrogen, alkyl, alkoxy and halogen.

US patent application, Serial No. 09/613843 discloses 1,2,3,4,5,6-hexahydroazepino[4,5-b]indoles containing arylsulfones at the 9-position.

### SUMMARY OF THE INVENTION

The present invention provides a compound of formula I 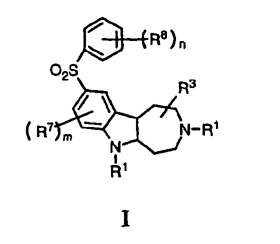 or a pharmaceutically acceptable salt, hydrate,
wherein each R¹ is independently
a) H,
b) C₁₋₄ alkyl,
c) C₁₋₄ alkyl substituted by a phenyl where the phenyl is optionally substituted with one or two R², or
d) phenyl, optionally substituted with one or two R²;
R² is
a) halo,
b) OR³,
c) CF₃
d) C(=O)-NR⁴R⁵,
e) NH-SO₂-R⁶,
f) NR⁴R⁵,
g) NR⁴-C(=O)-R⁴,
h) SO₂-NR⁴R⁵,
i) CN, or
j) NO₂;
R³ is H, C₁₋₄ alkyl, or phenyl;
R⁴ and R⁵ are independently H, C₁₋₄ alkyl, or R⁴ and R⁵ taken together with the attached nitrogen atom to form a ring selected from the group consisting of 1-pyrrolidinyl, 1-piperazinyl and 1-morpholinyl;
R⁶ is H or C₁₋₄ alkyl;
R⁷ is
a) H, or
b) halo,
c) OR³,
d) CF₃
e) C(=O)-NR⁴R⁵,
f) NH-SO₂-R⁶,
g) NR⁴R⁵,
h) NR⁴-C(=O)-R⁴,
i) SO₂-NR⁴R⁵,
j) CN, or
k) NO₂;
R⁸ is
a) H,
b) F,
c) Cl,
d) C₁₋₄ alkyl,
e) C₁₋₃ alkoxy,
f) CF₃,
g) C₁₋₄ alkyl substituted by a phenyl wherein the phenyl is optionally substituted with one or two R²,
h) phenyl, optionally substituted with one or two R²,
i) OR³,
j) CO-NR⁴R⁵,
k) NR⁴R⁵,
l) NH-SO₂-R⁶, or
m) NH-CO-R⁴;
at each occurrence, alkyl and alkoxy is optionally substituted with OH, halo, or NH₂. m is 1 to 2; and n is 1-3.

The compounds of formula I also can include isotopic labels. For example the compounds 6-methyl-9-(phenylsulfonyl)-1,2,3,4,5,5a,6,10b-octahydroazepino[4,5-b]indole and 9-[(4-fluorophenyl)sulfone]-6-methyl-1,2,3,4,5,5a,6,10b-octahydroazepino[4,5-b]indole, and pharmaceutically acceptable salts thereof may contain an isotopic label such as at least on atom selected from Carbon-11, Nitrogen-13, Oxygen-15, and Fluorine-18. Isotopically labeled compounds may be used in positron emission tomography.

In other embodiments, the compound 9-[(4-fluorophenyl)sulfone]-6-methyl-1,2,3,4,5,5a,6,10b-octahydroazepino[4,5-b]indole and pharmaceutically acceptable salts thereof, each containing at least one ¹⁹F atom may be used in nuclear magnetic resonance imaging.

The present invention further provides a pharmaceutical composition comprising a compound of formula I, or a pharmaceutically acceptable salt thereof, and a pharmaceutically acceptable carrier. In preferred embodiments, the composition preferably comprises a therapeutically effective amount of a compound of formula I or a pharmaceutically acceptable salt thereof.

The present invention further provides a method for treating a disease or condition in a mammal wherein a 5-HT₆ receptor is implicated and modulation of a 5-HT₆ function is desired comprising administering to the mammal a therapeutically effective amount of a compound of formula I, or a pharmaceutically acceptable salt thereof.

The present invention further provides a method for treating or preventing diseases or disorders of the central nervous system comprising administering a therapeutically effective amount of a compound of formula I, or a pharmaceutically acceptable salt thereof to the mammal. Diseases or disorders for which a compound of formula I may have activity include, but are not limited to the following: obesity, depression, schizophrenia, schizophreniform disorder, schizoaffective disorder, delusional disorder, a stress related disease (e.g. general anxiety disorder), panic disorder, a phobia, obsessive compulsive disorder, post-traumatic-stress syndrome, immune system depression, a stress induced problem with the urinary, gastrointestinal or cardiovascular system (e.g., stress incontinence), neurodegenerative disorders, autism, chemotherapy-induced vomiting, hypertension, migraine headaches, cluster headaches, sexual dysfunction in a mammal (e.g. a human), addictive disorder and withdrawal syndrome, an adjustment disorder, an age-associated learning and mental disorder, anorexia nervosa, apathy, an attention-deficit disorder due to general medical conditions, attention-deficit hyperactivity disorder, behavioral disturbance (including agitation in conditions associated with diminished cognition (e.g., dementia, mental retardation or delirium), bipolar disorder, bulimia nervosa, chronic fatigue syndrome, conduct disorder, cyclothymic disorder, dysthymic disorder, fibromyalgia and other somatoform disorders, generalized anxiety disorder, an inhalation disorder, an intoxication disorder, movement disorder (e.g., Huntington's disease or Tardive Dyskinesia), oppositional defiant disorder, peripheral neuropathy, post-traumatic stress disorder, premenstrual dysphoric disorder, a psychotic disorder (brief and long duration disorders, psychotic disorder due to medical condition, psychotic disorder NOS), mood disorder (major depressive or bipolar disorder with psychotic features) seasonal affective disorder, a sleep disorder, a specific developmental disorder, agitation disorder, stress incontinence such as urge or mixed incontinence, selective serotonin reuptake inhibition (SSRI) "poop out" syndrome or a Tic disorder (e.g., Tourette's syndrome).

The present invention further provides a method for treating anxiety, depression or stress related disorders comprising administering a therapeutically effective amount of a compound of formula I, or a pharmaceutically acceptable salt thereof to the mammal.

The present invention further provides the use of a compound of formula I or a pharmaceutically acceptable salt thereof to preparea medicament for treating or preventing diseases or disorders of the central nervous system.

The invention may also provide novel intermediates and processes for preparing compounds of formula I.

### DETAILED DESCRIPTION OF THE INVENTION

The compounds of the present invention are generally named according to the IUPAC or CAS nomenclature system. Abbreviations which are well known to one of ordinary skill in the art may be used (e.g. "Ph" for phenyl, 'Me" for methyl, "Et" for ethyl, "h" for hour or hours, "rt" for room temperature {20°-25°C}, and etc.).

The carbon atom content of various hydrocarbon-containing moieties is indicated by a prefix designating the minimum and maximum number of carbon atoms in the moiety, i.e., the prefix Cᵢ₋ⱼ indicates a moiety of the integer 'i" to the integer "j" carbon atoms, inclusive. Thus, for example, C₁₋₇alkyl refers to alkyl of one to seven carbon atoms, inclusive.

Specific and preferred values listed below for radicals, groups, moieties, substituents, or ranges, are for illustration only; they do not exclude other defined values or other values within defined ranges.

The following definitions are used, unless otherwise described.

The term "halo" denotes fluoro (F), chloro (Cl), bromo (Br), or iodo (I).

Alkyl denotes both straight and branched groups; but reference to an individual group or moiety such as "propyl" embraces only the straight chain group or moiety, a branched chain isomer such as "isopropyl" being specifically referred to.

Mammal denotes human and animals.

It is to be understood that the present invention encompasses any racemic, optically-active, polymorphic, tautomeric, or stereoisomeric form, or mixture thereof, of a compound of the invention, which possesses the useful properties described herein.

In cases where compounds are sufficiently basic or acidic to form stable nontoxic acid or base salts, administration of the compounds as salts may be appropriate. Examples of pharmaceutically acceptable salts are organic acid addition salts formed with acids which form a physiological acceptable anion, for example, tosylate, methanesulfonate, acetate, citrate, malonate, tartarate, succinate, benzoate, ascorbate, α-ketoglutarate, maleate, fumarate, benzenesulfonate and α-glycerophosphate. Suitable inorganic salts may also be formed, including hydrobromide, hydrochloride, sulfate, nitrate, bicarbonate, and carbonate salts.

Pharmaceutically acceptable salts may be obtained using standard procedures well known in the art, for example by reacting a sufficiently basic compound such as an amine with a suitable acid affording a physiologically acceptable anion. Alkali metal (for example, sodium, potassium or lithium) or alkaline earth metal (for example calcium) salts of carboxylic acids can also be made.

Prodrugs of the compounds of Formula I are an embodiment of the present invention. The term "prodrug" refers to compounds that are rapidly transformed in vivo to yield the parent compound of the above formula, for example, by hydrolysis in blood. Prodrugs are discussed in T. Higuchi and V. Stella, "Pro-drugs as Novel Delivery Systems," Vol. 14 of the A.C.S. Symposium Series; in Bioreversible Carriers in Drug Design, ed. Edward B. Roche, American Pharmaceutical Association and Pergamon Press, 1987; in Notari, R. E., "Theory and Practice of Prodrug Kinetics," Methods in Enzymology, 112:309-323 (1985); in Bodor, N., "Novel Approaches in Prodrug Design," Drugs of the Future, 6(3):165-182 (1981); and in Bundgaard, H., "Design of Prodrugs: Bioreversible-Derivatives for Various Functional Groups and Chemical Entities," in Design of Prodrugs (H. Bundgaard, ed.), Elsevier, N.Y. (1985) all of which are incorporated herein by reference.

In one embodiment, each R¹ is independently H or C₁₋₄alkyl.

In another embodiment, each R¹ is independently H or methyl.

In one embodiment, R⁷ is hydrogen.

In another embodiment, R⁸ is hydrogen.

In another embodiment R³ is hydrogen.

In another embodiment, R⁸ is F, n is 1 or 2.

In another embodiment, R⁸ is OCH₃.

In another embodiment, R⁸ is CF₃.
Examples of the present invention are
(a) (5aS, 10bS)-6-methyl-9-(phenylsulfonyl)-1,2,3,4,5,5a,6,10b-octahydroazepino[4,5-b]indole hydrochloride
(b) (5aR, 10bR)-6-methyl-9-(phenylsulfonyl)-1,2,3,4,5,5a,6,10b-octahydroazepino[4,5-b]indole hydrochloride
(c) (5aR, 10bS)-6-methyl-9-(phenylsulfonyl)-1,2,3,4,5,5a,6,10b-octahydroazepino[4,5-b]indole hydrochloride
(d) (5aS, 10bR)-6-methyl-9-(phenylsulfonyl)-1,2,3,4,5,5a,6,10b-octahydroazepino[4,5-b]indole hydrochloride
(5aS, 10bS)-9-[(4-fluorophenyl)sulfonyl]-6-methyl-1,2,3,4,5,5a,6,10b-octahydroazepino[4,5-b]indole
(5aS, 10bS)-9-[(3-fluorophenyl)sulfonyl]-6-methyl-1,2,3,4,5,5a,6,10b-octahydroazepino[4,5-b]indole
(5aS, 10bS)-9-[(3,4-difluorophenyl)sulfonyl]-6-methyl-1,2,3,4,5,5a,6,10b-octahydroazepino[4,5-b]indole
(5aS, 10bS)-9-[(3,5-difluorophenyl)sulfonyl]-6-methyl-1,2,3,4,5,5a,6,10b-octahydroazepino[4,5-b]indole
(5aS, 10bS)-6-methyl-9-[(4-methylphenyl)sulfonyl]-1,2,3,4,5,5a,6,10b-octahydroazepino[4,5-b]indole
(5aS, 10bS)-6-methyl-9-[(3-methylphenyl)sulfonyl]-1,2,3,4,5,5a,6,10b-octahydroazepino[4,5-b]indole
(5aS, 10bS)-9-[(4-methoxyphenyl)sulfonyl]-6-methyl-1,2,3,4,5,5a,6,10b-octahydroazepino[4,5-b]indole
(5aS, 10bS)-9-[(3-methoxyphenyl)sulfonyl]-6-methyl-1,2,3,4,5,5a,6,10b-octahydroazepino[4,5-b]indole
(5aS, 10bS)-6-methyl-9-[[(4-trifluoromethyl)phenyl]sulfonyl]-1,2,3,4,5,5a,6,10b-octahydroazepino[4,5-b]indole
(5aS, 10bS)-3,6-dimethyl-9-(phenylsulfonyl)-1,2,3,4,5,5a,6,10b-octahydroazepino[4,5-b]indole
(5aS, 10bS)-9-[(4-fluorophenyl)sulfonyl]-3,6-dimethyl-1,2,3,4,5,5a,6,10b-octahydroazepino[4,5-b]indole
(5aS, 10bS)-9-[(3-fluorophenyl)sulfonyl]-3,6-dimethyl-1,2,3,4,5,5a,6,10b-octahydroazepino[4,5-b]indole
(5aS, 10bS)-9-[(3,4-difluorophenyl)sulfonyl]-3,6-dimethyl-1,2,3,4,5,5a,6,10b-octahydroazepino[4,5-b]indole
(5aS, 10bS)-9-[(3,5-difluorophenyl)sulfonyl]-3,6-dimethyl-1,2,3,4,5,5a,6,10b-octahydroazepino[4,5-b]indole
(5aS, 10bS)-3,6-dimethyl-9-[(4-methylphenyl)sulfonyl]-1,2,3,4,5,5a,6,10b-octahydroazepino[4,5-b]indole
(5aS, 10bS)-3,6-dimethyl-9-[(3-methylphenyl)sulfonyl]-1,2,3,4,5,5a,6,10b-octahydroazepino[4,5-b]indole
(5aS, 10bS)-9-[(4-methoxyphenyl)sulfonyl]-3,6-dimethyl-1,2,3,4,5,5a,6,10b-octahydroazepino[4,5-b]indole
(5aS, 10bS)-9-[(3-methoxyphenyl)sulfonyl]-3,6-dimethyl-1,2,3,4,5,5a,6,10b-octahydroazepino[4,5-b]indole
(5aS, 10bS)-3,6-dimethyl-9-[[(4-trifluoromethyl)phenyl]sulfonyl]-1,2,3,4,5,5a,6,10b-octahydroazepino[4,5-b]indole
(5aS, 10bS)-3-ethyl-6-methyl-9-(phenylsulfonyl)-1,2,3,4,5,5a,6,10b-octahydroazepino[4,5-b]indole
(5aS, 10bS)-3-ethyl-9-[(4-fluorophenyl)sulfonyl]-6-methyl-1,2,3,4,5,5a,6,10b-octahydroazepino[4,5-b]indole
(5aS, 10bS)-3-ethyl-9-[(3-fluorophenyl)sulfonyl]-6-methyl-1,2,3,4,5,5a,6,10b-octahydroazepino[4,5-b]indole
(5aS, 10bS)-3-ethyl-9-[(3,4-difluorophenyl)sulfonyl]-6-methyl-1,2,3,4,5,5a,6,10b-octahydroazepino[4,5-b]indole
(5aS, 10bS)-3-ethyl-9-[(3,5-difluorophenyl)sulfonyl]-6-methyl-1,2,3,4,5,5a,6,10b-octahydroazepino[4,5-b]indole
(5aS, 10bS)-3-ethyl-6-methyl-9-[(4-methylphenyl)sulfonyl]-1,2,3,4,5,5a,6,10b-octahydroazepino[4,5-b]indole
(5aS, 10bS)-3-ethyl-6-methyl-9-[(3-methylphenyl)sulfonyl]-1,2,3,4,5,5a,6,10b-octahydroazepino[4,5-b]indole
(5aS, 10bS)-3-ethyl-9-[(4-methoxyphenyl)sulfonyl]-6-methyl-1,2,3,4,5,5a,6,10b-octahydroazepino[4,5-b]indole
(5aS, 10bS)-3-ethyl-9-[(3-methoxyphenyl)sulfonyl]-6-methyl-1,2,3,4,5,5a,6,10b-octahydroazepino[4,5-b]indole
(5aS, 10bS)-3-ethyl-6-methyl-9-[[(4-trifluoromethyl)phenyl]sulfonyl]-1,2,3,4,5,5a,6,10b-octahydroazepino[4,5-b]indole
(5aS, 10bS)-1,6-dimethyl-9-(phenylsulfonyl)-1,2,3,4,5,5a,6,10b-octahydroazepino[4,5-b]indole
(5aS, 10bS)-2,6-dimethyl-9-(phenylsulfonyl)-1,2,3,4,5,5a,6,10b-octahydroazepino[4,5-b]indole
(5aS, 10bS)-4,6-dimethyl-9-(phenylsulfonyl)-1,2,3,4,5,5a,6,10b-octahydroazepino[4,5-b]indole
(5aS, 10bS)-5,6-dimethyl-9-(phenylsulfonyl)-1,2,3,4,5,5a,6,10b-octahydroazepino[4,5-b]indole
(5aS, 10bS)-9-[(4-fluorophenyl)sulfonyl]-1,6-dimethyl-1,2,3,4,5,5a,6,10b-octahydroazepino[4,5-b]indole
(5aS, 10bS)-9-[(4-fluorophenyl)sulfonyl]-2,6-dimethyl-1,2,3,4,5,5a,6,10b-octahydroazepino[4,5-b]indole
(5aS, 10bS)-9-[(4-fluorophenyl)sulfonyl]-4,6-dimethyl-1,2,3,4,5,5a,6,10b-octahydroazepino[4,5-b]indole
(5aS, 10bS)-9-[(4-fluorophenyl)sulfonyl]-5,6-dimethyl-1,2,3,4,5,5a,6,10b-octahydroazepino[4,5-b]indole
(5aS, 10bS)-9-[(3-fluorophenyl)sulfonyl]-1,6-dimethyl-1,2,3,4,5,5a,6,10b-octahydroazepino[4,5-b]indole
(5aS, 10bS)-9-[(3-fluorophenyl)sulfonyl]-2,6-dimethyl-1,2,3,4,5,5a,6,10b-octahydroazepino[4,5-b]indole
(5aS, 10bS)-9-[(3-fluorophenyl)sulfonyl]-4,6-dimethyl-1,2,3,4,5,5a,6,10b-octahydroazepino[4,5-b]indole
(5aS, 10bS)-9-[(3-fluorophenyl)sulfonyl]-5,6-dimethyl-1,2,3,4,5,5a,6,10b-octahydroazepino[4,5-b]indole
(5aS, 10bS)-1,5,6-trimethyl-9-(phenylsulfonyl)-1,2,3,4,5,5a,6,10b-octahydroazepino[4,5-b]indole
(5aS, 10bS)-2,4,6-trimethyl-9-(phenylsulfonyl)-1,2,3,4,5,5a,6,10b-octahydroazepino[4,5-b]indole
(5aS, 10bS)-9-[(4-fluorophenyl)sulfonyl]-1,5,6-trimethyl-1,2,3,4,5,5a,6,10b-octahydroazepino[4,5-b]indole
(5aS, 10bS)-9-[(4-fluorophenyl)sulfonyl]-2,4,6-trimethyl-1,2,3,4,5,5a,6,10b-octahydroazepino[4,5-b]indole
(5aS, 10bS)-9-[(3-fluorophenyl)sulfonyl]-1,5,6-trimethyl-1,2,3,4,5,5a,6,10b-octahydroazepino[4,5-b]indole
(5aS, 10bS)-9-[(3-fluorophenyl)sulfonyl]-2,4,6-trimethyl-1,2,3,4,5,5a,6,10b-octahydroazepino[4,5-b]indole
(5aS, 10bS)-6-methyl-9-(phenylsulfonyl)-1,2,3,4,5,5a,6,10b-octahydroazepino[4,5-b]indole
(5aS, 10bS)-9-[(4-fluorophenyl)sulfonyl]-1,2,3,4,5,5a,6,10b-octahydroazepino[4,5-b]indole
(5aS, 10bS)-9-[(3-fluorophenyl)sulfonyl]-1,2,3,4,5,5a,6,10b-octahydroazepino[4,5-b]indole
(5aS, 10bS)-9-[(3,4-difluorophenyl)sulfonyl]-1,2,3,4,5,5a,6,10b-octahydroazepino[4,5-b]indole
(5aS, 10bS)-9-[(3,5-difluorophenyl)sulfonyl]-1,2,3,4,5,5a,6,10b-octahydroazepino[4,5-b]indole
(5aS, 10bS)-9-[(4-methylphenyl)sulfonyl]-1,2,3,4,5,5a,6,10b-octahydroazepino[4,5-b]indole
(5aS, 10bS)-9-[(3-methylphenyl)sulfonyl]-1,2,3,4,5,5a,6,10b-octahydroazepino[4,5-b]indole
(5aS, 10bS)-9-[(4-methoxyphenyl)sulfonyl]-1,2,3,4,5,5a,6,10b-octahydroazepino[4,5-b]indole
(5aS, 10bS)-9-[(3-methoxyphenyl)sulfonyl]-1,2,3,4,5,5a,6,10b-octahydroazepino[4,5-b]indole
(5aS, 10bS)-9-[[(4-trifluoromethyl)phenyl]sulfonyl]-1,2,3,4,5,5a,6,10b-octahydroazepino[4,5-b]indole
(5aS, 10bS)-3-methyl-9-(phenylsulfonyl)-1,2,3,4,5,5a,6,10b-octahydroazepino[4,5-b]indole
(5aS, 10bS)-9-[(4-fluorophenyl)sulfonyl]-3-methyl-1,2,3,4,5,5a,6,10b-octahydroazepino[4,5-b]indole
(5aS, 10bS)-9-[(3-fluorophenyl)sulfonyl]-3-methyl-1,2,3,4,5,5a,6,10b-octahydroazepino[4,5-b]indole
(5aS, 10bS)-9-[(3,4-difluorophenyl)sulfonyl]-3-methyl-1,2,3,4,5,5a,6,10b-octahydroazepino[4,5-b]indole
(5aS, 10bS)-9-[(3,5-difluorophenyl)sulfonyl]-3-methyl-1,2,3,4,5,5a,6,10b-octahydroazepino[4,5-b]indole
(5aS, 10bS)-3-methyl-9-[(4-methylphenyl)sulfonyl]-1,2,3,4,5,5a,6,10b-octahydxoazepino[4,5-b]indole
(5aS, 10bS)-3-methyl-9-[(3-methylphenyl)sulfonyl]-1,2,3,4,5,5a,6,10b-octahydroazepino[4,5-b]indole
(5aS, 10bS)-9-[(4-methoxyphenyl)sulfonyl]-3-methyl-1,2,3,4,5,5a,6,10b-octahydroazepino[4,5-b]indole
(5aS, 10bS)-9-[(3-methoxyphenyl)sulfonyl]-3-methyl-1,2,3,4,5,5a,6,10b-octahydroazepino[4,5-b]indole
(5aS, 10bS)-3-methyl-9-[[(4-trifluoromethyl)phenyl]sulfonyl]-1,2,3,4,5,5a,6,10b-octahydroazepino[4,5-b]indole
(5aS, 10bS)-3-ethyl-9-(phenylsulfonyl)-1,2,3,4,5,5a,6,10b-octahydroazepino[4,5-b]indole
(5aS, 10bS)-3-ethyl-9-[(4-fluorophenyl)sulfonyl]-1,2,3,4,5,5a,6,10b-octahydroazepino[4,5-b]indole
(5aS, 10bS)-3-ethyl-9-[(3-fluorophenyl)sulfonyl]-1,2,3,4,5,5a,6,10b-octahydroazepino[4,5-b]indole
(5aS, 10bS)-3-ethyl-9-[(3,4-difluorophenyl)sulfonyl]-1,2,3,4,5,5a,6,10b-octahydroazepino[4,5-b]indole
(5aS, 10bS)-3-ethyl-9-[(3,5-difluorophenyl)sulfonyl]-1,2,3,4,5,5a,6,10b-octahydroazepino[4,5-b]indole
(5aS, 10bS)-3-ethyl-9-[(4-methylphenyl)sulfonyl]-1,2,3,4,5,5a,6,10b-octahydroazepino[4,5-b]indole
(5aS, 10bS)-3-ethyl-9-[(3-methylphenyl)sulfonyl]-1,2,3,4,5,5a,6,10b-octahydroazepino[4,5-b]indole
(5aS, 10bS)-3-ethyl-9-[(4-methoxyphenyl)sulfonyl]-1,2,3,4,5,5a,6,10b-octahydroazepino[4,5-b]indole
(5aS, 10bS)-3-ethyl-9-[(3-methoxyphenyl)sulfonyl]-1,2,3,4,5,5a,6,10b-octahydroazepino[4,5-b]indole
(5aS, 10bS)-3-ethyl-9-[[(4-trifluoromethyl)phenyl]sulfonyl]-1,2,3,4,5,5a,6,10b-octahydroazepino[4,5-b]indole
(5aS, 10bS)-1-methyl-9-(phenylsulfonyl)-1,2,3,4,5,5a,6,10b-octahydroazepino[4,5-b]indole
(5aS, 10bS)-2-methyl-9-(phenylsulfonyl)-1,2,3,4,5,5a,6,10b-octahydroazepino[4,5-b]indole
(5aS, 10bS)-4-methyl-9-(phenylsulfonyl)-1,2,3,4,5,5a,6,10b-octahydroazepino[4,5-b]indole
(5aS, 10bS)-5-methyl-9-(phenylsulfonyl)-1,2,3,4,5,5a,6,10b-octahydroazepino[4,5-b]indole
(5aS, 10bS)-9-[(4-fluorophenyl)sulfonyl]-1-methyl-1,2,3,4,5,5a,6,10b-octahydroazepino[4,5-b]indole
(5aS, 10bS)-9-[(4-fluorophenyl)sulfonyl]-2-methyl-1,2,3,4,5,5a,6,10b-octahydroazepino[4,5-b]indole
(5aS, 10bS)-9-[(4-fluorophenyl)sulfonyl]-4-methyl-1,2,3,4,5,5a,6,10b-octahydroazepino[4,5-b]indole
(5aS, 10bS)-9-[(4-fluorophenyl)sulfonyl]-5-methyl-1,2,3,4,5,5a,6,10b-octahydroazepino[4,5-b]indole
(5aS, 10bS)-9-[(3-fluorophenyl)sulfonyl]-1-methyl-1,2,3,4,5,5a,6,10b-octahydroazepino[4,5-b]indole
(5aS, 10bS)-9-[(3-fluorophenyl)sulfonyl]-2-methyl-1,2,3,4,5,5a,6,10b-octahydroazepino[4,5-b]indole
(5aS, 10bS)-9-[(3-fluorophenyl)sulfonyl]-4-methyl-1,2,3,4,5,5a,6,10b-octahydroazepino[4,5-b]indole
(5aS, 10bS)-9-[(3-fluorophenyl)sulfonyl]-5-methyl-1,2,3,4,5,5a,6,10b-octahydroazepino[4,5-b]indole
(5aS, 10bS)-1,5-dimethyl-9-(phenylsulfonyl)-1,2,3,4,5,5a,6,10b-octahydroazepino[4,5-b]indole
(5aS, 10bS)-2,4-dimethyl-9-(phenylsulfonyl)-1,2,3,4,5,5a,6,10b-octahydroazepino[4,5-b]indole
(5aS, 10bS)-9-[(4-fluorophenyl)sulfonyl]-1,5-dimethyl-1,2,3,4,5,5a,6,10b-octahydroazepino[4,5-b]indole
(5aS, 10bS)-9-[(4-fluorophenyl)sulfonyl]-2,4-dimethyl-1,2,3,4,5,5a,6,10b-octahydroazepino[4,5-b]indole
(5aS, 10bS)-9-[(3-fluorophenyl)sulfonyl]-1,5-dimethyl-1,2,3,4,5,5a,6,10b-octahydroazepino[4,5-b]indole
(5aS, 10bS)-9-[(3-fluorophenyl)sulfonyl]-2,4-dimethyl-1,2,3,4,5,5a,6,10b-octahydroazepino[4,5-b]indole
(5aR, 10bS)-9-[(4-fluorophenyl)sulfonyl]-6-methyl-1,2,3,4,5,5a,6,10b-octahydroazepino[4,5-b]indole
(5aR, 10bS)-9-[(3-fluorophenyl)sulfonyl]-6-methyl-1,2,3,4,5,5a,6,10b-octahydroazepino[4,5-b]indole
(5aR, 10bS)-9-[(3,4-difluorophenyl)sulfonyl]-6-methyl-1,2,3,4,5,5a,6,10b-octahydroazepino[4,5-b]indole
(5aR, 10bS)-9-[(3,5-difluorophenyl)sulfonyl]-6-methyl-1,2,3,4,5,5a,6,10b-octahydroazepino[4,5-b]indole
(5aR, 10bS)-6-methyl-9-[(4-methylphenyl)sulfonyl]-1,2,3,4,5,5a,6,10b-octahydroazepino[4,5-b]indole
(5aR, 10bS)-6-methyl-9-[(3-methylphenyl)sulfonyl]-1,2,3,4,5,5a,6,10b-octahydroazepino[4,5-b]indole
(5aR, 10bS)-9-[(4-methoxyphenyl)sulfonyl]-6-methyl-1,2,3,4,5,5a,6,10b-octahydroazepino[4,5-b]indole
(5aR, 10bS)-9-[(3-methoxyphenyl)sulfonyl]-6-methyl-1,2,3,4,5,5a,6,10b-octahydroazepino[4,5-b]indole
(5aR, 10bS)-6-methyl-9-[[(4-trifluoromethyl)phenyl]sulfonyl]-1,2,3,4,5,5a,6,10b-octahydroazepino[4,5-b]indole
(5aR, 10bS)-3,6-dimethyl-9-(phenylsulfonyl)-1,2,3,4,5,5a,6,10b-octahydroazepino[4,5-b]indole
(5aR, 10bS)-9-[(4-fluorophenyl)sulfonyl]-3,6-dimethyl-1,2,3,4,5,5a,6,10b-octahydroazepino[4,5-b]indole
(5aR, 10bS)-9-[(3-fluorophenyl)sulfonyl]-3,6-dimethyl-1,2,3,4,5,5a,6,10b-octahydroazepino[4,5-b]indole
(5aR, 10bS)-9-[(3,4-difluorophenyl)sulfonyl]-3,6-dimethyl-1,2,3,4,5,5a,6,10b-octahydroazepino[4,5-b]indole
(5aR, 10bS)-9-[(3,5-difluorophenyl)sulfonyl]-3,6-dimethyl-1,2,3,4,5,5a,6,10b-octahydroazepino[4,5-b]indole
(5aR, 10bS)-3,6-dimethyl-9-[(4-methylphenyl)sulfonyl]-1,2,3,4,5,5a,6,10b-octahydroazepino[4,5-b]indole
(5aR, 10bS)-3,6-dimethyl-9-[(3-methylphenyl)sulfonyl]-1,2,3,4,5,5a,6,10b-octahydroazepino[4,5-b]indole
(5aR, 10bS)-9-[(4-methoxyphenyl)sulfonyl]-3,6-dimethyl-1,2,3,4,5,5a,6,10b-octahydroazepino[4,5-b]indole
(5aR, 10bS)-9-[(3-methoxyphenyl)sulfonyl]-3,6-dimethyl-1,2,3,4,5,5a,6,10b-octahydroazepino[4,5-b]indole
(5aR, 10bS)-3,6-dimethyl-9-[[(4-trifluoromethyl)phenyl]sulfonyl]-1,2,3,4,5,5a,6,10b-octahydroazepino[4,5-b]indole
(5aR, 10bS)-3-ethyl-6-methyl-9-(phenylsulfonyl)-1,2,3,4,5,5a,6,10b-octahydroazepino[4,5-b]indole
(5aR, 10bS)-3-ethyl-9-[(4-fluorophenyl)sulfonyl]-6-methyl-1,2,3,4,5,5a,6,10b-octahydroazepino[4,5-b]indole
(5aR, 10bS)-3-ethyl-9-[(3-fluorophenyl)sulfonyl]-6-methyl-1,2,3,4,5,5a,6,10b-octahydroazepino[4,5-b]indole
(5aR, 10bS)-3-ethyl-9-[(3,4-difluorophenyl)sulfonyl]-6-methyl-1,2,3,4,5,5a,6,10b-octahydroazepino[4,5-b]indole
(5aR, 10bS)-3-ethyl-9-[(3,5-difluorophenyl)sulfonyl]-6-methyl-1,2,3,4,5,5a,6,10b-octahydroazepino[4,5-b]indole
(5aR, 10bS)-3-ethyl-6-methyl-9-[(4-methylphenyl)sulfonyl]-1,2,3,4,5,5a,6,10b-octahydroazepino[4,5-b]indole
(5aR, 10bS)-3-ethyl-6-methyl-9-[(3-methylphenyl)sulfonyl]-1,2,3,4,5,5a,6,10b-octahydroazepino[4,5-b]indole
(5aR, 10bS)-3-ethyl-9-[(4-methoxyphenyl)sulfonyl]-6-methyl-1,2,3,4,5,5a,6,10b-octahydroazepino[4,5-b]indole
(5aR, 10bS)-3-ethyl-9-[(3-methoxyphenyl)sulfonyl]-6-methyl-1,2,3,4,5,5a,6,10b-octahydroazepino[4,5-b]indole
(5aR, 10bS)-3-ethyl-6-methyl-9-[[(4-trifluoromethyl)phenyl]sulfonyl]-1,2,3,4,5,5a,6,10b-octahydroazepino[4,5-b]indole
(5aR, 10bS)-1,6-dimethyl-9-(phenylsulfonyl)-1,2,3,4,5,5a,6,10b-octahydroazepino[4,5-b]indole
(5aR, 10bS)-2,6-dimethyl-9-(phenylsulfonyl)-1,2,3,4,5,5a,6,10b-octahydroazepino[4,5-b]indole
(5aR, 10bS)-4,6-dimethyl-9-(phenylsulfonyl)-1,2,3,4,5,5a,6,10b-octahydroazepino[4,5-b]indole
(5aR, 10bS)-5,6-dimethyl-9-(phenylsulfonyl)-1,2,3,4,5,5a,6,10b-octahydroazepino[4,5-b]indole
(5aR, 10bS)-9-[(4-fluorophenyl)sulfonyl]-1,6-dimethyl-1,2,3,4,5,5a,6,10b-octahydroazepino[4,5-b]indole
(5aR, 10bS)-9-[(4-fluorophenyl)sulfonyl]-2,6-dimethyl-1,2,3,4,5,5a,6,10b-octahydroazepino[4,5-b]indole
(5aR, 10bS)-9-[(4-fluorophenyl)sulfonyl]-4,6-dimethyl-1,2,3,4,5,5a,6,10b-octahydroazepino[4,5-b]indole
(5aR, 10bS)-9-[(4-fluorophenyl)sulfonyl]-5,6-dimethyl-1,2,3,4,5,5a,6,10b-octahydroazepino[4,5-b]indole
(5aR, 10bS)-9-[(3-fluorophenyl)sulfonyl]-1,6-dimethyl-1,2,3,4,5,5a,6,10b-octahydroazepino[4,5-b]indole
(5aR, 10bS)-9-[(3-fluorophenyl)sulfonyl]-2,6-dimethyl-1,2,3,4,5,5a,6,10b-octahydroazepino[4,5-b]indole
(5aR, 10bS)-9-[(3-fluorophenyl)sulfonyl]-4,6-dimethyl-1,2,3,4,5,5a,6,10b-octahydroazepino[4,5-b]indole
(5aR, 10bS)-9-[(3-fluorophenyl)sulfonyl]-5,6-dimethyl-1,2,3,4,5,5a,6,10b-octahydroazepino[4,5-b]indole
(5aR, 10bS)-1,5,6-trimethyl-9-(phenylsulfonyl)-1,2,3,4,5,5a,6,10b-octahydroazepino[4,5-b]indole
(5aR, 10bS)-2,4,6-trimethyl-9-(phenylsulfonyl)-1,2,3,4,5,5a,6,10b-octahydroazepino[4,5-b]indole
(5aR, 10bS)-9-[(4-fluorophenyl)sulfonyl]-1,5,6-trimethyl-1,2,3,4,5,5a,6,10b-octahydroazepino[4,5-b]indole
(5aR, 10bS)-9-[(4-fluorophenyl)sulfonyl]-2,4,6-trimethyl-1,2,3,4,5,5a,6,10b-octahydroazepino[4,5-b]indole
(5aR, 10bS)-9-[(3-fluorophenyl)sulfonyl]-1,5,6-trimethyl-1,2,3,4,5,5a,6,10b-octahydroazepino[4,5-b]indole
(5aR, 10bS)-9-[(3-fluorophenyl)sulfonyl]-2,4,6-trimethyl-1,2,3,4,5,5a,6,10b-octahydroazepino[4,5-b]indole
(5aR, 10bS)-6-methyl-9-(phenylsulfonyl)-1,2,3,4,5,5a,6,10b-octahydroazepino[4,5-b]indole
(5aR, 10bS)-9-[(4-fluorophenyl)sulfonyl]-1,2,3,4,5,5a,6,10b-octahydroazepino[4,5-b]indole
(5aR, 10bS)-9-[(3-fluorophenyl)sulfonyl]-1,2,3,4,5,5a,6,10b-octahydroazepino[4,5-b]indole
(5aR, 10bS)-9-[(3,4-difluorophenyl)sulfonyl]-1,2,3,4,5,5a,6,10b-octahydroazepino[4,5-b]indole
(5aR, 10bS)-9-[(3,5-difluorophenyl)sulfonyl]-1,2,3,4,5,5a,6,10b-octahydroazepino[4,5-b]indole
(5aR, 10bS)-9-[(4-methylphenyl)sulfonyl]-1,2,3,4,5,5a,6,10b-octahydroazepino[4,5-b]indole
(5aR, 10bS)-9-[(3-methylphenyl)sulfonyl]-1,2,3,4,5,5a,6,10b-octahydroazepino[4,5-b]indole
(5aR, 10bS)-9-[(4-methoxyphenyl)sulfonyl]-1,2,3,4,5,5a,6,10b-octahydroazepino[4,5-b]indole
(5aR, 10bS)-9-[(3-methoxyphenyl)sulfonyl]-1,2,3,4,5,5a,6,10b-ctahydroazepino[4,5-b]indole
(5aR, 10bS)-9-[[(4-trifluoromethyl)phenyl]sulfonyl]-1,2,3,4,5,5a,6,10b-octahydroazepino[4,5-b]indole
(5aR, 10bS)-3-methyl-9-(phenylsulfonyl)-1,2,3,4,5,5a,6,10b-octahydroazepino[4,5-b]indole
(5aR, 10bS)-9-[(4-fluorophenyl)sulfonyl]-3-methyl-1,2,3,4,5,5a,6,10b-octahydroazepino[4,5-b]indole
(5aR, 10bS)-9-[(3-fluorophenyl)sulfonyl]-3-methyl-1,2,3,4,5,5a,6,10b-octahydroazepino[4,5-b]indole
(5aR, 10bS)-9-[(3,4-difluorophenyl)sulfonyl]-3-methyl-1,2,3,4,5,5a,6,10b-octahydroazepino[4,5-b]indole
(5aR, 10bS)-9-[(3,5-difluorophenyl)sulfonyl]-3-methyl-1,2,3,4,5,5a,6,10b-octahydroazepino[4,5-b]indole
(5aR, 10bS)-3-methyl-9-[(4-methylphenyl)sulfonyl]-1,2,3,4,5,5a,6,10b-octahydroazepino[4,5-b]indole
(5aR, 10bS)-3-methyl-9-[(3-methylphenyl)sulfonyl]-1,2,3,4,5,5a,6,10b-octahydroazepino[4,5-b]indole
(5aR, 10bS)-9-[(4-methoxyphenyl)sulfonyl]-3-methyl-1,2,3,4,5,5a,6,10b-octahydroazepino[4,5-b]indole
(5aR, 10bS)-9-[(3-methoxyphenyl)sulfonyl]-3-methyl-1,2,3,4,5,5a,6,10b-octahydroazepino[4,5-b]indole
(5aR, 10bS)-3-methyl-9-[[(4-trifluoromethyl)phenyl]sulfonyl]-1,2,3,4,5,5a,6,10b-octahydroazepino[4,5-b]indole
(5aR, 10bS)-3-ethyl-9-(phenylsulfonyl)-1,2,3,4,5,5a,6,10b-octahydroazepino[4,5-b]indole
(5aR, 10bS)-3-ethyl-9-[(4-fluorophenyl)sulfonyl]-1,2,3,4,5,5a,6,10b-octahydroazepino[4,5-b]indole
(5aR, 10bS)-3-ethyl-9-[(3-fluorophenyl)sulfonyl]-1,2,3,4,5,5a,6,10b-octahydroazepino[4,5-b]indole
(5aR, 10bS)-3-ethyl-9-[(3,4-difluorophenyl)sulfonyl]-1,2,3,4,5,5a,6,10b-octahydroazepino[4,5-b]indole
(5aR, 10bS)-3-cthyl-9-[(3,5-difluorophenyl)sulfonyl]-1,2,3,4,5,5a,6,10b-octahydroazepino[4,5-b]indole
(5aR, 10bS)-3-ethyl-9-[(4-methylphenyl)sulfonyl]-1,2,3,4,5,5a,6,10b-octahydroazepino[4,5-b]indole
(5aR, 10bS)-3-ethyl-9-[(3-methylphenyl)sulfonyl]-1,2,3,4,5,5a,6,10b-octahydroazepino[4,5-b]indole
(5aR, 10bS)-3-ethyl-9-[(4-methoxyphenyl)sulfonyl]-1,2,3,4,5,5a,6,10b-octahydroazepino[4,5-b]indole
(5aR, 10bS)-3-ethyl-9-[(3-methoxyphenyl)sulfonyl]-1,2,3,4,5,5a,6,10b-octahydroazepino[4,5-b]indole
(5aR, 10bS)-3-ethyl-9-[[(4-trifluoromethyl)phenyl]sulfonyl]-1,2,3,4,5,5a,6,10b-octahydroazepino[4,5-b]indole
(5aR, 10bS)-1-methyl-9-(phenylsulfonyl)-1,2,3,4,5,5a,6,10b-octahydroazepino[4,5-b]indole
(5aR, 10bS)-2-methyl-9-(phenylsulfonyl)-1,2,3,4,5,5a,6,10b-octahydroazepino[4,5-b]indole
(5aR, 10bS)-4-methyl-9-(phenylsulfonyl)-1,2,3,4,5,5a,6,10b-octahydroazepino[4,5-b]indole
(5aR, 10bS)-5-methyl-9-(phenylsulfonyl)-1,2,3,4,5,5a,6,10b-octahydroazepino[4,5-b]indole
(5aR, 10bS)-9-[(4-fluorophenyl)sulfonyl]-1-methyl-1,2,3,4,5,5a,6,10b-octahydroazepino[4,5-b]indole
(5aR, 10bS)-9-[(4-fluorophenyl)sulfonyl]-2-methyl-1,2,3,4,5,5a,6,10b-octahydroazepino[4,5-b]indole
(5aR, 10bS)-9-[(4-fluorophenyl)sulfonyl]-4-methyl-1,2,3,4,5,5a,6,10b-octahydroazepino[4,5-b]indole
(5aR, 10bS)-9-[(4-fluorophenyl)sulfonyl]-5-methyl-1,2,3,4,5,5a,6,10b-octahydroazepino[4,5-b]indole
(5aR, 10bS)-9-[(3-fluomphenyl)sulfonyl]-1-methyl-1,2,3,4,5,5a,6,10b-octahydroazepino[4,5-b]indole
(5aR, 10bS)-9-[(3-fluorophenyl)sulfonyl]-2-methyl-1,2,3,4,5,5a,6,10b-octahydroazepino[4,5-b]indole
(5aR, 10bS)-9-[(3-fluorophenyl)sulfonyl]-4-methyl-1,2,3,4,5,5a,6,10b-octahydroazepino[4,5-b]indole
(5aR, 10bS)-9-[(3-fluorophenyl)sulfonyl]-5-methyl-1,2,3,4,5,5a,6,10b-octahydroazepino[4,5-b]indole
(5aR, 10bS)-1,5-dimethyl-9-(phenylsulfonyl)-1,2,3,4,5,5a,6,10b-octahydroazepino[4,5-b]indole
(5aR, 10bS)-2,4-dimethyl-9-(phenylsulfonyl)-1,2,3,4,5,5a,6,10b-octahydroazepino[4,5-b]indole
(5aR, 10bS)-9-[(4-fluorophenyl)sulfonyl]-1,5-dimethyl-1,2,3,4,5,5a,6,10b-octahydroazepino[4,5-b]indole
(5aR, 10bS)-9-[(4-fluorophenyl)sulfonyl]-2,4-dimethyl-1,2,3,4,5,5a,6,10b-octahydroazepino[4,5-b]indole
(5aR, 10bS)-9-[(3-fluorophenyl)sulfonyl]-1,5-dimethyl-1,2,3,4,5,5a,6,10b-octahydroazepino[4,5-b]indole
(5aR, 10bS)-9-[(3-fluorophenyl)sulfonyl]-2,4-dimethyl-1,2,3,4,5,5a,6,10b-octahydroazepino[4,5-b]indole
(5aS, 10bR)-9-[(4-fluorophenyl)sulfonyl]-6-methyl-1,2,3,4,5,5a,6,10b-octahydroazepino[4,5-b]indole
(5aS, 10bR)-9-[(3-fluorophenyl)sulfonyl]-6-methyl-1,2,3,4,5,5a,6,10b-octahydroazepino[4,5-b]indole
(5aS, 10bR)-9-[(3,4-difluorophenyl)sulfonyl]-6-methyl-1,2,3,4,5,5a,6,10b-octahydroazepino[4,5-b]indole
(5aS, 10bR)-9-[(3,5-difluorophenyl)sulfonyl]-6-methyl-1,2,3,4,5,5a,6,10b-octahydroazepino[4,5-b]indole
(5aS, 10bR)-6-methyl-9-[(4-methylphenyl)sulfonyl]-1,2,3,4,5,5a,6,10b-octahydroazepino[4,5-b]indole
(5aS, 10bR)-6-methyl-9-[(3-methylphenyl)sulfonyl]-1,2,3,4,5,5a,6,10b-octahydroazepino[4,5-b]indole
(5aS, 10bR)-9-[(4-methoxyphenyl)sulfonyl]-6-methyl-1,2,3,4,5,5a,6,10b-octahydroazepino[4,5-b]indole
(5aS, 10bR)-9-[(3-methoxyphenyl)sulfonyl]-6-methyl-1,2,3,4,5,5a,6,10b-octahydroazepino[4,5-b]indole
(5aS, 10bR)-6-methyl-9-[[(4-trifluoromethyl)phenyl]sulfonyl]-1,2,3,4,5,5a,6,10b-octahydroazepino[4,5-b]indole
(5aS, 10bR)-3,6-dimethyl-9-(phenylsulfonyl)-1,2,3,4,5,5a,6,10b-octahydroazepino[4,5-b]indole
(5aS, 10bR)-9-[(4-fluorophenyl)sulfonyl]-3,6-dimethyl-1,2,3,4,5,5a,6,10b-octahydroazepino[4,5-b]indole
(5aS, 10bR)-9-[(3-fluorophenyl)sulfonyl]-3,6-dimethyl-1,2,3,4,5,5a,6,10b-octahydroazepino[4,5-b]indole
(5aS, 10bR)-9-[(3,4-difluorophenyl)sulfonyl]-3,6-dimethyl-1,2,3,4,5,5a,6,10b-octahydroazepino[4,5-b]indole
(5aS, 10bR)-9-[(3,5-difluorophenyl)sulfonyl]-3,6-dimethyl-1,2,3,4,5,5a,6,10b-octahydroazepino[4,5-b]indole
(5aS, 10bR)-3,6-dimethyl-9-[(4-methylphenyl)sulfonyl]-1,2,3,4,5,5a,6,10b-octahydroazepino[4,5-b]indole
(5aS, 10bR)-3,6-dimethyl-9-[(3-methylphenyl)sulfonyl]-1,2,3,4,5,5a,6,10b-octahydroazepino[4,5-b]indole
(5aS, 10bR)-9-[(4-methoxyphenyl)sulfonyl]-3,6-dimethyl-1,2,3,4,5,5a,6,10b-octahydroazepino[4,5-b]indole
(5aS, 10bR)-9-[(3-methoxyphenyl)sulfonyl]-3,6-dimethyl-1,2,3,4,5,5a,6,10b-octahydroazepino[4,5-b]indole
(5aS, 10bR)-3,6-dimethyl-9-[[(4-trifluoromethyl)phenyl]sulfonyl]-1,2,3,4,5,5a,6,10b-octahydroazepino[4,5-b]indole
(5aS, 10bR)-3-ethyl-6-methyl-9-(phenylsulfonyl)-1,2,3,4,5,5a,6,10b-octahydroazepino[4,5-b]indole
(5aS, 10bR)-3-ethyl-9-[(4-fluorophenyl)sulfonyl]-6-methyl-1,2,3,4,5,5a,6,10b-octahydroazepino[4,5-b]indole
(5aS, 10bR)-3-ethyl-9-[(3-fluorophenyl)sulfonyl]-6-methyl-1,2,3,4,5,5a,6,10b-octahydroazepino[4,5-b]indole
(5aS, 10bR)-3-ethyl-9-[(3,4-difluorophenyl)sulfonyl]-6-methyl-1,2,3,4,5,5a,6,10b-octahydroazepino[4,5-b]indole
(5aS, 10bR)-3-ethyl-9-[(3,5-difluorophenyl)sulfonyl]-6-methyl-1,2,3,4,5,5a,6,10b-octahydroazepino[4,5-b]indole
(5aS, 10bR)-3-ethyl-6-methyl-9-[(4-methylphenyl)sulfonyl]-1,2,3,4,5,5a,6,10b-octahydroazepino[4,5-b]indole
(5aS, 10bR)-3-ethyl-6-methyl-9-[(3-methylphenyl)sulfonyl]-1,2,3,4,5,5a,6,10b-octahydroazepino[4,5-b]indole
(5aS, 10bR)-3-ethyl-9-[(4-methoxyphenyl)sulfonyl]-6-methyl-1,2,3,4,5,5a,6,10b-octahydroazepino[4,5-b]indole
(5aS, 10bR)-3-ethyl-9-[(3-methoxyphenyl)sulfonyl]-6-methyl-1,2,3,4,5,5a,6,10b-octahydroazepino[4,5-b]indole
(5aS, 10bR)-3-ethyl-6-methyl-9-[[(4-trifluoromethyl)phenyl]sulfonyl]-1,2,3,4,5,5a,6,10b-octahydroazepino[4,5-b]indole
(5aS, 10bR)-1,6-dimethyl-9-(phenylsulfonyl)-1,2,3,4,5,5a,6,10b-octahydroazepino[4,5-b]indole
(5aS, 10bR)-2,6-dimethyl-9-(phenylsulfonyl)-1,2,3,4,5,5a,6,10b-octahydroazepino[4,5-b]indole
(5aS, 10bR)-4,6-dimethyl-9-(phenylsulfonyl)-1,2,3,4,5,5a,6,10b-octahydroazepino[4,5-b]indole
(5aS, 10bR)-5,6-dimethyl-9-(phenylsulfonyl)-1,2,3,4,5,5a,6,10b-octahydroazepino[4,5-b]indole
(5aS, 10bR)-9-[(4-fluorophenyl)sulfonyl]-1,6-dimethyl-1,2,3,4,5,5a,6,10b-octahydroazepino[4,5-b]indole
(5aS, 10bR)-9-[(4-fluorophenyl)sulfonyl]-2,6-dimethyl-1,2,3,4,5,5a,6,10b-octahydroazepino[4,5-b]indole
(5aS, 10bR)-9-[(4-fluorophenyl)sulfonyl]4,6-dimethyl-1,2,3,4,5,5a,6,10b-octahydroazepino[4,5-b]indole
(5aS, 10bR)-9-[(4-fluorophenyl)sulfonyl]-5,6-dimethyl-1,2,3,4,5,5a,6,10b-octahydroazepino[4,5-b]indole
(5aS, 10bR)-9-[(3-fluorophenyl)sulfonyl]-1,6-dimethyl-1,2,3,4,5,5a,6,10b-octahydroazepino[4,5-b]indole
(5aS, 10bR)-9-[(3-fluorophenyl)sulfonyl]-2,6-dimethyl-1,2,3,4,5,5a,6,10b-octahydroazepino[4,5-b]indole
(5aS, 10bR)-9-[(3-fluorophenyl)sulfonyl]-4,6-dimethyl-1,2,3,4,5,5a,6,10b-octahydroazepino[4,5-b]indole
(5aS, 10bR)-9-[(3-fluorophenyl)sulfonyl]-5,6-dimethyl-1,2,3,4,5,5a,6,10b-octahydroazepino[4,5-b]indole
(5aS, 10bR)-1,5,6-trimethyl-9-(phenylsulfonyl)-1,2,3,4,5,5a,6,10b-octahydroazepino[4,5-b]indole
(5aS, 10bR)-2,4,6-trimethyl-9-(phenylsulfonyl)-1,2,3,4,5,5a,6,10b-octahydroazepino[4,5-b]indole
(5aS, 10bR)-9-[(4-fluorophenyl)sulfonyl]-1,5,6-trimethyl-1,2,3,4,5,5a,6,10b-octahydroazepino[4,5-b]indole
(5aS, 10bR)-9-[(4-fluorophenyl)sulfonyl]-2,4,6-trimethyl-1,2,3,4,5,5a,6,10b-octahydroazepino[4,5-b]indole
(5aS, 10bR)-9-[(3-fluorophenyl)sulfonyl]-1,5,6-trimethyl-1,2,3,4,5,5a,6,10b-octahydroazepino[4,5-b]indole
(5aS, 10bR)-9-[(3-fluorophenyl)sulfonyl]-2,4,6-trimethyl-1,2,3,4,5,5a,6,10b-octahydroazepino[4,5-b]indole
(5aS, 10bR)-6-methyl-9-(phenylsulfonyl)-1,2,3,4,5,5a,6,10b-octahydroazepino[4,5-b]indole
(5aS, 10bR)-9-[(4-fluorophenyl)sulfonyl]-1,2,3,4,5,5a,6,10b-octahydroazepino[4,5-b]indole
(5aS, 10bR)-9-[(3-fluorophenyl)sulfonyl]-1,2,3,4,5,5a,6,10b-octahydroazepino[4,5-b]indole
(5aS, 10bR)-9-[(3,4-difluorophenyl)sulfonyl]-1,2,3,4,5,5a,6,10b-octahydroazepino[4,5-b]indole
(5aS, 10bR)-9-[(3,5-difluorophenyl)sulfonyl]-1,2,3,4,5,5a,6,10b-octahydroazepino[4,5-b]indole
(5aS, 10bR)-9-[(4-methylphenyl)sulfonyl]-1,2,3,4,5,5a,6,10b-octahydroazepino[4,5-b]indole
(5aS, 10bR)-9-[(3-methylphenyl)sulfonyl]-1,2,3,4,5,5a,6,10b-octahydroazepino[4,5-b]indole
(5aS, 10bR)-9-[(4-methoxyphenyl)sulfonyl]-1,2,3,4,5,5a,6,10b-octahydroazepino[4,5-b]indole
(5aS, 10bR)-9-[(3-methoxyphenyl)sulfonyl]-1,2,3,4,5,5a,6,10b-octahydroazepino[4,5-b]indole
(5aS, 10bR)-9-[[(4-trifluoromethyl)phenyl]sulfonyl]-1,2,3,4,5,5a,6,10b-octahydroazepino[4,5-b]indole
(5aS, 10bR)-3-methyl-9-(phenylsulfonyl)-1,2,3,4,5,5a,6,10b-octahydroazepino[4,5-b]indole
(5aS, 10bR)-9-[(4-fluorophenyl)sulfonyl]-3-methyl-1,2,3,4,5,5a,6,10b-octahydroazepino[4,5-b]indole
(5aS, 10bR)-9-[(3-fluorophenyl)sulfonyl]-3-methyl-1,2,3,4,5,5a,6,10b-octahydroazepino[4,5-b]indole
(5aS, 10bR)-9-[(3,4-difluorophenyl)sulfonyl]-3-methyl-1,2,3,4,5,5a,6,10b-octahydroazepino[4,5-b]indole
(5aS, 10bR)-9-[(3,5-difluorophenyl)sulfonyl]-3-methyl-1,2,3,4,5,5a,6,10b-octahydroazepino[4,5-b]indole
(5aS, 10bR)-3-methyl-9-[(4-methylphenyl)sulfonyl]-1,2,3,4,5,5a,6,10b-octahydroazepino[4,5-b]indole
(5aS, 10bR)-3-methyl-9-[(3-methylphenyl)sulfonyl]-1,2,3,4,5,5a,6,10b-octahydroazepino[4,5-b]indole
(5aS, 10bR)-9-[(4-methoxyphenyl)sulfonyl]-3-methyl-1,2,3,4,5,5a,6,10b-octahydroazepino[4,5-b]indole
(5aS, 10bR)-9-[(3-methoxyphenyl)sulfonyl]-3-methyl-1,2,3,4,5,5a,6,10b-octahydroazepino[4,5-b]indole
(5aS, 10bR)-3-methyl-9-[[(4-trifluoromethyl)phenyl]sulfonyl]-1,2,3,4,5,5a,6,10b-octahydroazepino[4,5-b]indole
(5aS, 10bR)-3-ethyl-9-(phenylsulfonyl)-1,2,3,4,5,5a,6,10b-octahydroazepino[4,5-b]indole
(5aS, 10bR)-3-ethyl-9-[(4-fluorophenyl)sulfonyl]-1,2,3,4,5,5a,6,10b-octahydroazepino[4,5-b]indole
(5aS, 10bR)-3-ethyl-9-[(3-fluorophenyl)sulfonyl]-1,2,3,4,5,5a,6,10b-octahydroazepino[4,5-b]indole
(5aS, 10bR)-3-ethyl-9-[(3,4-difluorophenyl)sulfonyl]-1,2,3,4,5,5a,6,10b-octahydroazepino[4,5-b]indole
(5aS, 10bR)-3-ethyl-9-[(3,5-difluorophenyl)sulfonyl]-1,2,3,4,5,5a,6,10b-octahydroazepino[4,5-b]indole
(5aS, 10bR)-3-ethyl-9-[(4-methylphenyl)sulfonyl]-1,2,3,4,5,5a,6,10b-octahydroazepino[4,5-b]indole
(5aS, 10bR)-3-ethyl-9-[(3-methylphenyl)sulfonyl]-1,2,3,4,5,5a,6,10b-octahydroazepino[4,5-b]indole
(5aS, 10bR)-3-ethyl-9-[(4-methoxyphenyl)sulfonyl]-1,2,3,4,5,5a,6,10b-octahydroazepino[4,5-b]indole
(5aS, 10bR)-3-ethyl-9-[(3-methoxyphenyl)sulfonyl]-1,2,3,4,5,5a,6,10b-octahydroazepino[4,5-b]indole
(5aS, 10bR)-3-ethyl-9-[[(4-trifluoromethyl)phenyl]sulfonyl]-1,2,3,4,5,5a,6,10b-octahydroazepino[4,5-b]indole
(5aS, 10bR)-1-methyl-9-(phenylsulfonyl)-1,2,3,4,5,5a,6,10b-octahydroazepino[4,5-b]indole
(5aS, 10bR)-2-methyl-9-(phenylsulfonyl)-1,2,3,4,5,5a,6,10b-octahydroazepino[4,5-b]indole
(5as, 10bR)-4-methyl-9-(phenylsulfonyl)-1,2,3,4,5,5a,6,10b-octahydroazepino[4,5-b]indole
(5aS, 10bR)-5-methyl-9-(phenylsulfonyl)-1,2,3,4,5,5a,6,10b-octahydroazepino[4,5-b]indole
(5aS, 10bR)-9-[(4-fluorophenyl)sulfonyl]-1-methyl-1,2,3,4,5,5a,6,10b-octahydroazepino[4,5-b]indole
(5aS, 10bR)-9-[(4-fluorophenyl)sulfonyl]-2-methyl-1,2,3,4,5,5a,6,10b-octahydroazepino[4,5-b]indole
(5aS, 10bR)-9-[(4-fluorophenyl)sulfonyl]-4-methyl-1,2,3,4,5,5a,6,10b-octahydroazepino[4,5-b]indole
(5aS, 10bR)-9-[(4-fluorophenyl)sulfonyl]-5-methyl-1,2,3,4,5,5a,6,10b-octahydroazepino[4,5-b]indole
(5aS, 10bR)-9-[(3-fluorophenyl)sulfonyl]-1-methyl-1,2,3,4,5,5a,6,10b-octahydroazepino[4,5-b]indole
(5aS, 10bR)-9-[(3-fluorophenyl)sulfonyl]-2-methyl-1,2,3,4,5,5a,6,10b-octahydroazepino[4,5-b]indole
(5aS, 10bR)-9-[(3-fluorophenyl)sulfonyl]-4-methyl-1,2,3,4,5,5a,6,10b-octahydroazepino[4,5-b]indole
(5aS, 10bR)-9-[(3-fluorophenyl)sulfonyl]-5-methyl-1,2,3,4,5,5a,6,10b-octahydroazepino[4,5-b]indole
(5aS, 10bR)-1,5-dimethyl-9-(phenylsulfonyl)-1,2,3,4,5,5a,6,10b-octahydroazepino[4,5-b]indole
(5aS, 10bR)-2,4-dimethyl-9-(phenylsulfonyl)-1,2,3,4,5,5a,6,10b-octahydroazepino[4,5-b]indole
(5aS, 10bR)-9-[(4-fluorophenyl)sulfonyl]-1,5-dimethyl-1,2,3,4,5,5a,6,10b-octahydroazepino[4,5-b]indole
(5aS, 10bR)-9-[(4-fluorophenyl)sulfonyl]-2,4-dimethyl-1,2,3,4,5,5a,6,10b-octahydroazepino[4,5-b]indole
(5aS, 10bR)-9-[(3-fluorophenyl)sulfonyl]-1,5-dimethyl-1,2,3,4,5,5a,6,10b-octahydroazepino[4,5-b]indole
(5aS, 10bR)-9-[(3-fluorophenyl)sulfonyl]-2,4-dimethyl-1,2,3,4,5,5a,6,10b-octahydroazepino[4,5-b]indole
(5aR, 10bR)-9-[(4-fluorophenyl)sulfonyl]-6-methyl-1,2,3,4,5,5a,6,10b-octahydroazepino[4,5-b]indole
(5aR, 10bR)-9-[(3-fluorophenyl)sulfonyl]-6-methyl-1,2,3,4,5,5a,6,10b-octahydroazepino[4,5-b]indole
(5aR, 10bR)-9-[(3,4-difluorophenyl)sulfonyl]-6-methyl-1,2,3,4,5,5a,6,10b-octahydroazepino[4,5-b]indole
(5aR, 10bR)-9-[(3,5-difluorophenyl)sulfonyl]-6-methyl-1,2,3,4,5,5a,6,10b-octahydroazepino[4,5-b]indole
(5aR, 10bR)-6-methyl-9-[(4-methylphenyl)sulfonyl]-1,2,3,4,5,5a,6,10b-octahydroazepino[4,5-b]indole
(5aR, 10bR)-6-methyl-9-[(3-methylphenyl)sulfonyl]-1,2,3,4,5,5a,6,10b-octahydroazepino[4,5-b]indole
(5aR, 10bR)-9-[(4-methoxyphenyl)sulfonyl]-6-methyl-1,2,3,4,5,5a,6,10b-octahydroazepino[4,5-b]indole
(5aR, 10bR)-9-[(3-methoxyphenyl)sulfonyl]-6-methyl-1,2,3,4,5,5a,6,10b-octahydroazepino[4,5-b]indole
(5aR, 10bR)-6-methyl-9-[[(4-trifluoromethyl)phenyl]sulfonyl]-1,2,3,4,5,5a,6,10b-octahydroazepino[4,5-b]indole
(5aR, 10bR)-3,6-dimethyl-9-(phenylsulfonyl)-1,2,3,4,5,5a,6,10b-octahydroazepino[4,5-b]indole
(5aR, 10bR)-9-[(4-fluorophenyl)sulfonyl]-3,6-dimethyl-1,2,3,4,5,5a,6,10b-octahydroazepino[4,5-b]indole
(5aR, 10bR)-9-[(3-fluorophenyl)sulfonyl]-3,6-dimethyl-1,2,3,4,5,5a,6,10b-octahydroazepino[4,5-b]indole
(5aR, 10bR)-9-[(3,4-difluorophenyl)sulfonyl]-3,6-dimethyl-1,2,3,4,5,5a,6,10b-octahydroazepino[4,5-b]indole
(5aR, 10bR)-9-[(3,5-difluorophenyl)sulfonyl]-3,6-dimethyl-1,2,3,4,5,5a,6,10b-octahydroazepino[4,5-b]indole
(5aR, 10bR)-3,6-dimethyl-9-[(4-methylphenyl)sulfonyl]-1,2,3,4,5,5a,6,10b-octahydroazepino[4,5-b]indole
(5aR, 10bR)-3,6-dimethyl-9-[(3-methylphenyl)sulfonyl]-1,2,3,4,5,5a,6,10b-octahydroazepino[4,5-b]indole
(5aR, 10bR)-9-[(4-methoxyphenyl)sulfonyl]-3,6-dimethyl-1,2,3,4,5,5a,6,10b-octahydroazepino[4,5-b]indole
(5aR, 10bR)-9-[(3-methoxyphenyl)sulfonyl]-3,6-dimethyl-1,2,3,4,5,5a,6,10b-octahydroazepino[4,5-b]indole
(5aR, 10bR)-3,6-dimethyl-9-[[(4-trifluoromethyl)phenyl]sulfonyl]-1,2,3,4,5,5a,6,10b-octahydroazepino[4,5-b]indole
(5aR, 10bR)-3-ethyl-6-methyl-9-(phenylsulfonyl)-1,2,3,4,5,5a,6,10b-octahydroazepino[4,5-b]indole
(5aR, 10bR)-3-ethyl-9-[(4-fluorophenyl)sulfonyl]-6-methyl-1,2,3,4,5,5a,6,10b-octahydroazepino[4,5-b]indole
(5aR, 10bR)-3-ethyl-9-[(3-fluorophenyl)sulfonyl]-6-methyl-1,2,3,4,5,5a,6,10b-octahydroazepino[4,5-b]indole
(5aR, 10bR)-3-ethyl-9-[(3,4-difluorophenyl)sulfonyl]-6-methyl-1,2,3,4,5,5a,6,10b-octahydroazepino[4,5-b]indole
(5aR, 10bR)-3-ethyl-9-[(3,5-difluorophenyl)sulfonyl]-6-methyl-1,2,3,4,5,5a,6,10b-octahydroazepino[4,5-b]indole
(5aR, 10bR)-3-ethyl-6-methyl-9-[(4-methylphenyl)sulfonyl]-1,2,3,4,5,5a,6,10b-octahydroazepino[4,5-b]indole
(5aR, 10bR)-3-ethyl-6-methyl-9-[(3-methylphenyl)sulfonyl]-1,2,3,4,5,5a,6,10b-octahydroazepino[4,5-b]indole
(5aR, 10bR)-3-ethyl-9-[(4-methoxyphenyl)sulfonyl]-6-methyl-1,2,3,4,5,5a,6,10b-octahydroazepino[4,5-b]indole
(5aR, 10bR)-3-ethyl-9-[(3-methoxyphenyl)sulfonyl]-6-methyl-1,2,3,4,5,5a,6,10b-octahydroazepino[4,5-b]indole
(5aR, 10bR)-3-ethyl-6-methyl-9-[[(4-trifluoromethyl)phenyl]sulfonyl]-1,2,3,4,5,5a,6,10b-octahydroazepino[4,5-b]indole
(5aR, 10bR)-1,6-dimethyl-9-(phenylsulfonyl)-1,2,3,4,5,5a,6,10b-octahydroazepino[4,5-b]indole
(5aR, 10bR)-2,6-dimethyl-9-(phenylsulfonyl)-1,2,3,4,5,5a,6,10b-octahydroazepino[4,5-b]indole
(5aR, 10bR)-4,6-dimethyl-9-(phenylsulfonyl)-1,2,3,4,5,5a,6,10b-octahydroazepino[4,5-b]indole
(5aR, 10bR)-5,6-dimethyl-9-(phenylsulfonyl)-1,2,3,4,5,5a,6,10b-octahydroazepino[4,5-b]indole
(5aR, 10bR)-9-[(4-fluorophenyl)sulfonyl]-1,6-dimethyl-1,2,3,4,5,5a,6,10b-octahydroazepino[4,5-b]indole
(5aR, 10bR)-9-[(4-fluorophenyl)sulfonyl]-2,6-dimethyl-1,2,3,4,5,5a,6,10b-octahydroazepino[4,5-b]indole
(5aR, 10bR)-9-[(4-fluorophenyl)sulfonyl]-4,6-dimethyl-1,2,3,4,5,5a,6,10b-octahydroazepino[4,5-b]indole
(5aR, 10bR)-9-[(4-fluorophenyl)sulfonyl]-5,6-dimethyl-1,2,3,4,5,5a,6,10b-octahydroazepino[4,5-b]indole
(5aR, 10bR)-9-[(3-fluorophenyl)sulfonyl]-1,6-dimethyl-1,2,3,4,5,5a,6,10b-octahydroazepino[4,5-b]indole
(5aR, 10bR)-9-[(3-fluorophenyl)sulfonyl]-2,6-dimethyl-1,2,3,4,5,5a,6,10b-octahydroazepino[4,5-b]indole
(5aR, 10bR)-9-[(3-fluorophenyl)sulfonyl]-4,6-dimethyl-1,2,3,4,5,5a,6,10b-octahydroazepino[4,5-b]indole
(5aR, 10bR)-9-[(3-fluorophenyl)sulfonyl]-5,6-dimethyl-1,2,3,4,5,5a,6,10b-octahydroazepino[4,5-b]indole
(5aR, 10bR)-1,5,6-trimethyl-9-(phenylsulfonyl)-1,2,3,4,5,5a,6,10b-octahydroazepino[4,5-b]indole
(5aR, 10bR)-2,4,6-trimethyl-9-(phenylsulfonyl)-1,2,3,4,5,5a,6,10b-octahydroazepino[4,5-b]indole
(5aR, 10bR)-9-[(4-fluorophenyl)sulfonyl]-1,5,6-trimethyl-1,2,3,4,5,5a,6,10b-octahydroazepino[4,5-b]indole
(5aR, 10bR)-9-[(4-fluorophenyl)sulfonyl]-2,4,6-trimethyl-1,2,3,4,5,5a,6,10b-octahydroazepino[4,5-b]indole
(5aR, 10bR)-9-[(3-fluorophenyl)sulfonyl]-1,5,6-trimethyl-1,2,3,4,5,5a,6,10b-octahydroazepino[4,5-b]indole
(5aR, 10bR)-9-[(3-fluorophenyl)sulfonyl]-2,4,6-trimethyl-1,2,3,4,5,5a,6,10b-octahydroazepino[4,5-b]indole
(5aR, 10bR)-6-methyl-9-(phenylsulfonyl)-1,2,3,4,5,5a,6,10b-octahydroazepino[4,5-b]indole
(5aR, 10bR)-9-[(4-fluorophenyl)sulfonyl]-1,2,3,4,5,5a,6,10b-octahydroazepino[4,5-b]indole
(5aR, 10bR)-9-[(3-fluorophenyl)sulfonyl]-1,2,3,4,5,5a,6,10b-octahydroazepino[4,5-b]indole
(5aR, 10bR)-9-[(3,4-difluorophenyl)sulfonyl]-1,2,3,4,5,5a,6,10b-octahydroazepino[4,5-b]indole
(5aR, 10bR)-9-[(3,5-difluorophenyl)sulfonyl]-1,2,3,4,5,5a,6,10b-octahydroazepino[4,5-b]indole
(5aR, 10bR)-9-[(4-methylphenyl)sulfonyl]-1,2,3,4,5,5a,6,10b-octahydroazepino[4,5-b]indole
(5aR, 10bR)-9-[(3-methylphenyl)sulfonyl]-1,2,3,4,5,5a,6,10b-octahydroazepino[4,5-b]indole
(5aR, 10bR)-9-[(4-methoxyphenyl)sulfonyl]-1,2,3,4,5,5a,6,10b-octahydroazepino[4,5-b]indole
(5aR, 10bR)-9-[(3-methoxyphenyl)sulfonyl]-1,2,3,4,5,5a,6,10b-octahydroazepino[4,5-b]indole
(5aR, 10bR)-9-[[(4-trifluoromethyl)phenyl]sulfonyl]-1,2,3,4,5,5a,6,10b-octahydroazepino[4,5-b]indole
(5aR, 10bR)-3-methyl-9-(phenylsulfonyl)-1,2,3,4,5,5a,6,10b-octahydroazepino[4,5-b]indole
(5aR, 10bR)-9-[(4-fluorophenyl)sulfonyl]-3-methyl-1,2,3,4,5,5a,6,10b-octahydroazepino[4,5-b]indole
(5aR, 10bR)-9-[(3-fluorophenyl)sulfonyl]-3-methyl-1,2,3,4,5,5a,6,10b-octahydroazepino[4,5-b]indole
(5aR, 10bR)-9-[(3,4-difluorophenyl)sulfonyl]-3-methyl-1,2,3,4,5,5a,6,10b-octahydroazepino[4,5-b]indole
(5aR, 10bR)-9-[(3,5-difluorophenyl)sulfonyl]-3-methyl-1,2,3,4,5,5a,6,10b-octahydroazepino[4,5-b]indole
(5aR, 10bR)-3-methyl-9-[(4-methylphenyl)sulfonyl]-1,2,3,4,5,5a,6,10b-octahydroazepino[4,5-b]indole
(5aR, 10bR)-3-methyl-9-[(3-methylphenyl)sulfonyl]-1,2,3,4,5,5a,6,10b-octahydroazepino[4,5-b]indole
(5aR, 10bR)-9-[(4-methoxyphenyl)sulfonyl]-3-methyl-1,2,3,4,5,5a,6,10b-octahydroazepino[4,5-b]indole
(5aR, 10bR)-9-[(3-methoxyphenyl)sulfonyl]-3-methyl-1,2,3,4,5,5a,6,10b-octahydroazepino[4,5-b]indole
(5aR, 10bR)-3-methyl-9-[[(4-trifluoromethyl)phenyl]sulfonyl]-1,2,3,4,5,5a,6,10b-octahydroazepino[4,5-b]indole
(5aR, 10bR)-3-ethyl-9-(phenylsulfonyl)-1,2,3,4,5,5a,6,10b-octahydroazepino[4,5-b]indole
(5aR, 10bR)-3-ethyl-9-[(4-fluorophenyl)sulfonyl]-1,2,3,4,5,5a,6,10b-octahydroazepino[4,5-b]indole
(5aR, 10bR)-3-ethyl-9-[(3-fluorophenyl)sulfonyl]-1,2,3,4,5,5a,6,10b-octahydroazepino[4,5-b]indole
(5aR, 10bR)-3-ethyl-9-[(3,4-difluorophenyl)sulfonyl]-1,2,3,4,5,5a,6,10b-octahydroazepino[4,5-b]indole
(5aR, 10bR)-3-ethyl-9-[(3,5-difluorophenyl)sulfonyl]-1,2,3,4,5,5a,6,10b-octahydroazepino[4,5-b]indole
(5aR, 10bR)-3-ethyl-9-[(4-methylphenyl)sulfonyl]-1,2,3,4,5,5a,6,10b-octahydroazepino[4,5-b]indole
(5aR, 10bR)-3-ethyl-9-[(3-methylphenyl)sulfonyl]-1,2,3,4,5,5a,6,10b-octahydroazepino[4,5-b]indole
(5aR, 10bR)-3-ethyl-9-[(4-methoxyphenyl)sulfonyl]-1,2,3,4,5,5a,6,10b-octahydroazepino[4,5-b]indole
(5aR, 10bR)-3-ethyl-9-[(3-methoxyphenyl)sulfonyl]-1,2,3,4,5,5a,6,10b-octahydroazepino[4,5-b]indole
(5aR, 10bR)-3-ethyl-9-[[(4-trifluoromethyl)phenyl]sulfonyl]-1,2,3,4,5,5a,6,10b-octahydroazepino[4,5-b]indole
(5aR, 10bR)-1-methyl-9-(phenylsulfonyl)-1,2,3,4,5,5a,6,10b-octahydroazepino[4,5-b]indole
(5aR, 10bR)-2-methyl-9-(phenylsulfonyl)-1,2,3,4,5,5a,6,10b-octahydroazepino[4,5-b]indole
(5aR, 10bR)-4-methyl-9-(phenylsulfonyl)-1,2,3,4,5,5a,6,10b-octahydroazepino[4,5-b]indole
(5aR, 10bR)-5-methyl-9-(phenylsulfonyl)-1,2,3,4,5,5a,6,10b-octahydroazepino[4,5-b]indole
(5aR, 10bR)-9-[(4-fluorophenyl)sulfonyl]-1-methyl-1,2,3,4,5,5a,6,10b-octahydroazepino[4,5-b]indole
(5aR, 10bR)-9-[(4-fluorophenyl)sulfonyl]-2-methyl-1,2,3,4,5,5a,6,10b-octahydroazepino[4,5-b]indole
(5aR, 10bR)-9-[(4-fluorophenyl)sulfonyl]-4-methyl-1,2,3,4,5,5a,6,10b-octahydroazepino[4,5-b]indole
(5aR, 10bR)-9-[(4-fluorophenyl)sulfonyl]-5-methyl-1,2,3,4,5,5a,6,10b-octahydroazepino[4,5-b]indole
(5aR, 10bR)-9-[(3-fluorophenyl)sulfonyl]-1-methyl-1,2,3,4,5,5a,6,10b-octahydroazepino[4,5-b]indole
(5aR, 10bR)-9-[(3-fluorophenyl)sulfonyl]-2-methyl-1,2,3,4,5,5a,6,10b-octahydroazepino[4,5-b]indole
(5aR, 10bR)-9-[(3-fluorophenyl)sulfonyl]-4-methyl-1,2,3,4,5,5a,6,10b-octahydroazepino[4,5-b]indole
(5aR, 10bR)-9-[(3-fluorophenyl)sulfonyl]-5-methyl-1,2,3,4,5,5a,6,10b-octahydroazepino[4,5-b]indole
(5aR, 10bR)-1,5-dimethyl-9-(phenylsulfonyl)-1,2,3,4,5,5a,6,10b-octahydroazepino[4,5-b]indole
(5aR, 10bR)-2,4-dimethyl-9-(phenylsulfonyl)-1,2,3,4,5,5a,6,10b-octahydroazepino[4,5-b]indole
(5aR, 10bR)-9-[(4-fluorophenyl)sulfonyl]-1,5-dimethyl-1,2,3,4,5,5a,6,10b-octahydroazepino[4,5-b]indole
(5aR, 10bR)-9-[(4-fluorophenyl)sulfonyl]-2,4-dimethyl-1,2,3,4,5,5a,6,10b-octahydroazepino[4,5-b]indole
(5aR, 10bR)-9-[(3-fluorophenyl)sulfonyl]-1,5-dimethyl-1,2,3,4,5,5a,6,10b-octahydroazepino[4,5-b]indole
(5aR, 10bR)-9-[(3-fluorophenyl)sulfonyl]-2,4-dimethyl-1,2,3,4,5,5a,6,10b-octahydroazepino[4,5-b]indole

Compounds of the present invention can be prepared according to Scheme I as described below.

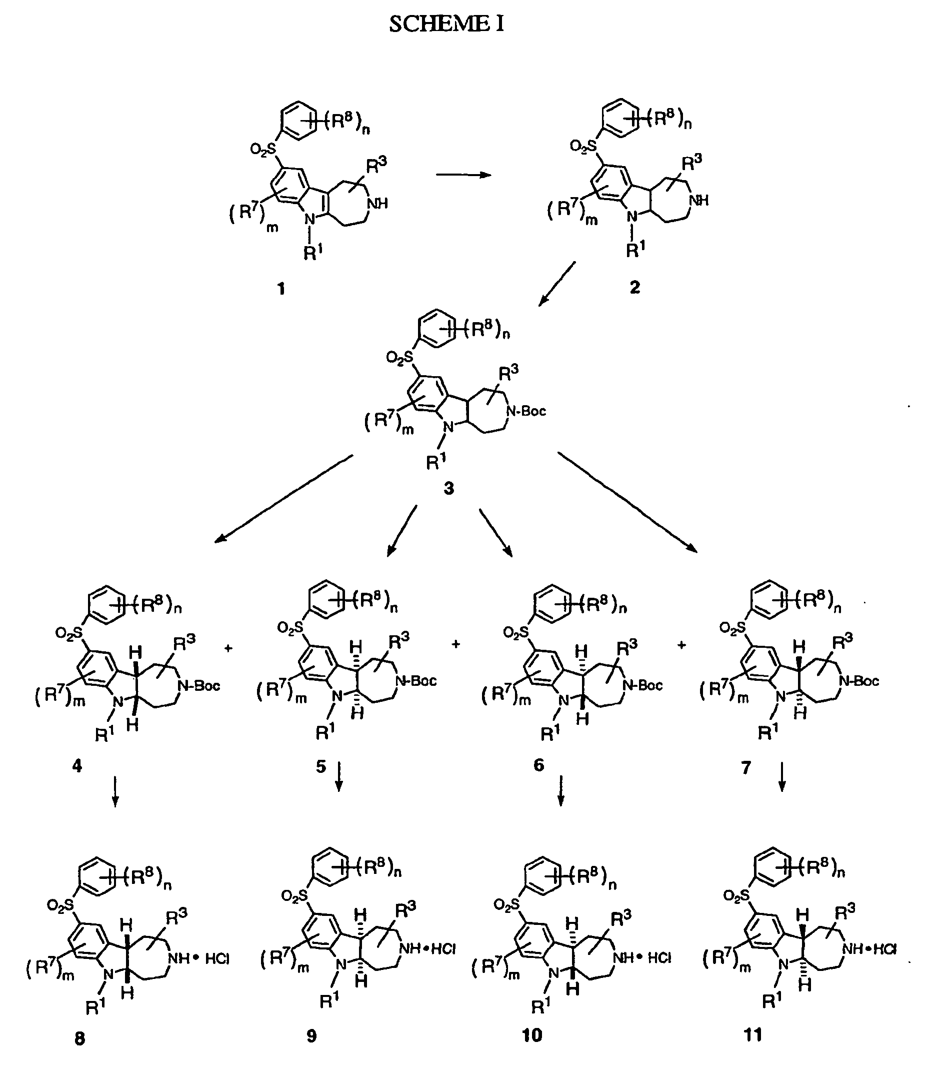

In Scheme I, the starting material 1 can be prepared according to the procedures described in US patent application, Serial No. 09/613843, entitled as 1,2,3,4,5,6-hexahydroazepino[4,5-b]indoles containing arylsulfones at the 9-position, incorporated herein by reference. The *cis-* and *trans*-indolines can be prepared stereoselectively. To prepare *cis*-indolines 4 and 5 the following method is used. Treatment of compound 1 with a reducing agent such as sodium cyanoborohydride in the presence of trifluoroacetic acid (TFA) in a solvent such as methanol at 0 °C provides reduced *cis*-indoline 2. Protection of this crude mixture as the *tert*-butyl carbamate (3) can be carried out as using di-*tert*-butyl dicarbonate in a solvent such as CH₂Cl₂ at ambient temperature. The enantiomers can be separated by chiral (Chiracel OD-H) HPLC to give the individual *cis*-enantiomers 4 and 5. Deprotection using TFA/CH₂Cl₂ or HCl/MeOH provides the individual enantiomers 8 and 9, respectively. Compounds 8 or 9 can be further alkylated by the procedures well known to one skilled in the art.

Alternatively, the *trans*-indolines (6 and 7) can be prepared as follows. Protection of 1 as the *tert*-butyl carbamate was carried out as described above. The crude mixture is then reduced to the corresponding *trans*-indoline 2 in the presence of a reducing agent such as BH₃ and an appropriate solvent such as THF at a temperature about 0°C to provide a compound of structure 2. The nitrogen on the azepine ring can be protected by an appropriate protecting group such as di-*tert*-butyl dicarbonate to provide structure 3. The *trans*-racemic compound 3 can be separated on a Chiral HPLC (Chiracel OD-H) to provide individual enantiomers 6 and 7. Deprotection of structures 6 or 7 using HCl/MeOH or TFA/CH₂Cl₂ provides compounds 10 and 11, respectively. Compounds 10 or 11 can be further alkylated by the procedures well know to one skilled in the art.

The invention also includes isotopically-labeled compounds, which are identical to those recited in Formula I, but for the fact that one or more atoms are replaced by an atom having an atomic mass or mass number different from the atomic mass or mass number usually found in nature. Examples of isotopes that can be incorporated into compounds of the invention include isotopes of hydrogen, carbon, nitrogen, oxygen, phosphorous, fluorine, iodine, and chlorine, such as ³H, ¹¹C, ¹⁴C, ¹³N, ¹⁵O, ¹⁸F, ^{99m}Tc, ¹²³I, and ¹²⁵I. Atoms that are not normally part of the molecule of Formula I, such as F, CL, I, Tc, and P may be added as substituents in a manner known to those skilled in the art. Compounds of the present invention and pharmaceutically acceptable salts and prodrugs of said compounds that contain the aforementioned isotopes and/or other isotopes of other atoms are within the scope of the invention. Isotopically labeled compounds of the present invention are useful in drug and/or substrate tissue distribution and target occupancy assays. For example, isotopically labeled compounds are particularly useful in SPECT (single photon emission computed tomography) and in PET (positron emission tomography).

Single-photon emission computed tomography (SPECT), acquires information on the concentration of isotopically labeled compounds introduced to a mammal's body. SPECT dates from the early 1960's, when the idea of emission traverse section tomography was introduced by D.E. Kuhl and R.Q. Edwards prior to either PET, x-ray CT, or MRI. In general, SPECT requires isotopes that decay by electron capture and/or gamma emission. Example of viable SPECT isotopes include, but are not limited to, 123-iodine (¹²³I) and 99m-technetium (^{99m}Tc). Subjects are injected with a radioactively labeled agent, typically at tracer doses. The nuclear decay resulting in the emission of a single gamma ray, which passes through the tissue and is measured externally with a SPECT camera. The uptake of radioactivity reconstructed by computers as a tomogram shows tissue distribution in cross-sectional images.

Positron emission tomography (PET) is a technique for measuring the concentrations of positron-emitting isotopes within the tissues. Like SPECT, these measurements are, typically, made using PET cameras outside of the living subjects. PET can be broken down into several steps including, but not limited to, synthesizing a compound to include a positron-emitting isotope; administering the isotopically labeled compound to a mammal; and imaging the distribution of the positron activity as a function of time by emission tomography. PET is described, for example, by Alavi et al. in Positron Emission Tomography. published by Alan R. Liss, Inc. in 1985.

Positron-emitting isotopes used in PET include, but are not limited to, Carbon-11, Nitrogen-13, Oxygen-15, and Fluorine-18. In general, positron-emitting isotopes should have short half-lives to help minimize the long-term radiation exposure that a patient receives from high dosages required during PET imaging.

In certain instances, PET imaging can be used to measure the binding kinetics of compounds of this invention with 5-HT₆ serotonin receptors. For example, administering an isotopically labeled compound of the invention that penetrates into the body and binds to a 5-HT₆ serotonin receptor creates a baseline PET signal which can be monitored while administering a second, different, non-isotopically labeled compound. The baseline PET signal will decrease as the non-isotopically labeled compound competes for the binding to the 5-HT₆ serotonin receptor.

In general, compounds of formula I that are useful in performing PET or SPECT are those which penetrate the blood-brain barrier, exhibit high selectivity and modest affinity to 5-HT₆ serotonin receptors, and are eventually metabolized. Compounds that are non-selective or those that exhibit excessive or small affinity for 5-HT₆ serotonin receptors are, generally, not useful in studying brain receptor binding kinetics with respect to 5-HT₆ serotonin receptors. Compounds that are not metabolized may harm the patient. Preferred compounds for isotopic labeling and use in performing PET or SPECT include 6-methyl-9-(phenylsulfonyl)-1,2,3,4,5,5a,6,10b-octahydroazepino[4,5-b]indole and 9-[(4-fluorophenyl)sulfonyl]6-methyl-1,2,3,4,5,5a,6,10b-octahydroazepino[4,5-b]indole; and pharmaceutically acceptable salts thereof.

In other embodiments, nuclear magnetic resonance spectroscopy (MRS) imaging can be used to detect the overall concentration of a compound or fragment thereof containing nuclei with a specific spin. In general, the isotopes useful in NMR imaging include, but are not limited to, hydrogen-1, carbon-13, phosphorus-31, and fluorine-19. For instance, the stereoisomers of compound 9-[(4-fluorophenyl)sulfonyl]6-methyl-1,2,3,4,5,5a,6,10b-octahydroazepino[4,5-b]indole and pharmaceutically acceptable salts thereof, when containing ¹⁹F are useful in conducting NMR imaging.

Further, substitution with heavier isotopes such as deuterium, i.e., ²H, can afford certain therapeutic advantages resulting from greater metabolic stability, for example increased in vivo half-life or reduced dosage requirements and, hence, maybe preferred in some circumstances. Isotopically labeled compounds of Formula I of this invention can generally be prepared by carrying out the synthetic procedures described above by substituting an isotopically labeled reagent for a non-isotopically labeled reagent.

Compounds of the present invention can conveniently be administered in a pharmaceutical composition containing the compound in combination with a suitable excipient. Such pharmaceutical compositions can be prepared by methods and contain excipients which are well known in the art. A generally recognized compendium of such methods and ingredients is Remington's Pharmaceutical Sciences by E.W. Martin (Mark Publ. Co., 15th Ed., 1975). The compounds and compositions of the present invention can be administered parenterally (for example, by intravenous, intraperitoneal or intramuscular injection), topically, orally, or rectally.

For oral therapeutic administration, the active compound may be combined with one or more excipients and used in the form of ingestible tablets, buccal tablets, troches, capsules, elixirs, suspensions, syrups, wafers, and the like. Such compositions and preparations should contain at least 0.1% of active compound. The percentage of the compositions and preparations may, of course, be varied and may conveniently be between about 2 to about 60% of the weight of a given unit dosage form. The amount of active compound in such therapeutically useful compositions is such that an effective dosage level will be obtained.

The tablets, troches, pills, capsules, and the like may also contain the following: binders such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, fructose, lactose or aspartame or a flavoring agent such as peppermint, oil of wintergreen, or cherry flavoring. The above listing is merely representative and one skilled in the art could envision other binders, excipients, sweetening agents and the like. When the unit dosage form is a capsule, it may contain, in addition to materials of the above type, a liquid carrier, such as a vegetable oil or a polyethylene glycol. Various other materials may be present as coatings or to otherwise modify the physical form of the solid unit dosage form. For instance, tablets, pills, or capsules may be coated with gelatin, wax, shellac or sugar and the like. A syrup or elixir may contain the active compound, sucrose or fructose as a sweetening agent, methyl and propylparabens as preservatives, a dye and flavoring such as cherry or orange flavor. Of course, any material used in preparing any unit dosage form should be pharmaceutically acceptable and substantially non-toxic in the amounts employed. In addition, the active compound may be incorporated into sustained-release preparations and devices including, but not limited to, those relying on osmotic pressures to obtain a desired release profile (e.g., the OROS drug delivery devices as designed and developed by Alza Corporation).

The compounds or compositions can also be administered intravenously or intraperitoneally by infusion or injection. Solutions of the active compound or its salts can be prepared in water, optionally mixed with a nontoxic surfactant. Dispersions can also be prepared in glycerol, liquid polyethylene glycols, triacetin, and mixtures thereof and in oils. Under ordinary conditions of storage and use, these preparations contain a preservative to prevent the growth of microorganisms.

Pharmaceutical dosage forms suitable for injection or infusion can include sterile aqueous solutions or dispersions or sterile powders comprising the active ingredient which are adapted for the extemporaneous preparation of sterile injectable or infusible solutions or dispersions, optionally encapsulated in liposomes. In all cases, the ultimate dosage form should be sterile, fluid and stable under the conditions of manufacture and storage. The liquid carrier or vehicle can be a solvent or liquid dispersion medium comprising, for example, water, ethanol, a polyol (for example, glycerol, propylene glycol, liquid polyethylene glycols, and the like), vegetable oils, nontoxic glyceryl esters, and suitable mixtures thereof. The proper fluidity can be maintained, for example, by the formation of liposomes, by the maintenance of the required particle size in the case of dispersions or by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars, buffers or sodium chloride. Prolonged absorption of the injectable compositions can be brought about by the use in the compositions of agents delaying absorption, for example, aluminum monostearate and gelatin.

Sterile injectable solutions can be prepared by incorporating the active compound in the required amount in the appropriate solvent with various of the other ingredients enumerated above, as required, followed by filter sterilization. In the case of sterile powders for the preparation of sterile injectable solutions, the preferred methods of preparation are vacuum drying and the freeze drying techniques, which yield a powder of the active ingredient plus any additional desired ingredient present in the previously sterile-filtered solutions. Sterilization of the powders may also be accomplished through irradiation and aseptic crystallization methods. The sterilization method selected is the choice of the skilled artisan.

For topical administration, the present compounds may be applied in pure form, i.e., when they are liquids. However, it will generally be desirable to administer them to the skin as compositions or formulations, in combination with a dermatologically acceptable carrier, which may be a solid or a liquid.

Useful solid carriers include finely divided solids such as talc, clay, microcrystalline cellulose, silica, alumina and the like. Useful liquid carriers include water, alcohols or glycols or water-alcohol/glycol blends, in which the present compounds can be dissolved or dispersed at effective levels, optionally with the aid of non-toxic surfactants. Adjuvants such as fragrances and additional antimicrobial agents can be added to optimize the properties for a given use. The resultant liquid compositions can be applied from absorbent pads, used to impregnate bandages and other dressings, or sprayed onto the affected area using pump-type or aerosol sprayers. Thickeners such as synthetic polymers, fatty acids, fatty acid salts and esters, fatty alcohols, modified celluloses or modified mineral materials can also be employed with liquid carriers to form spreadable pastes, gels, ointments, soaps, and the like, for application directly to the skin of the user. To this extent, the present invention further contemplates the use of the pharmaceutically active materials in personal care compositions such as lotions, cleansers, powders, cosmetics and the like.

The compound is conveniently administered in unit dosage form; for example, containing about 0.05 mg to about 500 mg, conveniently about 0.1 mg to about 250 mg, most conveniently, about 1 mg to about 150 mg of active ingredient per unit dosage form. The desired dose may conveniently be presented in a single dose or as divided doses administered at appropriate intervals, for example, as two, three, four or more sub-doses per day. The sub-dose itself may be further divided, e.g., into a number of discrete loosely spaced administrations.

The compositions can conveniently be administered orally, sublingually, transdermally, or parenterally at dose levels of about 0.01 to about 150 mg/kg, preferably about 0.1 to about 50 mg/kg, and more preferably about 0.1 to about 30 mg/kg of mammal body weight.

For parenteral administration the compounds are presented in aqueous solution in a concentration of from about 0.1 to about 10%, more preferably about 0.1 to about 7%. The solution may contain other ingredients, such as emulsifiers, antioxidants or buffers.

The exact regimen for administration of the compounds and compositions disclosed herein will necessarily be dependent upon the needs of the individual subject being treated, the type of treatment and, of course, the judgment of the attending practitioner.

Generally, compounds of the invention are 5-HT ligands. The ability of a compound of the invention to bind or act at a 5-HT receptor, or to bind or act selectively at a specific 5-HT receptor subtype can be determined using in vitro and in vivo assays that are known in the art. As used herein, the term "bind selectively" means a compound binds at least 2 times, preferably at least 10 times, and more preferably at least 50 times more readily to a given 5-HT subtype than to one or more other subtypes. Preferred compounds of the invention bind selectively to one or more 5-HT receptor subtypes.

The ability of a compound of the invention to act as a 5-HT receptor agonist or antagonist can also be determined using in vitro and in vivo assays that are known in the art. All of the Example compounds provided above are 5-HT ligands, with the ability to displace >50% of a radiolabeled test ligand from one or more 5-HT receptor subtypes at a concentration of 1 µM. The procedures used for testing such displacement are well known and illustrated below.

### 5-HT₆ RECEPORT BINDING ASSAY

### Growth of Cells and Mernbrane Preparation

Hela cells containing the cloned human 5-HT₆ receptor were acquired from Dr. David R. Sibley's laboratory in National Institute of Health (see Sibley, D.R., J. Neurochemistry, 66, 47-56, 1996). Cells were grown in high glucose Dulbecco's modified Eagle's medium, supplemented with L-glutamine, 0.5% sodium pyruvate, 0.3% penicillin-streptomycin, 0.025% G-418 and 5% Gibco fetal bovine serum and then were harvested, when confluent, in cold phosphate buffered saline.

Harvested intact cells were washed once in cold phosphate-buffered saline. The cells were pelleted and resuspended in 100 ml of cold 50 mM Tris, 5 mM EDTA and 5 mM EGTA, pH 7.4. Homogenization was with a Vir Tishear generator, 4 cycles for 30 seconds each at setting 50. The homogenized cells were centrifuged at 700 RPM (1000 X g) for 10 minutes and the supernatant was removed. The pellet was resuspended in 100 ml of the above buffer and rehomogenized for 2 cycles. The rehomogenized cells were then centrifuged at 700 RPM (1000 X g) for 10 minutes and the supernatant was removed. The combined supernatant (200ml) was centrifuged at 23,000 RPM (80,000 X g) for 1 hour in a Beckman Rotor (42.1 Ti). The membrane pellet was resuspended in 50-8- ml of assay buffer containing HEPES 20 mM, MgC12 10 mM, NaCl 150 mM, EDTA 1mM, pH 7.4 and stored frozen in aliqouts at -70°C.

### 5-HT₆ Receptor Binding Assay

The radioligand binding assay used [³H]-lysergic acid diethylamide (LSD). The assay was carried out in Wallac 96-well sample plates by the addition of 11 µl of the test sample at the appropriate dilution (the assay employed 11 serial concentrations of samples run in duplicate), 11 µl of radioligand, and 178 µl of a washed mixture of WGA-coated SPA beads and membranes in binding buffer. The plates were shaken for about 5 minutes and then incubated at room temperature for 1 hour. The plates were then loaded into counting cassettes and counted in a Wallac MicroBeta Trilux scintillation counter.

### Binding Constant (Ki) Determination

Eleven serial dilutions of test compounds were distributed to assay plates using the PE/Cetus Pro/Pette pipetter. These dilutions were, followed by radioligand and the bead-membrane mixture prepared as described above. The specifically bound cpm obtained were fit to a one-site binding model using GraphPad Prism ver. 2.0. Estimated IC₅₀ values were converted to Ki values using the Cheng-Prusoff equation (Cheng, Y. C. et al., Biochem. Pharmacol., 22, 3099-108, 1973). The Ki values obtained from the assay are shown in Table 1.

The compounds and their preparations of the present invention will be better understood in connection with the following examples, which are intended as an illustration of and not a limitation upon the scope of the invention.

### EXAMPLES

### EXAMPLE 1 (5aS, 10bS)-6-methyl-9-(phenylsulfonyl)-1,2,3,4,5,5a,6,10b-octahydroazepino[4,5-b]indole hydrochloride and (5aR, 10bR)-6-methyl-9-(phenylsulfonyl)-1,2,3,4,5,5a,6,10b-octahydroazepino[4,5-b]indole

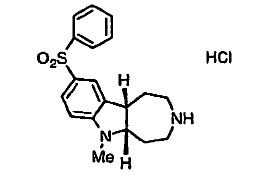

### Step 1: cis-6-methyl-9-(phenylsulfonyl)-1,2,3,4,5,5a,6,10b-octahydroazepino[4,5-b]indole hydrochloride

A cold (0 °C) solution of 6-methyl-9-(phenylsulfonyl)-1,2,3,4,5,6-hexahydroazepino[4,5-b]indole (3.21 g, 9.43 mmol) in trifluoroacetic acid (TFA, 95 mL) was treated with a solution of sodium cyanoborohydride (3.09 g, 49.2 mmol) in MeOH (25 mL). The brown solution turned yellow and was maintained at 0 °C for 45 min. The ice bath was removed and the solution stirred at rt under N₂ for 3 h. The resultant mixture was diluted with H₂O and concentrated to remove the majority of the acid. The residue was made basic with aq. KOH, and extracted with CH₂Cl₂ (3X). The extracts were dried (MgSO₄), filtered and concentrated to give 675 mg of the desired material as an amorphous solid. The HCl salt was prepared using HCl/EtOAc for analytical purposes mp = >300 °C: MS (EI) *m*/*z* 342.

### Step 2: cis-tert-butyl 6-methyl-9-(phenylsulfonyl)-1,4,5,5a,6,10b-hexahydroazepino[4,5-b]indole-3(2H)-carboxylate

A mixture of cis-6-methyl-9-(phenylsulfonyl)-1,2,3,4,5,6-hexahydroazepino[4,5-b]indole (675 mg, 1.97 mmol), di-*tert*-butyl dicarbonate (474 mg, 2.17 mmol) and a catalytic amount of 4-dimethylaminopyridine in dry CH₂Cl₂ (10 mL) was stirred at rt under N₂ for 16 h. The resultant solution was diluted with H₂O and extracted into CH₂Cl₂ (3X). The extracts were dried over anhydrous MgSO₄, filtered and concentrated under reduced pressure to give a white, amorphous solid. The solid was purified by silica gel chromatography (Biotage 40S; 40% EtOAc/heptane) to furnish 570 mg of the desired material. The material was crystallized from hot EtOAc/hexane to furnish 490 mg of the desired material as a white, crystalline solid, mp = 163-164 °C: MS(EI) m/z 442.

### Step 3: (5aS, 10bS)-6-methyl-9-(phenylsulfonyl)-1,2,3,4,5,5a,6,10b-octahydroazepino[4,5-b]indole hydrochloride and (5aR, 10bR)-6-methyl-9-(phenylsulfonyl)-1,2,3,4,5,5a,6,10b-octahydroazepino[4,5-b]indole hydrochloride

Racemic *cis*-*tert*-butyl 6-methyl-9-(phenylsulfonyl)-1,4,5,5a,6,10b-hexahydroazepino[4,5-b]indole-3(2H)-carboxylate can be separated on a Chiracel OD-H column (0.46X25 cm; 0.5 mL/min; 30% isopropanol/heptane) to give the desired enantiomers as pure and distinct entities. Each of these enantiomers are then stirred in saturated methanolic HCl (20 mL) at 50°C under N₂ for 2 h. The solvent was removed in vacuo and the residual solid was crystallized from hot MeOH/EtOAc to provide 106 mg of the desired 9-arylsulfonyl azepinoindolines as a white, crystalline solids, mp = >300 °C: MS (EI) m/z 342.

### EXAMPLE 2 (5aR, 10bS)-6-methyl-9-(phenylsulfonyl)-1,2,3,4,5,5a,6,10b-octahydroazepino[4,5-b]indole hydrochloride and (5aS, 10bR)-6-methyl-9-(phenylsulfonyl)-1,2,3,4,5,5a,6,10b-octahydroazepino[4,5-b]indole hydrochloride

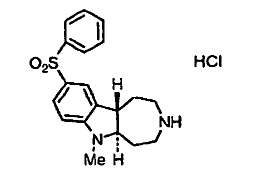

### Step 1: trans-9-(phenylsulfonyl)-1,2,3,4,5,5a,6,10b-octahydroazepino[4,5-b]indole

*tert*-Butyl 9-(phenylsulfonyl)-1,4,5,6-tetrahydroazepino[4,5-b]indole-3(2H)-carboxylate (408 mg, 1.25 mmol, 3-Boc-protected 1 (R¹ = H)) was treated with borane-tetrahydrofuran complex (1 M in THF, 5.0 mL, 5.00 mmol) and stirred at rt under N₂ for 1.5 h. The excess BH₃ was carefully quenched with H₂O and then the mixture was transferred to a cold (0 °C) solution of TFA (15 mL). After 20 min, the mixture was diluted with H₂O and concentrated under reduced pressure to near dryness. The residue was diluted with H₂O, made basic with 4N NaOH, and extracted with CH₂Cl₂ (3X). The extracts were dried (MgSO₄), filtered and concentrated to give 362 mg of the desired material as an amorphous solid.

### Step 2: trans-tert-butyl 9-(phenylsulfonyl)-1,4,5,5a,6,10b-hexahydroazepino[4,5-b]indole-3(2H)-carboxylate

A solution of *trans*-9-(phenylsulfonyl)-1,2,3,4,5,6-hexahydroazepino[4,5-b]indole (358 mg, 1.09 mmol), di-*tert*-butyl dicarbonate (262 mg, 1.20 mmol) and a catalytic amount of 4-dimethylaminopyridine in dry CH₂Cl₂ (5 mL) was stirred at rt under N₂ for 16 h. The resultant solution was diluted with H₂O and extracted into CH₂Cl₂ (3X). The extracts were dried over anhydrous MgSO₄, filtered and concentrated under reduced pressure to give 450 mg of a white, amorphous solid. The solid was purified by silica gel chromatography (Biotage 40S; 40% EtOAc/heptane) to furnish 353 mg of the desired material as a white, amorphous solid.

### Step 3: trans-tert-butyl 6-methyl-9-(phenylsulfonyl)-1,4,5,5a,6,10b-hexahydroazepino[4,5-b]indole-3(2H)-carboxylate

A cold (0 °C) solution of the *trans-tert*-butyl 9-(phenylsulfonyl)-1,2,3,4,5,6-hexahydroazepino[4,5-b]indole-3(2H)-carboxylate (170 mg, 0.397 mmol) in dry DMF (4 mL) was treated with NaH (60% in oil, 17 mg, 0.44 mmol). The resultant yellow solution was stirred at 0 °C under N₂ for 30 min and was then treated with iodomethane (27 µL, 0.44 mmol). The solution was allowed to slowly warm to rt over several hours, stirring under N₂. After dilution with EtOAc, the product was washed with H₂O (3X) and brine. The organic layer was dried over anhydrous MgSO₄, filtered, and concentrated under reduced pressure. The residue was purified via silica gel chromatography (Biotage 40S; 35% EtOAc/heptane) to furnish 139 mg of the desired product as a white, amorphous solid: MS(EI) m/z 442.

### Step 4: (5aR, 10bS)-6-methyl-9-(phenylsulfonyl)-1,2,3,4,5,5a,6,10b-octahydroazepino[4,5-b]indole hydrochloride and (5aS, 10bR)-6-methyl-9-(phenylsulfonyl)-1,2,3,4,5,5a,6,10b-octahydroazepino[4,5-b]indole hydrochloride

Racemic *tert*-butyl 6-methyl-9-(phenylsulfonyl)-1,4,5,5a,6,10b-hexahydroazepino[4,5-b]indole-3(2H)-carboxylate can be separated on a Chiracel OD-H column (0.46X25 cm; 0.5 mL/min; 30% isopropanol/heptane) to give the desired enantiomers as pure and distinct entities. A solution of either enantiomer (6 or 7; 136 mg, 0.307 mmol) in saturated methanolic HCl (20 mL) was heated at 50 °C under N₂ for 2 h. The solvent was removed *in vacuo* and the residual solid was crystallized from hot MeOH/EtOAc to provide 90 mg of the desired 9-arylsulfonyl azepinoindoline as a white, crystalline solid: MS (EI) m/z 342.

## Claims

1. A compound of formula I 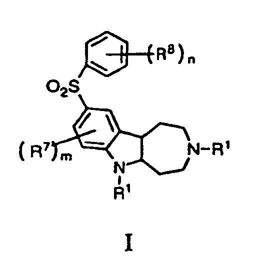 wherein each R¹ is independently
a) H,
b) C₁₋₄ alkyl,
c) C₁₋₄alkyl substituted by a phenyl where the phenyl is optionally substituted with one or two R², or
d) phenyl, optionally substituted with one or two R²;
R² is
a) halo,
b) OR³,
c) CF₃
d) C(=O)-NR⁴R⁵,
e) NH-SO₂-R⁶,
f) NR⁴R⁵,
g) NR⁴-C(=O)-R⁴,
h) SO₂-NR⁴R⁵,
i) CN, or
j) NO₂;
R³ is H, C₁₋₄ alkyl, or phenyl;
R⁴ and R⁵ are independently H, C₁₋₄ alkyl, or R⁴ and R⁵ taken together with the attached nitrogen atom to form a ring selected from the group consisting of 1-pyrrolidinyl, 1-piperazinyl and 1-morpholinyl;
R⁶ is H or C₁₋₄ alkyl;
R⁷ is
a) H, or
b) halo,
c) OR³,
d) CF₃
e) C(=O)-NR⁴R⁵,
f) NH-SO₂-R⁶,
g) NR⁴R⁵,
h) NR⁴-C(=O)-R⁴,
i) SO₂-NR⁴R⁵,
j) CN, or
k) NO₂;
R⁸ is
a) H,
b) F,
c) Cl,
d) C₁₋₄alkyl,
e) C₁₋₃alkoxy,
f) CF₃,
g) C₁₋₄alkyl substituted by a phenyl wherein the phenyl is optionally substituted with one or two R²,
h) phenyl, optionally substituted with one or two R²,
i) OR³,
j) CO-NR⁴R⁵,
k) NR⁴R⁵,
l) NH-SO₂-R⁶, or
m) NH-CO-R⁴;
at each occurrence, alkyl and alkoxy is optionally substituted with OH, halo, or NH₂; m is 1 to 2; and n is 1-3;
or a pharmaceutically acceptable salt thereof.

2. A compound of claim 1 wherein each R¹ is independently H, methyl, or ethyl.

3. A compound of claim 1 wherein R⁷ is hydrogen.

4. A compound of claim 1 wherein R⁸ is hydrogen.

5. A compound of claim 1 wherein R⁸ is F, OCH₃, or CF₃.

6. A compound of claim 1 which is
(a) (R, R,) 6-methyl-1,2,3,4,5,5a,6,10b-octahydroazepino[4,5-b]indol-9-yl phenyl sulfone hydrochloride,
(b) (S, S,) 6-methyl-1,2,3,4,5,5a,6,10b-octahydroazepino[4,5-b]indol-9-yl phenyl sulfone hydrochloride,
(c) (R, S,) 6-methyl-1,2,3,4,5,5a,6,10b-octahydroazepino[4,5-b]indol-9-yl phenyl sulfone hydrochloride, or
(d) (S, R,) 6-methyl-1,2,3,4,5,5a,6,10b-octahydroazepino[4,5-b]indol-9-yl phenyl sulfone hydrochloride.

7. A compound selected from:
(5aS, 10bS)-9-[(4-fluorophenyl)sulfonyl]-6-methyl-1,2,3,4,5,5a,6,10b-octahydroazepino[4,5-b]indole
(5aS, 10bS)-9-[(3-fluorophenyl)sulfonyl]-6-methyl-1,2,3,4,5,5a,6,10b-octahydroazepino[4,5-b]indole
(5aS, 10bS)-9-[(3,4-difluorophenyl)sulfonyl]-6-methyl-1,2,3,4,5,5a,6,10b-octahydroazepino[4,5-b]indole
(5aS, 10bS)-9-[(3,5-difluorophenyl)sulfonyl]-6-methyl-1,2,3,4,5,5a,6,10b-octahydroazepino[4,5-b]indole
(5aS, 10bS)-6-methyl-9-[(4-methylphenyl)sulfonyl]-1,2,3,4,5,5a,6,10b-octahydroazepino[4,5-b]indole
(5aS, 10bS)-6-methyl-9-[(3-methylphenyl)sulfonyl]-1,2,3,4,5,5a,6,10b-octahydroazepino[4,5-b]indole
(5aS, 10bS)-9-[(4-methoxyphenyl)sulfonyl]-6-methyl-1,2,3,4,5,5a,6,10b-octahydroazepino[4,5-b]indole
(5aS, 10bS)-9-[(3-methoxyphenyl)sulfonyl]-6-methyl-1,2,3,4,5,5a,6,10b-octahydroazepino[4,5-b]indole
(5aS, 10bS)-6-methyl-9-[[(4-trifluoromethyl)phenyl]sulfonyl]-1,2,3,4,5,5a,6,10b-octahydroazepino[4,5-b]indole
(5aS, 10bS)-3,6-dimethyl-9-(phenylsulfonyl)-1,2,3,4,5,5a,6,10b-octahydroazepino[4,5-b]indole
(5aS, 10bS)-9-[(4-fluorophenyl)sulfonyl]-3,6-dimethyl-1,2,3,4,5,5a,6,10b-octahydroazepino[4,5-b]indole
(5aS, 10bS)-9-[(3-fluorophenyl)sulfonyl]-3,6-dimethyl-1,2,3,4,5,5a,6,10b-octahydroazepino[4,5-b]indole
(5aS, 10bS)-9-[(3,4-difluorophenyl)sulfonyl]-3,6-dimethyl-1,2,3,4,5,5a,6,10b-octahydroazepino[4,5-b]indole
(5aS, 10bS)-9-[(3,5-difluorophenyl)sulfonyl]-3,6-dimethyl-1,2,3,4,5,5a,6,10b-octahydroazepino[4,5-b]indole
(5aS, 10bS)-3,6-dimethyl-9-[(4-methylphenyl)sulfonyl]-1,2,3,4,5,5a,6,10b-octahydroazepino[4,5-b]indole
(5aS, 10bS)-3,6-dimethyl-9-[(3-methylphenyl)sulfonyl]-1,2,3,4,5,5a,6,10b-octahydroazepino[4,5-b]indole
(5aS, 10bS)-9-[(4-methoxyphenyl)sulfonyl]-3,6-dimethyl-1,2,3,4,5,5a,6,10b-octahydroazepino[4,5-b]indole
(5aS, 10bS)-9-[(3-methoxyphenyl)sulfonyl]-3,6-dimethyl-1,2,3,4,5,5a,6,10b-octahydroazepino[4,5-b]indole
(5aS, 10bS)-3,6-dimethyl-9-[[(4-trifluoromethyl)phenyl]sulfonyl]-1,2,3,4,5,5a,6,10b-octahydroazepino[4,5-b]indole
(5aS, 10bS)-3-ethyl-6-methyl-9-(phenylsulfonyl)-1,2,3,4,5,5a,6,10b-octahydroazepino[4,5-b]indole
(5aS, 10bS)-3-ethyl-9-[(4-fluorophenyl)sulfonyl]-6-methyl-1,2,3,4,5,5a,6,10b-octahydroazepino[4,5-b]indole
(5aS, 10bS)-3-ethyl-9-[(3-fluorophenyl)sulfonyl]-6-methyl-1,2,3,4,5,5a,6,10b-octahydroazepino[4,5-b]indole
(5aS, 10bS)-3-ethyl-9-[(3,4-difluorophenyl)sulfonyl]-6-methyl-1,2,3,4,5,5a,6,10b-octahydroazepino[4,5-b]indole
(5aS, 10bS)-3-ethyl-9-[(3,5-difluorophenyl)sulfonyl]-6-methyl-1,2,3,4,5,5a,6,10b-octabydroazepino[4,5-b]indole
(5aS, 10bS)-3-ethyl-6-methyl-9-[(4-methylphenyl)sulfonyl]-1,2,3,4,5,5a,6,10b-octahydroazepino[4,5-b]indole
(5aS, 10bS)-3-ethyl-6-methyl-9-[(3-methylphenyl)sulfonyl]-1,2,3,4,5,5a,6,10b-octahydroazepino[4,5-b]indole
(5aS, 10bS)-3-ethyl-9-[(4-methoxyphenyl)sulfonyl]-6-methyl-1,2,3,4,5,5a,6,10b-octahydroazepino[4,5-b]indole
(5aS, 10bS)-3-ethyl-9-[(3-methoxyphenyl)sulfonyl]-6-methyl-1,2,3,4,5,5a,6,10b-octahydroazepino[4,5-b]indole
(5aS, 10bS)-3-ethyl-6-methyl-9-[[(4-trifluoromethyl)phenyl]sulfonyl]-1,2,3,4,5,5a,6,10b-octahydroazepino[4,5-b]indole
(5aS, 10bS)-1,6-dimethyl-9-(phenylsulfonyl)-1,2,3,4,5,5a,6,10b-octahydroazepino[4,5-b]indole
(5aS, 10bS)-2,6-dimethyl-9-(phenylsulfonyl)-1,2,3,4,5,5a,6,10b-octahydroazepino[4,5-b]indole
(5aS, 10bS)-4,6-dimethyl-9-(phenylsulfonyl)-1,2,3,4,5,5a,6,10b-octahydroazepino[4,5-b]indole
(5aS, 10bS)-5,6-dimethyl-9-(phenylsulfonyl)-1,2,3,4,5,5a,6,10b-octahydroazepino[4,5-b]indole
(5aS, 10bS)-9-[(4-fluorophenyl)sulfonyl]-1,6-dimethyl-1,2,3,4,5,5a,6,10b-octahydroazepino[4,5-b]indole
(5aS, 10bS)-9-[(4-fluorophenyl)sulfonyl]-2,6-dimethyl-1,2,3,4,5,5a,6,10b-octahydroazepino[4,5-b]indole
(5aS, 10bS)-9-[(4-fluorophenyl)sulfonyl]-4,6-dimethyl-1,2,3,4,5,5a,6,10b-octahydroazepino[4,5-b]indole
(5aS, 10bS)-9-[(4-fluorophenyl)sulfonyl]-5,6-dimethyl-1,2,3,4,5,5a,6,10b-octahydroazepino[4,5-b]indole
(5aS, 10bS)-9-[(3-fluorophenyl)sulfonyl]-1,6-dimethyl-1,2,3,4,5,5a,6,10b-octahydroazepino[4,5-b]indole
(5aS, 10bS)-9-[(3-fluorophenyl)sulfonyl]-2,6-dimethyl-1,2,3,4,5,5a,6,10b-octahydroazepino[4,5-b]indole
(5aS, 10bS)-9-[(3-fluorophenyl)sulfonyl]-4,6-dimethyl-1,2,3,4,5,5a,6,10b-octahydroazepino[4,5-b]indole
(5aS, 10bS)-9-[(3-fluorophenyl)sulfonyl]-5,6-dimethyl-1,2,3,4,5,5a,6,10b-octahydroazepino[4,5-b]indole
(5aS, 10bS)-1,5,6-trimethyl-9-(phenylsulfonyl)-1,2,3,4,5,5a,6,10b-octahydroazepino[4,5-b]indole
(5aS, 10bS)-2,4,6-trimethyl-9-(phenylsulfonyl)-1,2,3,4,5,5a,6,10b-octahydroazepino[4,5-b]indole
(5aS, 10bS)-9-[(4-fluorophenyl)sulfonyl]-1,5,6-trimethyl-1,2,3,4,5,5a,6,10b-octahydroazepino[4,5-b]indole
(5aS, 10bS)-9-[(4-fluorophenyl)sulfonyl]-2,4,6-trimethyl-1,2,3,4,5,5a,6,10b-octahydroazepino[4,5-b]indole
(5aS, 10bS)-9-[(3-fluorophenyl)sulfonyl]-1,5,6-trimethyl-1,2,3,4,5,5a,6,10b-octahydroazepino[4,5-b]indole
(5aS, 10bS)-9-[(3-fluorophenyl)sulfonyl]-2,4,6-trimethyl-1,2,3,4,5,5a,6,10b-octahydroazepino[4,5-b]indole
(5aS, 10bS)-6-methyl-9-(phenylsulfonyl)-1,2,3,4,5,5a,6,10b-octahydroazepino[4,5-b]indole
(5aS, 10bS)-9-[(4-fluorophenyl)sulfonyl]-1,2,3,4,5,5a,6,10b-octahydroazepino[4,5-b]indole
(5aS, 10bS)-9-[(3-fluorophenyl)sulfonyl]-1,2,3,4,5,5a,6,10b-octahydroazepino[4,5-b]indole
(5aS, 10bS)-9-[(3,4-difluorophenyl)sulfonyl]-1,2,3,4,5,5a,6,10b-octahydroazepino[4,5-b]indole
(5aS, 10bS)-9-[(3,5-difluorophenyl)sulfonyl]-1,2,3,4,5,5a,6,10b-octahydroazepino[4,5-b]indole
(5aS, 10bS)-9-[(4-methylphenyl)sulfonyl]-1,2,3,4,5,5a,6,10b-octahydroazepino[4,5-b]indole
(5aS, 10bS)-9-[(3-methylphenyl)sulfonyl]-1,2,3,4,5,5a,6,10b-octahydroazepino[4,5-b]indole
(5aS, 10bS)-9-[(4-methoxyphenyl)sulfonyl]-1,2,3,4,5,5a,6,10b-octahydroazepino[4,5-b]indole
(5aS, 10bS)-9-[(3-methoxyphenyl)sulfonyl]-1,2,3,4,5,5a,6,10b-octahydroazepino[4,5-b]indole
(5aS, 10bS)-9-[[(4-trifluoromethyl)phenyl]sulfonyl]-1,2,3,4,5,5a,6,10b-octahydroazepino[4,5-b]indole
(5aS, 10bS)-3-methyl-9-(phenylsulfonyl)-1,2,3,4,5,5a,6,10b-octahydroazepino[4,5-b]indole
(5aS, 10bS)-9-[(4-fluorophenyl)sulfonyl]-3-methyl-1,2,3,4,5,5a,6,10b-octahydroazepino[4,5-b]indole
(5aS, 10bS)-9-[(3-fluorophenyl)sulfonyl]-3-methyl-1,2,3,4,5,5a,6,10b-octahydroazepino[4,5-b]indole
(5aS, 10bS)-9-[(3,4-difluorophenyl)sulfonyl]-3-methyl-1,2,3,4,5,5a,6,10b-octahydroazepino[4,5-b]indole
(5aS, 10bS)-9-[(3,5-difluorophenyl)sulfonyl]-3-methyl-1,2,3,4,5,5a,6,10b-octahydroazepino[4,5-b]indole
(5aS, 10bS)-3-methyl-9-[(4-methylphenyl)sulfonyl]-1,2,3,4,5,5a,6,10b-octahydroazepino[4,5-b]indole
(5aS, 10bS)-3-methyl-9-[(3-methylphenyl)sulfonyl]-1,2,3,4,5,5a,6,10b-octahydroazepino[4,5-b]indole
(5aS, 10bS)-9-[(4-methoxyphenyl)sulfonyl]-3-methyl-1,2,3,4,5,5a,6,10b-octahydroazepino[4,5-b]indole
(5aS, 10bS)-9-[(3-methoxyphenyl)sulfonyl]-3-methyl-1,2,3,4,5,5a,6,10b-octahydroazepino[4,5-b]indole
(5aS, 10bS)-3-methyl-9-[[(4-trifluoromethyl)phenyl]sulfonyl]-1,2,3,4,5,5a,6,10b-octahydroazepino[4,5-b]indole
(5aS, 10bS)-3-ethyl-9-(phenylsulfonyl)-1,2,3,4,5,5a,6,10b-octahydroazepino[4,5-b]indole
(5aS, 10bS)-3-ethyl-9-[(4-fluorophenyl)sulfonyl]-1,2,3,4,5,5a,6,10b-octahydroazepino[4,5-b]indole
(5aS, 10bS)-3-ethyl-9-[(3-fluorophenyl)sulfonyl]-1,2,3,4,5,5a,6,10b-octahydroazepino[4,5-b]indole
(5aS, 10bS)-3-ethyl-9-[(3,4-difluorophenyl)sulfonyl]-1,2,3,4,5,5a,6,10b-octahydroazepino[4,5-b]indole
(5aS, 10bS)-3-ethyl-9-[(3,5-difluorophenyl)sulfonyl]-1,2,3,4,5,5a,6,10b-octahydroazepino[4,5-b]indole
(5aS, 10bS)-3-ethyl-9-[(4-methylphenyl)sulfonyl]-1,2,3,4,5,5a,6,10b-octahydroazepino[4,5-b]indole
(5aS, 10bS)-3-ethyl-9-[(3-methylphenyl)sulfonyl]-1,2,3,4,5,5a,6,10b-octahydroazepino[4,5-b]indole
(5aS, 10bS)-3-ethyl-9-[(4-methoxyphenyl)sulfonyl]-1,2,3,4,5,5a,6,10b-octahydroazepino[4,5-b]indole
(5aS, 10bS)-3-ethyl-9-[(3-methoxyphenyl)sulfonyl]-1,2,3,4,5,5a,6,10b-octahydroazepino[4,5-b]indole
(5aS, 10bS)-3-ethyl-9-[[(4-trifluoromethyl)phenyl]sulfonyl]-1,2,3,4,5,5a,6,10b-octahydroazepino[4,5-b]indole
(5aS, 10bS)-1-methyl-9-(phenylsulfonyl)-1,2,3,4,5,5a,6,10b-octahydroazepino[4,5-b]indole
(5aS, 10bS)-2-methyl-9-(phenylsulfonyl)-1,2,3,4,5,5a,6,10b-octahydroazepino[4,5-b]indole
(5aS, 10bS)-4-methyl-9-(phenylsulfonyl)-1,2,3,4,5,5a,6,10b-octahydroazepino[4,5-b]indole
(5aS, 10bS)-5-methyl-9-(phenylsulfonyl)-1,2,3,4,5,5a,6,10b-octahydroazepino[4,5-b]indole
(5aS, 10bS)-9-[(4-fluorophenyl)sulfonyl]-1-methyl-1,2,3,4,5,5a,6,10b-octahydroazepino[4,5-b]indole
(5aS, 10bS)-9-[(4-fluorophenyl)sulfonyl]-2-methyl-1,2,3,4,5,5a,6,10b-octahydroazepino[4,5-b]indole
(5aS, 10bS)-9-[(4-fluorophenyl)sulfonyl]-4-methyl-1,2,3,4,5,5a,6,10b-octahydroazepino[4,5-b]indole
(5aS, 10bS)-9-[(4-fluorophenyl)sulfonyl]-5-methyl-1,2,3,4,5,5a,6,10b-octahydroazepino[4,5-b]indole
(5aS, 10bS)-9-[(3-fluorophenyl)sulfonyl]-1-methyl-1,2,3,4,5,5a,6,10b-octahydroazepino[4,5-b]indole
(5aS, 10bS)-9-[(3-fluorophenyl)sulfonyl]-2-methyl-1,2,3,4,5,5a,6,10b-octahydroazepino[4,5-b]indole
(5aS, 10bS)-9-[(3-fluorophenyl)sulfonyl]-4-methyl-1,2,3,4,5,5a,6,10b-octahydroazepino[4,5-b]indole
(5aS, 10bS)-9-[(3-fluorophenyl)sulfonyl]-5-methyl-1,2,3,4,5,5a,6,10b-octahydroazepino[4,5-b]indole
(5aS, 10bS)-1,5-dimethyl-9-(phenylsulfonyl)-1,2,3,4,5,5a,6,10b-octahydroazepino[4,5-b]indole
(5aS, 10bS)-2,4-dimethyl-9-(phenylsulfonyl)-1,2,3,4,5,5a,6,10b-octahydroazepino[4,5-b]indole
(5aS, 10bS)-9-[(4-fluorophenyl)sulfonyl]-1,5-dimethyl-1,2,3,4,5,5a,6,10b-octahydroazepino[4,5-b]indole
(5aS, 10bS)-9-[(4-fluorophenyl)sulfonyl]-2,4-dimethyl-1,2,3,4,5,5a,6,10b-octahydroazepino[4,5-b]indole
(5aS, 10bS)-9-[(3-fluorophenyl)sulfonyl]-1,5-dimethyl-1,2,3,4,5,5a,6,10b-octahydroazepino[4,5-b]indole
(5aS, 10bS)-9-[(3-fluorophenyl)sulfonyl]-2,4-dimethyl-1,2,3,4,5,5a,6,10b-octahydroazepino[4,5-b]indole
(5aR, 10bS)-9-[(4-fluorophenyl)sulfonyl]-6-methyl-1,2,3,4,5,5a,6,10b-octahydroazepino[4,5-b]indole
(5aR, 10bS)-9-[(3-fluorophenyl)sulfonyl]-6-methyl-1,2,3,4,5,5a,6,10b-octahydroazepino[4,5-b]indole
(5aR, 10bS)-9-[(3,4-difluorophenyl)sulfonyl]-6-methyl-1,2,3,4,5,5a,6,10b-octahydroazepino[4,5-b]indole
(5aR, 10bS)-9-[(3,5-difluorophenyl)sulfonyl]-6-methyl-1,2,3,4,5,5a,6,10b-octahydroazepino[4,5-b]indole
(5aR, 10bS)-6-methyl-9-[(4-methylphenyl)sulfonyl]-1,2,3,4,5,5a,6,10b-octahydroazepino[4,5-b]indole
(5aR, 10bS)-6-methyl-9-[(3-methylphenyl)sulfonyl]-1,2,3,4,5,5a,6,10b-octahydroazepino[4,5-b]indole
(5aR, 10bS)-9-[(4-methoxyphenyl)sulfonyl]-6-methyl-1,2,3,4,5,5a,6,10b-octahydroazepino[4,5-b]indole
(5aR, 10bS)-9-[(3-methoxyphenyl)sulfonyl]-6-methyl-1,2,3,4,5,5a,6,10b-octahydroazepino[4,5-b]indole
(5aR, 10bS)-6-methyl-9-[[(4-trifluoromethyl)phenyl]sulfonyl]-1,2,3,4,5,5a,6,10b-octahydroazepino[4,5-b]indole
(5aR, 10bS)-3,6-dimethyl-9-(phenylsulfonyl)-1,2,3,4,5,5a,6,10b-octahydroazepino[4,5-b]indole
(5aR, 10bS)-9-[(4-fluorophenyl)sulfonyl]-3,6-dimethyl-1,2,3,4,5,5a,6,10b-octahydroazepino[4,5-b]indole
(5aR, 10bS)-9-[(3-fluorophenyl)sulfonyl]-3,6-dimethyl-1,2,3,4,5,5a,6,10b-octahydroazepino[4,5-b]indole
(5aR, 10bS)-9-[(3,4-difluorophenyl)sulfonyl]-3,6-dimethyl-1,2,3,4,5,5a,6,10b-octahydroazepino[4,5-b]indole
(5aR, 10bS)-9-[(3,5-difluorophenyl)sulfonyl]-3,6-dimethyl-1,2,3,4,5,5a,6,10b-octahydroazepino[4,5-b]indole
(5aR, 10bS)-3,6-dimethyl-9-[(4-methylphenyl)sulfonyl]-1,2,3,4,5,5a,6,10b-octahydroazepino[4,5-b]indole
(5aR, 10bS)-3,6-dimethyl-9-[(3-methylphenyl)sulfonyl]-1,2,3,4,5,5a,6,10b-octahydroazepino[4,5-b]indole
(5aR, 10bS)-9-[(4-methoxyphenyl)sulfonyl]-3,6-dimethyl-1,2,3,4,5,5a,6,10b-octahydroazepino[4,5-b]indole
(5aR, 10bS)-9-[(3-methoxyphenyl)sulfonyl]-3,6-dimethyl-1,2,3,4,5,5a,6,10b-octahydroazepino[4,5-b]indole
(5aR, 10bS)-3,6-dimethyl-9-[[(4-trifluoromethyl)phenyl]sulfonyl]-1,2,3,4,5,5a,6,10b-octahydroazepino[4,5-b]indole
(5aR, 10bS)-3-ethyl-6-methyl-9-(phenylsulfonyl)-1,2,3,4,5,5a,6,10b-octahydroazepino[4,5-b]indole
(5aR, 10bS)-3-ethyl-9-[(4-fluorophenyl)sulfonyl]-6-methyl-1,2,3,4,5,5a,6,10b-octahydroazepino[4,5-b]indole
(5aR, 10bS)-3-ethyl-9-[(3-fluorophenyl)sulfonyl]-6-methyl-1,2,3,4,5,5a,6,10b-octahydroazepino[4,5-b]indole
(5aR, 10bS)-3-ethyl-9-[(3,4-difluorophenyl)sulfonyl]-6-methyl-1,2,3,4,5,5a,6,10b-octahydroazepino[4,5-b]indole
(5aR, 10bS)-3-ethyl-9-[(3,5-difluorophenyl)sulfonyl]-6-methyl-1,2,3,4,5,5a,6,10b-octahydroazepino[4,5-b]indole
(5aR, 10bS)-3-ethyl-6-methyl-9-[(4-methylphenyl)sulfonyl]-1,2,3,4,5,5a,6,10b-octahydroazepino[4,5-b]indole
(5aR, 10bS)-3-ethyl-6-methyl-9-[(3-methylphenyl)sulfonyl]-1,2,3,4,5,5a,6,10b-octahydroazepino[4,5-b]indole
(5aR, 10bS)-3-ethyl-9-[(4-methoxyphenyl)sulfonyl]-6-methyl-1,2,3,4,5,5a,6,10b-octahydroazepino[4,5-b]indole
(5aR, 10bS)-3-ethyl-9-[(3-methoxyphenyl)sulfonyl]-6-methyl-1,2,3,4,5,5a,6,10b-octahydroazepino[4,5-b]indole
(5aR, 10bS)-3-ethyl-6-methyl-9-[[(4-trifluoromethyl)phenyl]sulfonyl]-1,2,3,4,5,5a,6,10b-octahydroazepino[4,5-b]indole
(5aR, 10bS)-1,6-dimethyl-9-(phenylsulfonyl)-1,2,3,4,5,5a,6,10b-octahydroazepino[4,5-b]indole
(5aR, 10bS)-2,6-dimethyl-9-(phenylsulfonyl)-1,2,3,4,5,5a,6,10b-octahydroazepino[4,5-b]indole
(5aR, 10bS)-4,6-dimethyl-9-(phenylsulfonyl)-1,2,3,4,5,5a,6,10b-octahydroazepino[4,5-b]indole
(5aR, 10bS)-5,6-dimethyl-9-(phenylsulfonyl)-1,2,3,4,5,5a,6,10b-octahydroazepino[4,5-b]indole
(5aR, 10bS)-9-[(4-fluorophenyl)sulfonyl]-1,6-dimethyl-1,2,3,4,5,5a,6,10b-octahydroazepino[4,5-b]indole
(5aR, 10bS)-9-[(4-fluorophenyl)sulfonyl]-2,6-dimethyl-1,2,3,4,5,5a,6,10b-octahydroazepino[4,5-b]indole
(5aR, 10bS)-9-[(4-fluorophenyl)sulfonyl]-4,6-dimethyl-1,2,3,4,5,5a,6,10b-octahydroazepino[4,5-b]indole
(5aR, 10bS)-9-[(4-fluorophenyl)sulfonyl]-5,6-dimethyl-1,2,3,4,5,5a,6,10b-octahydroazepino[4,5-b]indole
(5aR, 10bS)-9-[(3-fluorophenyl)sulfonyl]-1,6-dimethyl-1,2,3,4,5,5a,6,10b-octahydroazepino[4,5-b]indole
(5aR, 10bS)-9-[(3-fluorophenyl)sulfonyl]-2,6-dimethyl-1,2,3,4,5,5a,6,10b-octahydroazepino[4,5-b]indole
(5aR, 10bS)-9-[(3-fluorophenyl)sulfonyl]-4,6-dimethyl-1,2,3,4,5,5a,6,10b-octahydroazepino[4,5-b]indole
(5aR, 10bS)-9-[(3-fluorophenyl)sulfonyl]-5,6-dimethyl-1,2,3,4,5,5a,6,10b-octahydroazepino[4,5-b]indole
(5aR, 10bS)-1,5,6-trimethyl-9-(phenylsulfonyl)-1,2,3,4,5,5a,6,10b-octahydroazepino[4,5-b]indole
(5aR, 10bS)-2,4,6-trimethyl-9-(phenylsulfonyl)-1,2,3,4,5,5a,6,10b-octahydroazepino[4,5-b]indole
(5aR, 10bS)-9-[(4-fluorophenyl)sulfonyl]-1,5,6-trimethyl-1,2,3,4,5,5a,6,10b-octahydroazepino[4,5-b]indole
(5aR, 10bS)-9-[(4-fluorophenyl)sulfonyl]-2,4,6-trimethyl-1,2,3,4,5,5a,6,10b-octahydroazepino[4,5-b]indole
(5aR, 10bS)-9-[(3-fluorophenyl)sulfonyl]-1,5,6-trimethyl-1,2,3,4,5,5a,6,10b-octahydroazepino[4,5-b]indole
(5aR, 10bS)-9-[(3-fluorophenyl)sulfonyl]-2,4,6-trimethyl-1,2,3,4,5,5a,6,10b-octahydroazepino[4,5-b]indole
(5aR, 10bS)-6-methyl-9-(phenylsulfonyl)-1,2,3,4,5,5a,6,10b-octahydroazepino[4,5-b]indole
(5aR, 10bS)-9-[(4-fluorophenyl)sulfonyl]-1,2,3,4,5,5a,6,10b-octahydroazepino[4,5-b]indole
(5aR, 10bS)-9-[(3-fluorophenyl)sulfonyl]-1,2,3,4,5,5a,6,10b-octahydroazepino[4,5-b]indole
(5aR, 10bS)-9-[(3,4-difluorophenyl)sulfonyl]-1,2,3,4,5,5a,6,10b-octahydroazepino[4,5-b]indole
(5aR, 10bS)-9-[(3,5-difluorophenyl)sulfonyl]-1,2,3,4,5,5a,6,10b-octahydroazepino[4,5-b]indole
(5aR, 10bS)-9-[(4-methylphenyl)sulfonyl]-1,2,3,4,5,5a,6,10b-octahydroazepino[4,5-b]indole
(5aR, 10bS)-9-[(3-methylphenyl)sulfonyl]-1,2,3,4,5,5a,6,10b-octahydroazepino[4,5-b]indole
(5aR, 10bS)-9-[(4-methoxyphenyl)sulfonyl]-1,2,3,4,5,5a,6,10b-octahydroazepino[4,5-b]indole
(5aR, 10bS)-9-[(3-methoxyphenyl)sulfonyl]-1,2,3,4,5,5a,6,10b-octahydroazepino[4,5-b]indole
(5aR, 10bS)-9-[[(4-trifluoromethyl)phenyl]sulfonyl]-1,2,3,4,5,5a,6,10b-octahydroazepino[4,5-b]indole
(5aR, 10bS)-3-methyl-9-(phenylsulfonyl)-1,2,3,4,5,5a,6,10b-octahydroazepino[4,5-b]indole
(5aR, 10bS)-9-[(4-fluorophenyl)sulfonyl]-3-methyl-1,2,3,4,5,5a,6,10b-octahydroazepino[4,5-b]indole
(5aR, 10bS)-9-[(3-fluorophenyl)sulfonyl]-3-methyl-1,2,3,4,5,5a,6,10b-octahydroazepino[4,5-b]indole
(5aR, 10bS)-9-[(3,4-difluorophenyl)sulfonyl]-3-methyl-1,2,3,4,5,5a,6,10b-octahydroazepino[4,5-b]indole
(5aR, 10bS)-9-[(3,5-difluorophenyl)sulfonyl]-3-methyl-1,2,3,4,5,5a,6,10b-octahydroazepino[4,5-b]indole
(5aR, 10bS)-3-methyl-9-[(4-methylphenyl)sulfonyl]-1,2,3,4,5,5a,6,10b-octahydroazepino[4,5-b]indole
(5aR, 10bS)-3-methyl-9-[(3-methylphenyl)sulfonyl]-1,2,3,4,5,5a,6,10b-octahydroazepino[4,5-b]indole
(5aR, 10bS)-9-[(4-methoxyphenyl)sulfonyl]-3-methyl-1,2,3,4,5,5a,6,10b-octahydroazepino[4,5-b]indole
(5aR, 10bS)-9-[(3-methoxyphenyl)sulfonyl]-3-methyl-1,2,3,4,5,5a,6,10b-octahydroazepino[4,5-b]indole
(5aR, 10bS)-3-methyl-9-[[(4-trifluoromethyl)phenyl]sulfonyl]-1,2,3,4,5,5a,6,10b-octahydroazepino[4,5-b]indole
(5aR, 10bS)-3-ethyl-9-(phenylsulfonyl)-1,2,3,4,5,5a,6,10b-octahydroazepino[4,5-b]indole
(5aR, 10bS)-3-ethyl-9-[(4-fluorophenyl)sulfonyl]-1,2,3,4,5,5a,6,10b-octahydroazepino[4,5-b]indole
(5aR, 10bS)-3-ethyl-9-[(3-fluorophenyl)sulfonyl]-1,2,3,4,5,5a,6,10b-octahydroazepino[4,5-b]indole
(5aR, 10bS)-3-ethyl-9-[(3,4-difluorophenyl)sulfonyl]-1,2,3,4,5,5a,6,10b-octahydroazepino[4,5-b]indole
(5aR, 10bS)-3-ethyl-9-[(3,5-difluorophenyl)sulfonyl]-1,2,3,4,5,5a,6,10b-octahydroazepino[4,5-b]indole
(5aR, 10bS)-3-ethyl-9-[(4-methylphenyl)sulfonyl]-1,2,3,4,5,5a,6,10b-octahydroazepino[4,5-b]indole
(5aR, 10bS)-3-ethyl-9-[(3-methylphenyl)sulfonyl]-1,2,3,4,5,5a,6,10b-octahydroazepino[4,5-b]indole
(5aR, 10bS)-3-ethyl-9-[(4-methoxyphenyl)sulfonyl]-1,2,3,4,5,5a,6,10b-octahydroazepino[4,5-b]indole
(5aR, 10bS)-3-ethyl-9-[(3-methoxyphenyl)sulfonyl]-1,2,3,4,5,5a,6,10b-octahydroazepino[4,5-b]indole
(5aR, 10bS)-3-ethyl-9-[[(4-trifluoromethyl)phenyl]sulfonyl]-1,2,3,4,5,5a,6,10b-octahydroazepino[4,5-b]indole
(5aR, 10bS)-1-methyl-9-(phenylsulfonyl)-1,2,3,4,5,5a,6,10b-octahydroazepino[4,5-b]indole
(5aR, 10bS)-2-methyl-9-(phenylsulfonyl)-1,2,3,4,5,5a,6,10b-octahydroazepino[4,5-b]indole
(5aR, 10bS)-4-methyl-9-(phenylsulfonyl)-1,2,3,4,5,5a,6,10b-octahydroazepino[4,5-b]indole
(5aR, 10bS)-5-methyl-9-(phenylsulfonyl)-1,2,3,4,5,5a,6,10b-octahydroazepino[4,5-b]indole
(5aR, 10bS)-9-[(4-fluorophenyl)sulfonyl]-1-methyl-1,2,3,4,5,5a,6,10b-octahydroazepino[4,5-b]indole
(5aR, 10bS)-9-[(4-fluorophenyl)sulfonyl]-2-methyl-1,2,3,4,5,5a,6,10b-octahydroazepino[4,5-b]indole
(5aR, 10bS)-9-[(4-fluorophenyl)sulfonyl]-4-methyl-1,2,3,4,5,5a,6,10b-octahydroazepino[4,5-b]indole
(5aR, 10bS)-9-[(4-fluorophenyl)sulfonyl]-5-methyl-1,2,3,4,5,5a,6,10b-octahydroazepino[4,5-b]indole
(5aR, 10bS)-9-[(3-fluorophenyl)sulfonyl]-1-methyl-1,2,3,4,5,5a,6,10b-octahydroazepino[4,5-b]indole
(5aR, 10bS)-9-[(3-fluorophenyl)sulfonyl]-2-methyl-1,2,3,4,5,5a,6,10b-octahydroazepino[4,5-b]indole
(5aR, 10bS)-9-[(3-fluorophenyl)sulfonyl]-4-methyl-1,2,3,4,5,5a,6,10b-octahydroazepino[4,5-b]indole
(5aR, 10bS)-9-[(3-fluorophenyl)sulfonyl]-5-methyl-1,2,3,4,5,5a,6,10b-octahydroazepino[4,5-b]indole
(5aR, 10bS)-1,5-dimethyl-9-(phenylsulfonyl)-1,2,3,4,5,5a,6,10b-octahydroazepino[4,5-b]indole
(5aR, 10bS)-2,4-dimethyl-9-(phenylsulfonyl)-1,2,3,4,5,5a,6,10b-octahydroazepino[4,5-b]indole
(5aR, 10bS)-9-[(4-fluorophenyl)sulfonyl]-1,5-dimethyl-1,2,3,4,5,5a,6,10b-octahydroazepino[4,5-b]indole
(5aR, 10bS)-9-[(4-fluorophenyl)sulfonyl]-2,4-dimethyl-1,2,3,4,5,5a,6,10b-octahydroazepino[4,5-b]indole
(5aR, 10bS)-9-[(3-fluorophenyl)sulfonyl]-1,5-dimethyl-1,2,3,4,5,5a,6,10b-octahydroazepino[4,5-b]indole
(5aR, 10bS)-9-[(3-fluorophenyl)sulfonyl]-2,4-dimethyl-1,2,3,4,5,5a,6,10b-octahydroazepino[4,5-b]indole
(5aS, 10bR)-9-[(4-fluorophenyl)sulfonyl]-6-methyl-1,2,3,4,5,5a,6,10b-octahydroazepino[4,5-b]indole
(5aS, 10bR)-9-[(3-fluorophenyl)sulfonyl]-6-methyl-1,2,3,4,5,5a,6,10b-octahydroazepino[4,5-b]indole
(5aS, 10bR)-9-[(3,4-difluorophenyl)sulfonyl]-6-methyl-1,2,3,4,5,5a,6,10b-octahydroazepino[4,5-b]indole
(5aS, 10bR)-9-[(3,5-difluorophenyl)sulfonyl]-6-methyl-1,2,3,4,5,5a,6,10b-octahydroazepino[4,5-b]indole
(5aS, 10bR)-6-methyl-9-[(4-methylphenyl)sulfonyl]-1,2,3,4,5,5a,6,10b-octahydroazepino[4,5-b]indole
(5aS, 10bR)-6-methyl-9-[(3-methylphenyl)sulfonyl]-1,2,3,4,5,5a,6,10b-octahydroazepino[4,5-b]indole
(5aS, 10bR)-9-[(4-methoxyphenyl)sulfonyl]-6-methyl-1,2,3,4,5,5a,6,10b-octahydroazepino[4,5-b]indole
(5aS, 10bR)-9-[(3-methoxyphenyl)sulfonyl]-6-methyl-1,2,3,4,5,5a,6,10b-octahydroazepino[4,5-b]indole
(5aS, 10bR)-6-methyl-9-[[(4-trifluoromethyl)phenyl]sulfonyl]-1,2,3,4,5,5a,6,10b-octahydroazepino[4,5-b]indole
(5aS, 10bR)-3,6-dimethyl-9-(phenylsulfonyl)-1,2,3,4,5,5a,6,10b-octahydroazepino[4,5-b]indole
(5aS, 10bR)-9-[(4-fluorophenyl)sulfonyl]-3,6-dimethyl-1,2,3,4,5,5a,6,10b-octahydroazepino [4,5-b]indole
(5aS, 10bR)-9-[(3-fluorophenyl)sulfonyl]-3,6-dimethyl-1,2,3,4,5,5a,6,10b-octahydroazepino[4,5-b]indole
(5aS, 10bR)-9-[(3,4-difluorophenyl)sulfonyl]-3,6-dimethyl-1,2,3,4,5,5a,6,10b-octahydroazepino[4,5-b]indole
(5aS, 10bR)-9-[(3,5-difluorophenyl)sulfonyl]-3,6-dimethyl-1,2,3,4,5,5a,6,10b-octahydroazepino[4,5-b]indole
(5aS, 10bR)-3,6-dimethyl-9-[(4-methylphenyl)sulfonyl]-1,2,3,4,5,5a,6,10b-octahydroazepino[4,5-b]indole
(5aS, 10bR)-3,6-dimethyl-9-[(3-methylphenyl)sulfonyl]-1,2,3,4,5,5a,6,10b-octahydroazepino[4,5-b]indole
(5aS, 10bR)-9-[(4-methoxyphenyl)sulfonyl]-3,6-dimethyl-1,2,3,4,5,5a,6,10b-octahydroazepino[4,5-b]indole
(5aS, 10bR)-9-[(3-methoxyphenyl)sulfonyl]-3,6-dimethyl-1,2,3,4,5,5a,6,10b-octahydroazepino[4,5-b]indole
(5aS, 10bR)-3,6-dimethyl-9-[[(4-trifluoromethyl)phenyl]sulfonyl]-1,2,3,4,5,5a,6,10b-octahydroazepino[4,5-b]indole
(5aS, 10bR)-3-ethyl-6-methyl-9-(phenylsulfonyl)-1,2,3,4,5,5a,6,10b-octahydroazepino[4,5-b]indole
(5aS, 10bR)-3-ethyl-9-[(4-fluorophenyl)sulfonyl]-6-methyl-1,2,3,4,5,5a,6,10b-octahydroazepino[4,5-b]indole
(5aS, 10bR)-3-ethyl-9-[(3-fluorophenyl)sulfonyl]-6-methyl-1,2,3,4,5,5a,6,10b-octahydroazepino[4,5-b]indole
(5aS, 10bR)-3-ethyl-9-[(3,4-difluorophenyl)sulfonyl]-6-methyl-1,2,3,4,5,5a,6,10b-octahydroazepino[4,5-b]indole
(5aS, 10bR)-3-ethyl-9-[(3,5-difluorophenyl)sulfonyl]-6-methyl-1,2,3,4,5,5a,6,10b-octahydroazepino[4,5-b]indole
(5aS, 10bR)-3-ethyl-6-methyl-9-[(4-methylphenyl)sulfonyl]-1,2,3,4,5,5a,6,10b-octahydroazepino[4,5-b]indole
(5aS, 10bR)-3-ethyl-6-methyl-9-[(3-methylphenyl)sulfonyl]-1,2,3,4,5,5a,6,10b-octahydroazepino[4,5-b]indole
(5aS, 10bR)-3-ethyl-9-[(4-methoxyphenyl)sulfonyl]-6-methyl-1,2,3,4,5,5a,6,10b-octahydroazepino[4,5-b]indole
(5aS, 10bR)-3-ethyl-9-[(3-methoxyphenyl)sulfonyl]-6-methyl-1,2,3,4,5,5a,6,10b-octahydroazepino [4,5-b] indole
(5aS, 10bR)-3-ethyl-6-methyl-9-[[(4-trifluoromethyl)phenyl]sulfonyl]-1,2,3,4,5,5a,6,10b-octahydroazepino[4,5-b]indole
(5aS, 10bR)-1,6-dimethyl-9-(phenylsulfonyl)-1,2,3,4,5,5a,6,10b-octahydroazepino[4,5-b]indole
(5aS, 10bR)-2,6-dimethyl-9-(phenylsulfonyl)-1,2,3,4,5,5a,6,10b-octahydroazepino[4,5-b]indole
(5aS, 10bR)-4,6-dimethyl-9-(phenylsulfonyl)-1,2,3,4,5,5a,6,10b-octahydroazepino[4,5-b]indole
(5aS, 10bR)-5,6-dimethyl-9-(phenylsulfonyl)-1,2,3,4,5,5a,6,10b-octahydroazepino[4,5-b]indole
(5aS, 10bR)-9-[(4-fluorophenyl)sulfonyl]-1,6-dimethyl-1,2,3,4,5,5a,6,10b-octahydroazepino[4,5-b]indole
(5aS, 10bR)-9-[(4-fluorophenyl)sulfonyl]-2,6-dimethyl-1,2,3,4,5,5a,6,10b-octahydroazepino[4,5-b]indole
(5aS, 10bR)-9-[(4-fluorophenyl)sulfonyl]-4,6-dimethyl-1,2,3,4,5,5a,6,10b-octahydroazepino[4,5-b]indole
(5aS, 10bR)-9-[(4-fluorophenyl)sulfonyl]-5,6-dimethyl-1,2,3,4,5,5a,6,10b-octahydroazepino[4,5-b]indole
(5aS, 10bR)-9-[(3-fluorophenyl)sulfonyl]-1,6-dimethyl-1,2,3,4,5,5a,6,10b-octahydroazepino[4,5-b]indole
(5aS, 10bR)-9-[(3-fluorophenyl)sulfonyl]-2,6-dimethyl-1,2,3,4,5,5a,6,10b-octahydroazepino[4,5-b]indole
(5aS, 10bR)-9-[(3-fluorophenyl)sulfonyl]-4,6-dimethyl-1,2,3,4,5,5a,6,10b-octahydroazepino[4,5-b]indole
(5aS, 10bR)-9-[(3-fluorophenyl)sulfonyl]-5,6-dimethyl-1,2,3,4,5,5a,6,10b-octahydroazepino[4,5-b]indole
(5aS, 10bR)-1,5,6-trimethyl-9-(phenylsulfonyl)-1,2,3,4,5,5a,6,10b-octahydroazepino[4,5-b]indole
(5aS, 10bR)-2,4,6-trimethyl-9-(phenylsulfonyl)-1,2,3,4,5,5a,6,10b-octahydroazepino[4,5-b]indole
(5aS, 10bR)-9-[(4-fluorophenyl)sulfonyl]-1,5,6-trimethyl-1,2,3,4,5,5a,6,10b-octahydroazepino[4,5-b]indole
(5aS, 10bR)-9-[(4-fluorophenyl)sulfonyl]-2,4,6-trimethyl-1,2,3,4,5,5a,6,10b-octahydroazepino[4,5-b]indole
(5aS, 10bR)-9-[(3-fluorophenyl)sulfonyl]-1,5,6-trimethyl-1,2,3,4,5,5a-6,10b-octahydroazepino[4,5-b]indole
(5aS, 10bR)-9-[(3-fluorophenyl)sulfonyl]-2,4,6-trimethyl-1,2,3,4,5,5a,6,10b-octahydroazepino[4,5-b]indole
(5aS, 10bR)-6-methyl-9-(phenylsulfonyl)-1,2,3,4,5,5a,6,10b-octahydroazepino[4,5-b]indole
(5aS, 10bR)-9-[(4-fluorophenyl)sulfonyl]-1,2,3,4,5,5a,6,10b-octahydroazepino[4,5-b]indole
(5aS, 10bR)-9-[(3-fluorophenyl)sulfonyl]-1,2,3,4,5,5a,6,10b-octahydroazepino[4,5-b]indole
(5aS, 10bR)-9-[(3,4-difluorophenyl)sulfonyl]-1,2,3,4,5,5a,6,10b-octahydroazepino[4,5-b]indole
(5aS, 10bR)-9-[(3,5-difluorophenyl)sulfonyl]-1,2,3,4,5,5a,6,10b-octahydroazepino[4,5-b]indole
(5aS, 10bR)-9-[(4-methylphenyl)sulfonyl]-1,2,3,4,5,5a,6,10b-octahydroazepino[4,5-b]indole
(5aS, 10bR)-9-[(3-methylphenyl)sulfonyl]-1,2,3,4,5,5a,6,10b-octahydroazepino[4,5-b]indole
(5aS, 10bR)-9-[(4-methoxyphenyl)sulfonyl]-1,2,3,4,5,5a,6,10b-octahydroazepino[4,5-b]indole
(5aS, 10bR)-9-[(3-methoxyphenyl)sulfonyl]-1,2,3,4,5,5a,6,10b-octahydroazepino[4,5-b]indole
(5aS, 10bR)-9-[[(4-trifluoromethyl)phenyl]sulfonyl]-1,2,3,4,5,5a,6,10b-octahydroazepino[4,5-b]indole
(5aS, 10bR)-3-methyl-9-(phenylsulfonyl)-1,2,3,4,5,5a,6,10b-octahydroazepino[4,5-b]indole
(5aS, 10bR)-9-[(4-fluorophenyl)sulfonyl]-3-methyl-1,2,3,4,5,5a,6,10b-octahydroazepino[4,5-b]indole
(5aS, 10bR)-9-[(3-fluorophenyl)sulfonyl]-3-methyl-1,2,3,4,5,5a,6,10b-octahydroazepino[4,5-b]indole
(5aS, 10bR)-9-[(3,4-difluorophenyl)sulfonyl]-3-methyl-1,2,3,4,5,5a,6,10b-octahydroazepino[4,5-b]indole
(5aS, 10bR)-9-[(3,5-difluorophenyl)sulfonyl]-3-methyl-1,2,3,4,5,5a,6,10b-octahydroazepino[4,5-b]indole
(5aS, 10bR)-3-methyl-9-[(4-methylphenyl)sulfonyl]-1,2,3,4,5,5a,6,10b-octahydroazepino[4,5-b]indole
(5aS, 10bR)-3-methyl-9-[(3-methylphenyl)sulfonyl]-1,2,3,4,5,5a,6,10b-octahydroazepino[4,5-b]indole
(5aS, 10bR)-9-[(4-methoxyphenyl)sulfonyl]-3-methyl-1,2,3,4,5,5a,6,10b-octahydroazepino[4,5-b]indole
(5aS, 10bR)-9-[(3-methoxyphenyl)sulfonyl]-3-methyl-1,2,3,4,5,5a,6,10b-octahydroazepino[4,5-b]indole
(5aS, 10bR)-3-methyl-9-[[(4-trifluoromethyl)phenyl]sulfonyl]-1,2,3,4,5,5a,6,10b-octahydroazepino[4,5-b]indole
(5aS, 10bR)-3-ethyl-9-(phenylsulfonyl)-1,2,3,4,5,5a,6,10b-octahydroazepino[4,5-b]indole
(5aS, 10bR)-3-ethyl-9-[(4-fluorophenyl)sulfonyl]-1,2,3,4,5,5a,6,10b-octahydroazepino[4,5-b]indole
(5aS, 10bR)-3-ethyl-9-[(3-fluorophenyl)sulfonyl]-1,2,3,4,5,5a,6,10b-octahydroazepino[4,5-b]indole
(5aS, 10bR)-3-ethyl-9-[(3,4-difluorophenyl)sulfonyl]-1,2,3,4,5,5a,6,10b-octahydroazepino[4,5-b]indole
(5aS, 10bR)-3-ethyl-9-[(3,5-difluorophenyl)sulfonyl]-1,2,3,4,5,5a,6,10b-octahydroazepino[4,5-b]indole
(5aS, 10bR)-3-ethyl-9-[(4-methylphenyl)sulfonyl]-1,2,3,4,5,5a,6,10b-octahydroazepino[4,5-b]indole
(5aS, 10bR)-3-ethyl-9-[(3-methylphenyl)sulfonyl]-1,2,3,4,5,5a,6,10b-octahydroazepino[4,5-b]indole
(5aS, 10bR)-3-ethyl-9-[(4-methoxyphenyl)sulfonyl]-1,2,3,4,5,5a,6,10b-octahydroazepino[4,5-b]indole
(5aS, 10bR)-3-ethyl-9-[(3-methoxyphenyl)sulfonyl]-1,2,3,4,5,5a,6,10b-octahydroazepino[4,5-b]indole
(5aS, 10bR)-3-ethyl-9-[[(4-trifluoromethyl)phenyl]sulfonyl]-1,2,3,4,5,5a,6,10b-octahydroazepino[4,5-b]indole
(5aS, 10bR)-1-methyl-9-(phenylsulfonyl)-1,2,3,4,5,5a,6,10b-octahydroazepino[4,5-b]indole
(5aS, 10bR)-2-methyl-9-(phenylsulfonyl)-1,2,3,4,5,5a,6,10b-octahydroazepino[4,5-b]indole
(5aS, 10bR)-4-methyl-9-(phenylsulfonyl)-1,2,3,4,5,5a,6,10b-octahydroazepino[4,5-b]indole
(5aS, 10bR)-5-methyl-9-(phenylsulfonyl)-1,2,3,4,5,5a,6,10b-octahydroazepino[4,5-b]indole
(5aS, 10bR)-9-[(4-fluorophenyl)sulfonyl]-1-methyl-1,2,3,4,5,5a,6,10b-octahydroazepino[4,5-b]indole
(5aS, 10bR)-9-[(4-fluorophenyl)sulfonyl]-2-methyl-1,2,3,4,5,5a,6,10b-octahydroazepino[4,5-b]indole
(5aS, 10bR)-9-[(4-fluorophenyl)sulfonyl]-4-methyl-1,2,3,4,5,5a,6,10b-octahydroazepino[4,5-b]indole
(5aS, 10bR)-9-[(4-fluorophenyl)sulfonyl]-5-methyl-1,2,3,4,5,5a,6,10b-octahydroazepino[4,5-b]indole
(5aS, 10bR)-9-[(3-fluorophenyl)sulfonyl]-1-methyl-1,2,3,4,5,5a,6,10b-octahydroazepino[4,5-b]indole
(5aS, 10bR)-9-[(3-fluorophenyl)sulfonyl]-2-methyl-1,2,3,4,5,5a,6,10b-octahydroazepino [4,5-b]indole
(5aS, 10bR)-9-[(3-fluorophenyl)sulfonyl]-4-methyl-1,2,3,4,5,5a,6,10b-octahydroazepino[4,5-b]indole
(5aS, 10bR)-9-[(3-fluorophenyl)sulfonyl]-5-methyl-1,2,3,4,5,5a,6,10b-octahydroazepino[4,5-b]indole
(5aS, 10bR)-1,5-dimethyl-9-(phenylsulfonyl)-1,2,3,4,5,5a,6,10b-octahydroazepino[4,5-b]indole
(5aS, 10bR)-2,4-dimethyl-9-(phenylsulfonyl)-1,2,3,4,5,5a,6,10b-octahydroazepino[4,5-b]indole
(5aS, 10bR)-9-[(4-fluorophenyl)sulfonyl]-1,5-dimethyl-1,2,3,4,5,5a,6,10b-octahydroazepino[4,5-b]indole
(5aS, 10bR)-9-[(4-fluorophenyl)sulfonyl]-2,4-dimethyl-1,2,3,4,5,5a,6,10b-octahydroazepino[4,5-b]indole
(5aS, 10bR)-9-[(3-fluorophenyl)sulfonyl]-1,5-dimethyl-1,2,3,4,5,5a,6,10b-octahydroazepino[4,5-b]indole
(5aS, 10bR)-9-[(3-fluorophenyl)sulfonyl]-2,4-dimethyl-1,2,3,4,5,5a,6,10b-octahydroazepino[4,5-b]indole
(5aR, 10bR)-9-[(4-fluorophenyl)sulfonyl]-6-methyl-1,2,3,4,5,5a,6,10b-octahydroazepino[4,5-b]indole
(5aR, 10bR)-9-[(3-fluorophenyl)sulfonyl]-6-methyl-1,2,3,4,5,5a,6,10b-octahydroazepino[4,5-b]indole
(5aR, 10bR)-9-[(3,4-difluorophenyl)sulfonyl]-6-methyl-1,2,3,4,5,5a,6,10b-octahydroazepino[4,5-b]indole
(5aR, 10bR)-9-[(3,5-difluorophenyl)sulfonyl]-6-methyl-1,2,3,4,5,5a,6,10b-octahydroazepino[4,5-b]indole
(5aR, 10bR)-6-methyl-9-[(4-methylphenyl)sulfonyl]-1,2,3,4,5,5a,6,10b-octahydroazepino[4,5-b]indole
(5aR, 10bR)-6-methyl-9-[(3-methylphenyl)sulfonyl]-1,2,3,4,5,5a,6,10b-octahydroazepino[4,5-b]indole
(5aR, 10bR)-9-[(4-methoxyphenyl)sulfonyl]-6-methyl-1,2,3,4,5,5a,6,10b-octahydroazepino[4,5-b]indole
(5aR, 10bR)-9-[(3-methoxyphenyl)sulfonyl]-6-methyl-1,2,3,4,5,5a,6,10b-octahydroazepino[4,5-b]indole
(5aR, 10bR)-6-methyl-9-[[(4-trifluoromethyl)phenyl]sulfonyl]-1,2,3,4,5,5a,6,10b-octahydroazepino[4,5-b]indole
(5aR, 10bR)-3,6-dimethyl-9-(phenylsulfonyl)-1,2,3,4,5,5a,6,10b-octahydroazepino[4,5-b]indole
(5aR, 10bR)-9-[(4-fluorophenyl)sulfonyl]-3,6-dimethyl-1,2,3,4,5,5a,6,10b-octahydroazepino[4,5-b]indole
(5aR, 10bR)-9-[(3-fluorophenyl)sulfonyl]-3,6-dimethyl-1,2,3,4,5,5a,6,10b-octahydroazepino[4,5-b]indole
(5aR, 10bR)-9-[(3,4-difluorophenyl)sulfonyl]-3,6-dimethyl-1,2,3,4,5,5a,6,10b-octahydroazepino[4,5-b]indole
(5aR, 10bR)-9-[(3,5-difluorophenyl)sulfonyl]-3,6-dimethyl-1,2,3,4,5,5a,6,10b-octahydroazepino[4,5-b]indole
(5aR, 10bR)-3,6-dimethyl-9-[(4-methylphenyl)sulfonyl]-1,2,3,4,5,5a,6,10b-octahydroazepino[4,5-b]indole
(5aR, 10bR)-3,6-dimethyl-9-[(3-methylphenyl)sulfonyl]-1,2,3,4,5,5a,6,10b-octahydroazepino[4,5-b]indole
(5aR, 10bR)-9-[(4-methoxyphenyl)sulfonyl]-3,6-dimethyl-1,2,3,4,5,5a,6,10b-octahydroazepino[4,5-b]indole
(5aR, 10bR)-9-[(3-methoxyphenyl)sulfonyl]-3,6-dimethyl-1,2,3,4,5,5a,6,10b-octahydroazepino[4,5-b]indole
(5aR, 10bR)-3,6-dimethyl-9-[[(4-trifluoromethyl)phenyl]sulfonyl]-1,2,3,4,5,5a,6,10b-octahydroazepino[4,5-b]indole
(5aR, 10bR)-3-ethyl-6-methyl-9-(phenylsulfonyl)-1,2,3,4,5,5a,6,10b-octahydroazepino[4,5-b]indole
(5aR, 10bR)-3-ethyl-9-[(4-fluorophenyl)sulfonyl]-6-methyl-1,2,3,4,5,5a,6,10b-octahydroazepino[4,5-b]indole
(5aR, 10bR)-3-ethyl-9-[(3-fluorophenyl)sulfonyl]-6-methyl-1,2,3,4,5,5a,6,10b-octahydroazepino[4,5-b]indole
(5aR, 10bR)-3-ethyl-9-[(3,4-difluorophenyl)sulfonyl]-6-methyl-1,2,3,4,5,5a,6,10b-octahydroazepino[4,5-b]indole
(5aR, 10bR)-3-ethyl-9-[(3,5-difluorophenyl)sulfonyl]-6-methyl-1,2,3,4,5,5a,6,10b-octahydroazepino[4,5-b]indole
(5aR, 10bR)-3-ethyl-6-methyl-9-[(4-methylphenyl)sulfonyl]-1,2,3,4,5,5a,6,10b-octahydroazepino[4,5-b]indole
(5aR, 10bR)-3-ethyl-6-methyl-9-[(3-methylphenyl)sulfonyl]-1,2,3,4,5,5a,6,10b-octahydroazepino[4,5-b]indole
(5aR, 10bR)-3-ethyl-9-[(4-methoxyphenyl)sulfonyl]-6-methyl-1,2,3,4,5,5a,6,10b-octahydroazepino[4,5-b]indole
(5aR, 10bR)-3-ethyl-9-[(3-methoxyphenyl)sulfonyl]-6-methyl-1,2,3,4,5,5a,6,10b-octahydroazepino[4,5-b]indole
(5aR, 10bR)-3-ethyl-6-methyl-9-[[(4-trifluoromethyl)phenyl]sulfonyl]-1,2,3,4,5,5a,6,10b-octahydroazepino[4,5-b]indole
(5aR, 10bR)-1,6-dimethyl-9-(phenylsulfonyl)-1,2,3,4,5,5a,6,10b-octahydroazepino[4,5-b]indole
(5aR, 10bR)-2,6-dimethyl-9-(phenylsulfonyl)-1,2,3,4,5,5a,6,10b-octahydroazepino[4,5-b]indole
(5aR, 10bR)-4,6-dimethyl-9-(phenylsulfonyl)-1,2,3,4,5,5a,6,10b-octahydroazepino[4,5-b]indole
(5aR, 10bR)-5,6-dimethyl-9-(phenylsulfonyl)-1,2,3,4,5,5a,6,10b-octahydroazepino[4,5-b]indole
(5aR, 10bR)-9-[(4-fluorophenyl)sulfonyl]-1,6-dimethyl-1,2,3,4,5,5a,6,10b-octahydroazepino[4,5-b]indole
(5aR, 10bR)-9-[(4-fluorophenyl)sulfonyl]-2,6-dimethyl-1,2,3,4,5,5a,6,10b-octahydroazepino[4,5-b]indole
(5aR, 10bR)-9-[(4-fluorophenyl)sulfonyl]-4,6-dimethyl-1,2,3,4,5,5a,6,10b-octahydroazepino[4,5-b]indole
(5aR, 10bR)-9-[(4-fluorophenyl)sulfonyl]-5,6-dimethyl-1,2,3,4,5,5a,6,10b-octahydroazepino[4,5-b]indole
(5aR, 10bR)-9-[(3-fluorophenyl)sulfonyl]-1,6-dimethyl-1,2,3,4,5,5a,6,10b-octahydroazepino[4,5-b]indole
(5aR, 10bR)-9-[(3-fluorophenyl)sulfonyl]-2,6-dimethyl-1,2,3,4,5,5a,6,10b-octahydroazepino[4,5-b]indole
(5aR, 10bR)-9-[(3-fluorophenyl)sulfonyl]-4,6-dimethyl-1,2,3,4,5,5a,6,10b-octahydroazepino[4,5-b]indole
(5aR, 10bR)-9-[(3-fluorophenyl)sulfonyl]-5,6-dimethyl-1,2,3,4,5,5a,6,10b-octahydroazepino[4,5-b]indole
(5aR, 10bR)-1,5,6-trimethyl-9-(phenylsulfonyl)-1,2,3,4,5,5a,6,10b-octahydroazepino[4,5-b]indole
(5aR, 10bR)-2,4,6-trimethyl-9-(phenylsulfonyl)-1,2,3,4,5,5a,6,10b-octahydroazepino[4,5-b]indole
(5aR, 10bR)-9-[(4-fluorophenyl)sulfonyl]-1,5,6-trimethyl-1,2,3,4,5,5a,6,10b-octahydroazepino[4,5-b]indole
(5aR, 10bR)-9-[(4-fluorophenyl)sulfonyl]-2,4,6-trimethyl-1,2,3,4,5,5a,6,10b-octahydroazepino[4,5-b]indole
(5aR, 10bR)-9-[(3-fluorophenyl)sulfonyl]-1,5,6-trimethyl-1,2,3,4,5,5a,6,10b-octahydroazepino[4,5-b]indole
(5aR, 10bR)-9-[(3-fluorophenyl)sulfonyl]-2,4,6-trimethyl-1,2,3,4,5,5a,6,10b-octahydroazepino[4,5-b]indole
(5aR, 10bR)-6-methyl-9-(phenylsulfonyl)-1,2,3,4,5,5a,6,10b-octahydroazepino[4,5-b]indole
(5aR, 10bR)-9-[(4-fluorophenyl)sulfonyl]-1,2,3,4,5,5a,6,10b-octahydroazepino[4,5-b]indole
(5aR, 10bR)-9-[(3-fluorophenyl)sulfonyl]-1,2,3,4,5,5a,6,10b-octahydroazepino[4,5-b]indole
(5aR, 10bR)-9-[(3,4-difluorophenyl)sulfonyl]-1,2,3,4,5,5a,6,10b-octahydroazepino[4,5-b]indole
(5aR, 10bR)-9-[(3,5-difluorophenyl)sulfonyl]-1,2,3,4,5,5a,6,10b-octahydroazepino[4,5-b]indole
(5aR, 10bR)-9-[(4-methylphenyl)sulfonyl]-1,2,3,4,5,5a,6,10b-octahydroazepino[4,5-b]indole
(5aR, 10bR)-9-[(3-methylphenyl)sulfonyl]-1,2,3,4,5,5a,6,10b-octahydroazepino[4,5-b]indole
(5aR, 10bR)-9-[(4-methoxyphenyl)sulfonyl]-1,2,3,4,5,5a,6,10b-octahydroazepino[4,5-b]indole
(5aR, 10bR)-9-[(3-methoxyphenyl)sulfonyl]-1,2,3,4,5,5a,6,10b-octahydroazepino[4,5-b]indole
(5aR, 10bR)-9-[[(4-trifluoromethyl)phenyl]sulfonyl]-1,2,3,4,5,5a,6,10b-octahydroazepino[4,5-b]indole
(5aR, 10bR)-3-methyl-9-(phenylsulfonyl)-1,2,3,4,5,5a,6,10b-octahydroazepino[4,5-b]indole
(5aR, 10bR)-9-[(4-fluorophenyl)sulfonyl]-3-methyl-1,2,3,4,5,5a,6,10b-octahydroazepino[4,5-b]indole
(5aR, 10bR)-9-[(3-fluorophenyl)sulfonyl]-3-methyl-1,2,3,4,5,5a,6,10b-octahydroazepino[4,5-b]indole
(5aR, 10bR)-9-[(3,4-difluorophenyl)sulfonyl]-3-methyl-1,2,3,4,5,5a,6,10b-octahydroazepino[4,5-b]indole
(5aR, 10bR)-9-[(3,5-difluorophenyl)sulfonyl]-3-methyl-1,2,3,4,5,5a,6,10b-octahydroazepino[4,5-b]indole
(5aR, 10bR)-3-methyl-9-[(4-methylphenyl)sulfonyl]-1,2,3,4,5,5a,6,10b-octahydroazepino[4,5-b]indole
(5aR, 10bR)-3-methyl-9-[(3-methylphenyl)sulfonyl]-1,2,3,4,5,5a,6,10b-octahydroazepino[4,5-b]indole
(5aR, 10bR)-9-[(4-methoxyphenyl)sulfonyl]-3-methyl-1,2,3,4,5,5a,6,10b-octahydroazepino[4,5-b]indole
(5aR, 10bR)-9-[(3-methoxyphenyl)sulfonyl]-3-methyl-1,2,3,4,5,5a,6,10b-octahydroazepino[4,5-b]indole
(5aR, 10bR)-3-methyl-9-[[(4-trifluoromethyl)phenyl]sulfonyl]-1,2,3,4,5,5a,6,10b-octahydroazepino[4,5-b]indole
(5aR, 10bR)-3-ethyl-9-(phenylsulfonyl)-1,2,3,4,5,5a,6,10b-octahydroazepino[4,5-b]indole
(5aR, 10bR)-3-ethyl-9-[(4-fluorophenyl)sulfonyl]-1,2,3,4,5,5a,6,10b-octahydroazepino[4,5-b]indole
(5aR, 10bR)-3-ethyl-9-[(3-fluorophenyl)sulfonyl]-1,2,3,4,5,5a,6,10b-octahydroazepino[4,5-b]indole
(5aR, 10bR)-3-ethyl-9-[(3,4-difluorophenyl)sulfonyl]-1,2,3,4,5,5a,6,10b-octahydroazepino[4,5-b]indole
(5aR, 10bR)-3-ethyl-9-[(3,5-difluorophenyl)sulfonyl]-1,2,3,4,5,5a,6,10b-octahydroazepino[4,5-b]indole
(5aR, 10bR)-3-ethyl-9-[(4-methylphenyl)sulfonyl]-1,2,3,4,5,5a,6,10b-octahydroazepino[4,5-b]indole
(5aR, 10bR)-3-ethyl-9-[(3-methylphenyl)sulfonyl]-1,2,3,4,5,5a,6,10b-octahydroazepino[4,5-b]indole
(5aR, 10bR)-3-ethyl-9-[(4-methoxyphenyl)sulfonyl]-1,2,3,4,5,5a,6,10b-octahydroazepino[4,5-b]indole
(5aR, 10bR)-3-ethyl-9-[(3-methoxyphenyl)sulfonyl]-1,2,3,4,5,5a,6,10b-octahydroazepino[4,5-b]indole
(5aR, 10bR)-3-ethyl-9-[[(4-trifluoromethyl)phenyl]sulfonyl]-1,2,3,4,5,5a,6,10b-octahydroazepino[4,5-b]indole
(5aR, 10bR)-1-methyl-9-(phenylsulfonyl)-1,2,3,4,5,5a,6,10b-octahydroazepino[4,5-b]indole
(5aR, 10bR)-2-methyl-9-(phenylsulfonyl)-1,2,3,4,5,5a,6,10b-octahydroazepino[4,5-b]indole
(5aR, 10bR)-4-methyl-9-(phenylsulfonyl)-1,2,3,4,5,5a,6,10b-octahydroazepino[4,5-b]indole
(5aR, 10bR)-5-methyl-9-(phenylsulfonyl)-1,2,3,4,5,5a,6,10b-octahydroazepino[4,5-b]indole
(5aR, 10bR)-9-[(4-fluorophenyl)sulfonyl]-1-methyl-1,2,3,4,5,5a,6,10b-octahydroazepino[4,5-b]indole
(5aR, 10bR)-9-[(4-fluorophenyl)sulfonyl]-2-methyl-1,2,3,4,5,5a,6,10b-octahydroazepino[4,5-b]indole
(5aR, 10bR)-9-[(4-fluorophenyl)sulfonyl]-4-methyl-1,2,3,4,5,5a,6,10b-octahydroazepino[4,5-b]indole
(5aR, 10bR)-9-[(4-fluorophenyl)sulfonyl]-5-methyl-1,2,3,4,5,5a,6,10b-octahydroazepino[4,5-b]indole
(5aR, 10bR)-9-[(3-fluorophenyl)sulfonyl]-1-methyl-1,2,3,4,5,5a,6,10b-octahydroazepino[4,5-b]indole
(5aR, 10bR)-9-[(3-fluorophenyl)sulfonyl]-2-methyl-1,2,3,4,5,5a,6,10b-octahydroazepino[4,5-b]indole
(5aR, 10bR)-9-[(3-fluorophenyl)sulfonyl]-4-methyl-1,2,3,4,5,5a,6,10b-octahydroazepino[4,5-b]indole
(5aR, 10bR)-9-[(3-fluorophenyl)sulfonyl]-5-methyl-1,2,3,4,5,5a,6,10b-octahydroazepino[4,5-b]indole
(5aR, 10bR)-1,5-dimethyl-9-(phenylsulfonyl)-1,2,3,4,5,5a,6,10b-octahydroazepino[4,5-b]indole
(5aR, 10bR)-2,4-dimethyl-9-(phenylsulfonyl)-1,2,3,4,5,5a,6,10b-octahydroazepino[4,5-b]indole
(5aR, 10bR)-9-[(4-fluorophenyl)sulfonyl]-1,5-dimethyl-1,2,3,4,5,5a,6,10b-octahydroazepino[4,5-b]indole
(5aR, 10bR)-9-[(9-fluorophenyl)sulfonyl]-2,4-dimethyl-1,2,3,4,5,5a,6,10b-octahydroazepino[4,5-b]indole
(5aR, 10bR)-9-[(3-fluorophenyl)sulfonyl]-1,5-dimethyl-1,2,3,4,5,5a,6,10b-octahydroazepino[4,5-b]indole, and
(5aR, 10bR)-9-[(3-fluorophenyl)sulfonyl]-2,4-dimethyl-1,2,3,4,5,5a,6,10b-octahydroazepino[4,5-b]indole.

8. A compound according to claim 1, which includes an isotopic label.

9. A compound according to claim 8, which is labelled 6-methyl-1,2,3,4,5,5a,6,10b-octahydroazepino[4,5-b]indol-9-ylphenyl sulfone or 6-methyl-1,2,3,4,5,5a,6,10b-octahydroazepino[4,5-b]indol-9-yl-4-fluorophenyl sulfone, or a pharmaceutically acceptable salt thereof.

10. A compound according to claim 8, wherein the isotope is selected from ³H, ¹¹C, ¹⁴C, ¹³N, ¹⁵O, ¹⁸F, ^{99m}Tc, ¹²³I and ¹²⁵I.

11. A compound according to claim 10, wherein the isotope is selected from ¹¹C, ¹³N, ¹⁵O and ¹⁸F.

12. A compound according to claims 9 and 11, for use in performing positron emission tomography.

13. A compound according to claim 9, which is 6-methyl-1,2,3,4,5,5a,6,10b-octahydroazepino[4,5-b]indol-9-yl-4-fluorophenyl sulfone or a pharmaceutically acceptable salt thereof, which includes ¹⁹F.

14. Use of a compound according to claim 1, for the manufacture of a medicament for treating a disease or disorder in a mammal wherein the 5-HT receptor is implicated and modulation of 5-HT function is desired.

15. The use of claim 14, wherein the disease or disorder is anxiety, depression, schizophrenia, a stress-related disease, panic disorder, a phobia, obsessive compulsive disorder, post-traumatic stress syndrome, immune system depression, psychosis, paraphrenia, mania, a convulsive disorder, a personality disorder, migraine headache, drug addiction, alcoholism, obesity, an eating disorder or a sleep disorder.

16. The use of claim 15, wherein the disease or disorder is anxiety, obesity, depression or a stress-related disease.

17. The use of claim 14, wherein the disease or disorder involves psychotic, affective, vegatative or psychomotor symptoms of schizophrenia, or the extrapyramidal motor side-effects of other antipsychotic drugs.

18. The use of claim 14, wherein the disease or disorder is obesity, depression, schizophrenia, schizophreniform disorder, schizoaffective disorder, delusional disorder, a stress-related disease (e.g. general anxiety disorder), panic disorder, a phobia, obsessive-compulsive disorder, post-traumatic stress syndrome, immune system depression, a stress-induced problem with the urinary, gastrointestinal or cardiovascular system (e.g., stress incontinence), a neurodegenerative disorder, autism, chemotherapy-induced vomiting, hypertension, migraine headache, cluster headache, sexual dysfunction, addictive disorder, withdrawal syndrome, an adjustment disorder, an age-associated learning and mental disorder, anorexia nervosa, apathy, an attention-deficit disorder due to general medical conditions, attention-deficit hyperactivity disorder, behavioural disturbance (including agitation in conditions associated with diminished cognition, e.g., dementia, mental retardation or delirium), bipolar disorder, bulimia nervosa, chronic fatigue syndrome, conduct disorder, cyclothymic disorder, dysthymic disorder, fibromyalgia or other somatoform disorder, generalized anxiety disorder, an inhalation disorder, an intoxication disorder, movement disorder (e.g. Huntington's disease or tardive dyskinesia), oppositional defiant disorder, peripheral neuropathy, post-traumatic stress disorder, premenstrual dysphoric disorder, a psychotic disorder (brief and long duration disorders, psychotic disorder due to medical condition, psychotic disorder NOS), mood disorder (major depressive or bipolar disorder with psychotic features), seasonal affective disorder, a sleep disorder, a specific development disorder, agitation disorder, stress incontinence such as urge or mixed incontinence, selective serotonin reuptake inhibition (SSRI) "poop out" syndrome or Tourette's syndrome.

## Patentansprüche

1. Verbindung der Formel I 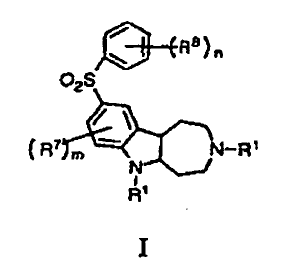 worin jeder Rest R¹ unabhängig voneinander
a) H,
b) C₁₋₄-Alkyl,
c) C₁₋₄-Alkyl, das mit einem Phenyl substituiert ist, wobei das Phenyl optional mit einem oder zwei Resten R² substituiert ist, oder
d) Phenyl, das optional mit einem oder zwei Resten R² substituiert ist, bedeutet;
R²
a) Halogen,
b) OR³,
c) CF₃,
d) C(=O)-NR⁴R⁵,
e) NH-SO₂-R⁶,
f) NR⁴R⁵,
g) NR⁴-C(=O)-R⁴,
h) SO₂-NR⁴R⁵,
i) CN oder
j) NO₂ bedeutet;
R³ H, C₁₋₄-Alkyl oder Phenyl bedeutet;
R⁴ und R⁵ unabhängig voneinander H, C₁₋₄-Alkyl bedeuten oder R⁴ und R⁵ zusammen mit dem gebundenen Stickstoffatom einen Ring bilden, der aus der Gruppe von 1-Pyrrolidinyl, 1-Piperazinyl und 1-Morpholinyl ausgewählt ist;
R⁶ H oder C₁₋₄-Alkyl bedeutet;
R⁷
a) H oder
b) Halogen,
c) OR³,
d) CF₃,
e) C(=O)-NR⁴R⁵,
f) NH-SO₂-R⁶,
g) NR⁴R⁵,
h) NR⁴-C(=O)-R⁴,
i) SO₂-NR⁴R⁵,
j) CN oder
k) NO₂ bedeutet;
R⁸
a) H,
b) F,
c) Cl,
d) C₁₋₄-Alkyl,
e) C₁₋₃-Alkoxy,
f) CF₃,
g) C₁₋₄-Alkyl, das mit einem Phenyl substituiert ist, wobei das Phenyl optional mit einem oder zwei Resten R² substituiert ist,
h) Phenyl, das optional mit einem oder zwei Resten R² substituiert ist,
i) OR³,
j) CO-NR⁴R⁵,
k) NR⁴R⁵,
l) NH-SO₂-R⁶ oder
m) NH-CO-R⁴ bedeutet;
Alkyl und Alkoxy bei jedem Vorkommen optional mit OH, Halogen oder NH₂ substituiert sind;
m 1 bis 2 ist; und n 1-3 ist;
oder ein pharmazeutisch akzeptables Salz derselben.

2. Verbindung nach Anspruch 1, wobei jeder Rest R¹ unabhängig voneinander H, Methyl oder Ethyl bedeutet.

3. Verbindung nach Anspruch 1, wobei R⁷ Wasserstoff ist.

4. Verbindung nach Anspruch 1, wobei R⁸ Wasserstoff ist.

5. Verbindung nach Anspruch 1, wobei R⁸ F, OCH₃ oder CF₃ ist.

6. Verbindung nach Anspruch 1, die
(a) (R,R)-6-Methyl-1,2,3,4,5,5a,6,10b-octahydroazepino[4,5-b]indol-9-yl-phenylsulfonhydrochlorid,
(b) (S,S)-6-Methyl-1,2,3,4,5,5a,6,10b-octahydroazepino[4,5-b]indol-9-yl-phenylsulfonhydrochlorid,
(c) (R,S)-6-Methyl-1,2,3,4,5,5a,6,10b-octahydroazepino[4,5-b]indol-9-yl-phenylsulfonhydrochlorid oder
(d) (S,R)-6-Methyl-1,2,3,4,5,5a,6,10b-octahydroazepino[4,5-b]indol-9-yl-phenylsulfonhydrochlorid ist.

7. Verbindung, die ausgewählt ist aus:
(5aS,10bS)-9-[(4-Fluorphenyl)sulfonyl]-6-methyl-1,2,3,4,5,5a,6,10b-octahydroazepino[4,5-b]indol,
(5aS,10bs)-9-[(3-Fluorphenyl)sulfonyl]-6-methyl-1,2,3,4,5,5a,6,10b-octahydroazepino[4,5-b]indol,
(5aS,10bS)-9-[(3,4-Difluorphenyl)sulfonyl]-6-methyl-1,2,3,4,5,5a,6,10b-octahydroazepino[4,5-b]indol,
(5aS,10bS)-9-[(3,5-Difluorphenyl)sulfonyl]-6-methyl-1,2,3,4,5,5a,6,10b-octahydroazepino[4,5-b]indol,
(5aS,10bS)-6-Methyl-9-[(4-methylphenyl)sulfonyl]-1,2,3,4,5,5a,6,10b-octahydroazepino[4,5-b]indol,
(5aS,10bS)-6-Methyl-9-[(3-methylphenyl)sulfonyl]-1,2,3,4,5,5a,6,10b-octahydroazepino[4,5-b]indol,
(5aS,10bS)-9-[(4-Methoxyphenyl)sulfonyl]-6-methyl-1,2,3,4,5,5a,6,10b-octahydroazepino[4,5-b]indol,
(5aS,10bS)-9-[(3-Methoxyphenyl)sulfonyl]-6-methyl-1,2,3,4,5,5a,6,10b-octahydroazepino[4,5-b]indol,
(5aS,10bS)-6-Methyl-9-[[(4-trifluormethyl)phenyl]sulfonyl]-1,2,3,4,5,5a,6,10b-octahydroazepino[4,5-b]indol,
(5aS,10bS)-3,6-Dimethyl-9-(phenylsulfonyl)-1,2,3,4,5,5a,6,10b-octahydroazepino[4,5-b]indol,
(5aS,10bS)-9-[(4-Fluorphenyl)sulfonyl]-3,6-dimethyl-1,2,3,4,5,5a,6,10b-octahydroazepino[4,5-b]indol,
(5aS,10bS)-9-[(3-Fluorphenyl)sulfonyl]-3,6-dimethyl-1,2,3,4,5,5a,6,10b-octahydroazepino[4,5-b]indol,
(5aS,10bS)-9-[(3,4-Difluorphenyl)sulfonyl]-3,6-dimethyl-1,2,3,4,5,5a,6,10b-octahydroazepino[4,5-b]indol,
(5aS,10bS)-9-[(3,5-Difluorphenyl)sulfonyl]-3,6-dimethyl-1,2,3,4,5,5a,6,10b-octahydroazepino[4,5-b]indol,
(5aS,10bS)-3,6-Dimethyl-9-[(4-methylphenyl)sulfonyl]-1,2,3,4,5,5a,6,10b-octahydroazepino[4,5-b]indol,
(5aS,10bS)-3,6-Dimethyl-9-[(3-methylphenyl)sulfonyl]-1,2,3,4,5,5a,6,10b-octahydroazepino[4,5-b]indol,
(5aS,10bS)-9-[(4-Methoxylphenyl)sulfonyl]-3,6-dimethyl-1,2,3,4,5,5a,6,10b-octahydroazepino[4,5-b]indol,
(5aS,10bS)-9-[(3-Methoxylphenyl)sulfonyl]-3,6-dimethyl-1,2,3,4,5,5a,6,10b-octahydroazepino[4,5-b]indol,
(5aS,10bS)-3,6-Dimethyl-9-[[(4-trifluormethyl)phenyl]sulfonyl]-1,2,3,4,5,5a,6,10b-octahydroazepino[4,5-b]indol,
(5aS,10bS)-3-Ethyl-6-methyl-9-(phenylsulfonyl)-1,2,3,4,5,5a,6,10b-octahydroazepino[4,5-b]indol,
(5aS,10bS)-3-Ethyl-9-[(4-fluorphenyl)sulfonyl]-6-methyl-1,2,3,4,5,5a,6,10b-octahydroazepino[4,5-b]indol,
(5aS,10bS)-3-Ethyl-9-[(3-fluorphenyl)sulfonyl]-6-methyl-1,2,3,4,5,5a,6,10b-octahydroazepino[4,5-b]indol,
(5aS,10bS)-3-Ethyl-9-[(3,4-difluorphenyl) sulfonyl] -6-methyl-1,2,3,4,5,5a,6,10b-octahydroazepino[4,5-b]indol,
(5aS,10bS)-3-Ethyl-9-[(3,5-difluorphenyl)sulfonyl]-6-methyl-1,2,3,4,5,5a,6,10b-octahydroazepino[4,5-b]indol,
(5aS,10bS)-3-Ethyl-6-methyl-9-[(4-methylphenyl)sulfonyl]-1,2,3,4,5,5a,6,10b-octahydroazepino[4,5-b]indol,
(5aS,10bS)-3-Ethyl-6-methyl-9-[(3-methylphenyl)sulfonyl]-1,2,3,4,5,5a,6,10b-octahydroazepino[4,5-b]indol,
(5aS,10bS)-3-Ethyl-9-[(4-methoxyphenyl)sulfonyl]-6-methyl-1,2,3,4,5,5a,6,10b-octahydroazepino[4,5-b]indol,
(5aS,10bS)-3-Ethyl-9-[(3-methoxyphenyl)sulfonyl]-6-methyl-1,2,3,4,5,5a,6,10b-octahydroazepino[4,5-b]indol,
(5aS,10bS)-3-Ethyl-6-methyl-9-[[(4-trifluormethyl)phenyl]sulfonyl]-1,2,3,4,5,5a,6,10b-octahydroazepino[4,5-b]indol,
(5aS,10bS)-1,6-Dimethyl-9-(phenylsulfonyl)-1,2,3,4,5,5a,6,10b-octahydroazepino[4,5-b]indol,
(5aS,10bS)-2,6-Dimethyl-9-(phenylsulfonyl)-1,2,3,4,5,5a,6,10b-octahydroazepino[4,5-b]indol,
(5aS,10bS)-4,6-Dimethyl-9-(phenylsulfonyl)-1,2,3,4,5,5a,6,10b-octahydroazepino[4,5-b]indol,
(5aS,10bS)-5,6-Dimethyl-9-(phenylsulfonyl)-1,2,3,4,5,5a,6,10b-octahydroazepino[4,5-b]indol,
(5aS,10bS)-9-[(4-Fluorphenyl)sulfonyl]-1,6-dimethyl-1,2,3,4,5,5a,6,10b-octahydroazepino[4,5-b]indol,
(5aS,10bS)-9-[(4-Fluorphenyl)sulfonyl]-2,6-dimethyl-1,2,3,4,5,5a,6,10b-octahydroazepino[4,5-b]indol,
(5aS,10bS)-9-[(4-Fluorphenyl)sulfonyl]-4,6-dimethyl-1,2,3,4,5,5a,6,10b-octahydroazepino[4,5-b]indol,
(5aS,10bS)-9-[(4-Fluorphenyl)sulfonyl]-5,6-dimethyl-1,2,3,4,5,5a,6,10b-octahydroazepino[4,5-b]indol,
(5aS,10bS)-9-[(3-Fluorphenyl)sulfonyl]-1,6-dimethyl-1,2,3,4,5,5a,6,10b-octahydroazepino[4,5-b]indol,
(5aS,10bS)-9-[(3-Fluorphenyl)sulfonyl]-2,6-dimethyl-1,2,3,4,5,5a,6,10b-octahydroazepino[4,5-b]indol,
(5aS,10bS)-9-[(3-Fluorphenyl)sulfonyl]-4,6-dimethyl-1,2,3,4,5,5a,6,10b-octahydroazepino[4,5-b]indol,
(5aS,10bS)-9-[(3-Fluorphenyl)sulfonyl]-5,6-dimethyl-1,2,3,4,5,5a,6,10b-octahydroazepino[4,5-b]indol,
(5aS,10bS)-1,5,6-Trimethyl-9-(phenylsulfonyl)-1,2,3,4,5,5a,6,10b-octahydroazepino[4,5-b]indol,
(5aS,10bS)-2,4,6-Trimethyl-9-(phenylsulfonyl)-1,2,3,4,5,5a,6,10b-octahydroazepino[4,5-b]indol,
(5aS,10bS)-9-[(4-Fluorphenyl)sulfonyl]-1,5,6-trimethyl-1,2,3,4,5,5a,6,10b-octahydroazepino[4,5-b]indol,
(5aS,10bS)-9-[(4-Fluorphenyl)sulfonyl]-2,4,6-trimethyl-1,2,3,4,5,5a,6,10b-octahydroazepino[4,5-b]indol,
(5aS,10bS)-9-[(3-Fluorphenyl)sulfonyl]-1,5,6-trimethyl-1,2,3,4,5,5a,6,10b-octahydroazepino[4,5-b]indol,
(5aS,10bS)-9-[(3-Fluorphenyl)sulfonyl]-2,4,6-trimethyl-1,2,3,4,5,5a,6,10b-octahydroazepino[4,5-b]indol,
(5aS,10bS)-6-Methyl-9-(phenylsulfonyl)-1,2,3,4,5,5a,6,10b-octahydroazepino[4,5-b]indol,
(5aS,10bS)-9-[(4-Fluorphenyl)sulfonyl]-1,2,3,4,5,5a,6,10b-octahydroazepino[4,5-b]indol,
(5aS,10bS)-9-[(3-Fluorphenyl)sulfonyl]-1,2,3,4,5,5a,6,10b-octahydroazepino[4,5-b]indol,
(5aS,10bS)-9-[(3,4-Difluorphenyl)sulfonyl]-1,2,3,4,5,5a,6,10b-octahydroazepino[4,5-b]indol,
(5aS,10bS)-9-[(3,5-Difluorphenyl)sulfonyl]-1,2,3,4,5,5a,6,10b-octahydroazepino[4,5-b]indol,
(5aS,10bS)-9-[(4-Methylphenyl)sulfonyl]-1,2,3,4,5,5a,6,10b-octahydroazepino[4,5-b]indol,
(5aS,10bS)-9-[(3-Methylphenyl)sulfonyl]-1,2,3,4,5,5a,6,10b-octahydroazepino[4,5-b]indol,
(5aS,10bS)-9-[(4-Methoxyphenyl)sulfonyl]-1,2,3,4,5,5a,6,10b-octahydroazepino[4,5-b]indol,
(5aS,10bS)-9-[(3-Methoxyphenyl)sulfonyl]-1,2,3,4,5,5a,6,10b-octahydroazepino[4,5-b]indol,
(5aS,10bS)-9-[[(4-Trifluormethyl)phenyl]sulfonyl]-1,2,3,4,5,5a,6,10b-octahydroazepino[4,5-b]indol,
(5aS,10bS)-3-Methyl-9-(phenylsulfonyl)-1,2,3,4,5,5a,6,10b-octahydroazepino[4,5-b]indol,
(5aS,10bS)-9-[(4-Fluorphenyl)sulfonyl]-3-methyl-1,2,3,4,5,5a,6,10b-octahydroazepino[4,5-b]indol,
(5aS,10bS)-9-[(3-Fluorphenyl)sulfonyl]-3-methyl-1,2,3,4,5,5a,6,10b-octahydroazepino[4,5-b]indol,
(5aS,10bS)-9-[(3,4-Difluorphenyl)sulfonyl]-3-methyl-1,2,3,4,5,5a,6,10b-octahydroazepino[4,5-b]indol,
(5aS,10bS)-9-[(3,5-Difluorphenyl)sulfonyl]-3-methyl-1,2,3,4,5,5a,6,10b-octahydroazepino[4,5-b]indol,
(5aS,10bS)-3-Methyl-9-[(4-methylphenyl)sulfonyl]-1,2,3,4,5,5a,6,10b-octahydroazepino[4,5-b]indol,
(5aS,10bS)-3-Methyl-9-[(3-methylphenyl)sulfonyl]-1,2,3,4,5,5a,6,10b-octahydroazepino[4,5-b]indol,
(5aS,10bS)-9-[(4-Methoxyphenyl)sulfonyl]-3-methyl-1,2,3,4,5,5a,6,10b-octahydroazepino[4,5-b]indol,
(5aS,10bS)-9-[(3-Methoxyphenyl)sulfonyl]-3-methyl-1,2,3,4,5,5a,6,10b-octahydroazepino[4,5-b]indol,
(5aS,10bS)-3-Methyl-9-[[(4-trifluormethyl)phenyl]sulfonyl]-1,2,3,4,5,5a,6,10b-octahydroazepino[4,5-b]indol,
(5aS,10bS)-3-Ethyl-9-(phenylsulfonyl)-1,2,3,4,5,5a,6,10b-octahydroazepino[4,5-b]indol,
(5aS,10bS)-3-Ethyl-9-[(4-fluorphenyl)sulfonyl]-1,2,3,4,5,5a,6,10b-octahydroazepino[4,5-b]indol,
(5aS,10bS)-3-Ethyl-9-[(3-fluorphenyl)sulfonyl]-1,2,3,4,5,5a,6,10b-octahydroazepino[4,5-b]indol,
(5aS,10bS)-3-Ethyl-9-[(3,4-difluorphenyl)sulfonyl]-1,2,3,4,5,5a,6,10b-octahydroazepino[4,5-b]indol,
(5aS,10bS)-3-Ethyl-9-[(3,5-difluorphenyl)sulfonyl]-1,2,3,4,5,5a,6,10b-octahydroazepino[4,5-b]indol,
(5aS,10bS)-3-Ethyl-9-[(4-methylphenyl)sulfonyl]-1,2,3,4,5,5a,6,10b-octahydroazepino[4,5-b]indol,
(5aS,10bS)-3-Ethyl-9-[(3-methylphenyl)sulfonyl]-1,2,3,4,5,5a,6,10b-octahydroazepino[4,5-b]indol,
(5aS,10bS)-3-Ethyl-9-[(4-methoxyphenyl)sulfonyl]-1,2,3,4,5,5a,6,10b-octahydroazepino[4,5-b]indol,
(5aS,10bS)-3-Ethyl-9-[(3-methoxyphenyl)sulfonyl]-1,2,3,4,5,5a,6,10b-octahydroazepino[4,5-b]indol,
(5aS,10bS-3-Ethyl-9-[[(4-trifluormethyl)phenyl]sulfonyl]-1,2,3,4,5,5a,6,10b-octahydroazepino[4,5-b]indol,
(5aS,10bS)-1-Methyl-9-(phenylsulfonyl)-1,2,3,4,5,5a,6,10b-octahydroazepino[4,5-b]indol,
(5aS,10bS)-2-Methyl-9-(phenylsulfonyl)-1,2,3,4,5,5a,6,10b-octahydroazepino[4,5-b]indol,
(5aS,10bS)-4-Methyl-9-(phenylsulfonyl)-1,2,3,4,5,5a,6,10b-octahydroazepino[4,5-b]indol,
(5aS,10bS)-5-Methyl-9-(phenylsulfonyl)-1,2,3,4,5,5a,6,10b-octahydroazepino[4,5-b]indol,
(5aS,10bS)-9-[(4-Fluorphenyl)sulfonyl]-1-methyl-1,2,3,4,5,5a,6,10b-octahydroazepino[4,5-b]indol,
(5aS,10bS)-9-[(4-Fluorphenyl)sulfonyl]-2-methyl-1,2,3,4,5,5a,6,10b-octahydroazepino[4,5-b]indol,
(5aS,10bS)-9-[(4-Fluorphenyl)sulfonyl]-4-methyl-1,2,3,4,5,5a,6,10b-octahydroazepino[4,5-b]indol,
(5aS,10bS)-9-[(4-Fluorphenyl)sulfonyl]-5-methyl-1,2,3,4,5,5a,6,10b-octahydroazepino[4,5-b]indol,
(5aS,10bS)-9-[(3-Fluorphenyl)sulfonyl]-1-methyl-1,2,3,4,5,5a,6,10b-octahydroazepino[4,5-b]indol,
(5aS,10bS)-9-[(3-Fluorphenyl)sulfonyl]-2-methyl-1,2,3,4,5,5a,6,10b-octahydroazepino[4,5-b]indol,
(5aS,10bS)-9-[(3-Fluorphenyl)sulfonyl]-4-methyl-1,2,3,4,5,5a,6,10b-octahydroazepino[4,5-b]indol,
(5aS,10bS)-9-[(3-Fluorphenyl)sulfonyl]-5-methyl-1,2,3,4,5,5a,6,10b-octahydroazepino[4,5-b]indol,
(5aS,10bS)-1,5-Dimethyl-9-(phenylsulfonyl)-1,2,3,4,5,5a,6,10b-octahydroazepino[4,5-b]indol,
(5aS,10bS)-2,4-Dimethyl-9-(phenylsulfonyl)-1,2,3,4,5,5a,6,10b-octahydroazepino[4,5-b]indol,
(5aS,10bS)-9-[(4-Fluorphenyl)sulfonyl]-1,5-dimethyl-1,2,3,4,5,5a,6,10b-octahydroazepino[4,5-b]indol,
(5aS,10bS)-9-[(4-Fluorphenyl)sulfonyl]-2,4-dimethyl-1,2,3,4,5,5a,6,10b-octahydroazepino[4,5-b]indol,
(5aS,10bS)-9-[(3-Fluorphenyl)sulfonyl]-1,5-dimethyl-1,2,3,4,5,5a,6,10b-octahydroazepino[4,5-b]indol,
(5aS,10bS)-9-[(3-Fluorphenyl)sulfonyl]-2,4-dimethyl-1,2,3,4,5,5a,6,10b-octahydroazepino[4,5-b]indol,
(5aR,10bS)-9-[(4-Fluorphenyl)sulfonyl]-6-methyl-1,2,3,4,5,5a,6,10b-octahydroazepino[4,5-b]indol,
(5aR,10bS)-9-[(3-Fluorphenyl)sulfonyl]-6-methyl-1,2,3,4,5,5a,6,10b-octahydroazepino[4,5-b]indol,
(5aR,10bS)-9-[(3,4-Difluorphenyl)sulfonyl]-6-methyl-1,2,3,4,5,5a,6,10b-octahydroazepino[4,5-b]indol,
(5aR,10bS)-9-[(3,5-Difluorphenyl)sulfonyl]-6-methyl-1,2,3,4,5,5a,6,10b-octahydroazepino[4,5-b]indol,
(5aR,10bS)-6-Methyl-9-[(4-methylphenyl)sulfonyl]-1,2,3,4,5,5a,6,10b-octahydroazepino[4,5-b]indol,
(5aR,10bS)-6-Methyl-9-[(3-methylphenyl)sulfonyl]-1,2,3,4,5,5a,6,10b-octahydroazepino[4,5-b]indol,
(5aR,10bS)-9-[(4-Methoxyphenyl)sulfonyl]-6-methyl-1,2,3,4,5,5a,6,10b-octahydroazepino[4,5-b]indol,
(5aR,10bS)-9-[(3-Methoxyphenyl)sulfonyl]-6-methyl-1,2,3,4,5,5a,6,10b-octahydroazepino[4,5-b]indol,
(5aR,10bS)-6-Methyl-9-[[(4-trifluormethyl)phenylsulfonyl]-1,2,3,4,5,5a,6,10b-octahydroazepino[4,5-b]indol,
(5aR,10bS)-3,6-Dimethyl-9-(phenylsulfonyl)-1,2,3,4,5,5a,6,10b-octahydroazepino[4,5-b]indol,
(5aR,10bS)-9-[(4-Fluorphenyl)sulfonyl]-3,6-dimethyl-1,2,3,4,5,5a,6,10b-octahydroazepino[4,5-b]indol,
(5aR,10bS)-9-[(3-Fluorphenyl)sulfonyl]-3,6-dimethyl-1,2,3,4,5,5a,6,10b-octahydroazepino[4,5-b]indol,
(5aR,10bS)-9-[(3,4-Difluorphenyl)sulfonyl]-3,6-dimethyl-1,2,3,4,5,5a,6,10b-octahydroazepino[4,5-b]indol,
(5aR,10bS)-9-[(3,5-Difluorphenyl)sulfonyl]-3,6-dimethyl-1,2,3,4,5,5a,6,10b-octahydroazepino[4,5-b]indol,
(5aR,10bS)-3,6-Dimethyl-9-[(4-methylphenyl)sulfonyl]-1,2,3,4,5,5a,6,10b-octahydroazepino[4,5-b]indol,
(5aR,10bS)-3,6-Dimethyl-9-[(3-methylphenyl)sulfonyl]-1,2,3,4,5,5a,6,10b-octahydroazepino[4,5-b]indol,
(5aR,10bS)-9-[(4-Methoxyphenyl)sulfonyl]-3,6-dimethyl-1,2,3,4,5,5a,6,10b-octahydroazepino[4,5-b]indol,
(5aR,10bS)-9-[(3-Methoxyphenyl)sulfonyl]-3,6-dimethyl-1,2,3,4,5,5a,6,10b-octahydroazepino[4,5-b]indol,
(5aR,10bS)-3,6-Dimethyl-9-[[(4-trifluormethyl)phenyl]sulfonyl]-1,2,3,4,5,5a,6,10b-octahydroazepino[4,5-b]indol,
(5aR,10bS)-3-Ethyl-6-methyl-9-(phenylsulfonyl)-1,2,3,4,5,5a,6,10b-octahydroazepino[4,5-b]indol,
(5aR,10bS)-3-Ethyl-9-[(4-fluorphenyl)sulfonyl]-6-methyl-1,2,3,4,5,5a,6,10b-octahydroazepino[4,5-b]indol,
(5aR,10bS)-3-Ethyl-9-[(3-fluorphenyl)sulfonyl]-6-methyl-1,2,3,4,5,5a,6,10b-octahydroazepino[4,5-b]indol,
(5aR,10bS)-3-Ethyl-9-[(3,4-difluorphenyl)sulfonyl]-6-methyl-1,2,3,4,5,5a,6,10b-octahydroazepino[4,5-b]indol,
(5aR,10bS)-3-Ethyl-9-[(3,5-difluorphenyl)sulfonyl]-6-methyl-1,2,3,4,5,5a,6,10b-octahydroazepino[4,5-b]indol,
(5aR,10bS)-3-Ethyl-6-methyl-9-[(4-methylphenyl)sulfonyl]-1,2,3,4,5,5a,6,10b-octahydroazepino[4,5-b]indol,
(5aR,10bS)-3-Ethyl-6-methyl-9-[(3-methylphenyl)sulfonyl]-1,2,3,4,5,5a,6,10b-octahydroazepino[4,5-b]indol,
(5aR,10bS)-3-Ethyl-9-[(4-methoxyphenyl)sulfonyl]-6-methyl-1,2,3,4,5,5a,6,10b-octahydroazepino[4,5-b]indol,
(5aR,10bS)-3-Ethyl-9-[(3-methoxyphenyl)sulfonyl]-6-methyl-1,2,3,4,5,5a,6,10b-octahydroazepino[4,5-b]indol,
(5aR,10bS)-3-Ethyl-6-methyl-9-[[(4-trifluormethyl)phenyl]sulfonyl]-1,2,3,4,5,5a,6,10b-octahydroazepino[4,5-b]indol,
(5aR,10bS)-1,6-Dimethyl-9-(phenylsulfonyl)-1,2,3,4,5,5a,6,10b-octahydroazepino[4,5-b]indol,
(5aR,10bS)-2,6-Dimethyl-9-(phenylsulfonyl)-1,2,3,4,5,5a,6,10b-octahydroazepino[4,5-b]indol,
(5aR,10bS)-4,6-Dimethyl-9-(phenylsulfonyl)-1,2,3,4,5,5a,6,10b-octahydroazepino[4,5-b]indol,
(5aR,10bS)-5,6-Dimethyl-9-(phenylsulfonyl)-1,2,3,4,5,5a,6,10b-octahydroazepino[4,5-b]indol,
(5aR,10bS)-9-[(4-Fluorphenyl)sulfonyl]-1,6-dimethyl-1,2,3,4,5,5a,6,10b-octahydroazepino[4,5-b]indol,
(5aR,10bS)-9-[(4-Fluorphenyl)sulfonyl]-2,6-dimethyl-1,2,3,4,5,5a,6,10b-octahydroazepino[4,5-b]indol,
(5aR,10bS)-9-[(4-Fluorphenyl)sulfonyl]-4,6-dimethyl-1,2,3,4,5,5a,6,10b-octahydroazepino[4,5-b]indol,
(5aR,10bS)-9-[(4-Fluorphenyl)sulfonyl]-5,6-dimethyl-1,2,3,4,5,5a,6,10b-octahydroazepino[4,5-b]indol,
(5aR,10bS)-9-[(3-Fluorphenyl)sulfonyl]-1,6-dimethyl-1,2,3,4,5,5a,6,10b-octahydroazepino[4,5-b]indol,
(5aR,10bS)-9-[(3-Fluorphenyl)sulfonyl]-2,6-dimethyl-1,2,3,4,5,5a,6,10b-octahydroazepino[4,5-b]indol,
(5aR,10bS)-9-[(3-Fluorphenyl)sulfonyl]-4,6-dimethyl-1,2,3,4,5,5a,6,10b-octahydroazepino[4,5-b]indol,
(5aR,10bS)-9-[(3-Fluorphenyl)sulfonyl]-5,6-dimethyl-1,2,3,4,5,5a,6,10b-octahydroazepino[4,5-b]indol,
(5aR,10bS)-1,5,6-Trimethyl-9-(phenylsulfonyl)-1,2,3,4,5,5a,6,10b-octahydroazepino[4,5-b]indol,
(5aR,10bS)-2,4,6-Trimethyl-9-(phenylsulfonyl)-1,2,3,4,5,5a,6,10b-octahydroazepino[4,5-b]indol,
(5aR,10bS)-9-[(4-Fluorphenyl)sulfonyl]-1,5,6-trimethyl-1,2,3,4,5,5a,6,10b-octahydroazepino[4,5-b]indol,
(5aR,10bS)-9-[(4-Fluorphenyl)sulfonyl]-2,4,6-trimethyl-1,2,3,4,5,5a,6,10b-octahydroazepino[4,5-b]indol,
(5aR,10bS)-9-[(3-Fluorphenyl)sulfonyl]-1,5,6-trimethyl-1,2,3,4,5,5a,6,10b-octahydroazepino[4,5-b]indol,
(5aR,10bS)-9-[(3-Fluorphenyl)sulfonyl]-2,4,6-trimethyl-1,2,3,4,5,5a,6,10b-octahydroazepino[4,5-b]indol,
(5aR,10bS)-6-Methyl-9-(phenylsulfonyl)-1,2,3,4,5,5a,6,10b-octahydroazepino[4,5-b]indol,
(5aR,10bS)-9-[(4-Fluorphenyl)sulfonyl]-1,2,3,4,5,5a,6,10b-octahydroazepino[4,5-b]indol,
(5aR,10bS)-9-[(3-Fluorphenyl)sulfonyl]-1,2,3,4,5,5a,6,10b-octahydroazepino[4,5-b]indol,
(5aR,10bs)-9-1(3,4-Difluorphenyl)sulfonyl]-1,2,3,4,5,5a,6,10b-octahydroazepino[4,5-b]indol,
(5aR,10bS)-9-[(3,5-Difluorphenyl)sulfonyl]-1,2,3,4,5,5a,6,10b-octahydroazepino[4,5-b]indol,
(5aR,10bS)-9-[(4-Methylphenyl)sulfonyl]-1,2,3,4,5,5a,6,10b-octahydroazepino[4,5-b]indol,
(5aR,10bS)-9-[(3-Methylphenyl)sulfonyl]-1,2,3,4,5,5a,6,10b-octahydroazepino[4,5-b]indol,
(5aR,10bS)-9-[(4-Methoxyphenyl)sulfonyl]-1,2,3,4,5,5a,6,10b-octahydroazepino[4,5-b]indol,
(5aR,10bS)-9-[(3-Methoxyphenyl)sulfonyl]-1,2,3,4,5,5a,6,10b-octahydroazepino[4,5-b]indol,
(5aR,10bS)-9-[[(4-Trifluormethyl)phenyl]sulfonyl]-1,2,3,4,5,5a,6,10b-octahydroazepino[4,5-b]indol,
(5aR,10bS)-3-Methyl-9-(phenylsulfonyl)-1,2,3,4,5,5a,6,10b-octahydroazepino[4,5-b]indol,
(5aR,10bS)-9-[(4-Fluorphenyl)sulfonyl]-3-methyl-1,2,3,4,5,5a,6,10b-octahydroazepino[4,5-b]indol,
(5aR,10bS)-9-[(3-Fluorphenyl)sulfonyl]-3-methyl-1,2,3,4,5,5a,6,10b-octahydroazepino[4,5-b]indol,
(5aR,10bS)-9-[(3,4-Difluorphenyl)sulfonyl]-3-methyl-1,2,3,4,5,5a,6,10b-octahydroazepino[4,5-b]indol,
(5aR,10bS)-9-[(3,5-Difluorphenyl)sulfonyl]-3-methyl-1,2,3,4,5,5a,6,10b-octahydroazepino[4,5-b]indol,
(5aR,10bS)-3-Methyl-9-[(4-methylphenyl)sulfonyl]-1,2,3,4,5,5a,6,10b-octahydroazepino[4,5-b]indol,
(5aR,10bS)-3-Methyl-9-[(3-methylphenyl)sulfonyl]-1,2,3,4,5,5a,6,10b-octahydroazepino[4,5-b]indol,
(5aR,10bS)-9-[(4-Methoxyphenyl)sulfonyl]-3-methyl-1,2,3,4,5,5a,6,10b-octahydroazepino[4,5-b]indol,
(5aR,10bS)-9-[(3-Methoxyphenyl)sulfonyl]-3-methyl-1,2,3,4,5,5a,6,10b-octahydroazepino[4,5-b]indol,
(5aR,10bS)-3-Methyl-9-[[(4-trifluormethyl)phenyl]sulfonyl]-1,2,3,4,5,5a,6,10b-octahydroazepino[4,5-b]indol,
(5aR,10bS)-3-Ethyl-9-(phenylsulfonyl)-1,2,3,4,5,5a,6,10b-octahydroazepino[4,5-b]indol,
(5aR,10bS)-3-Ethyl-9-[(4-fluorphenyl)sulfonyl]-1,2,3,4,5,5a,6,10b-octahydroazepino[4,5-b]indol,
(5aR,10bS)-3-Ethyl-9-[(3-fluorphenyl)sulfonyl]-1,2,3,4,5,5a,6,10b-octahydroazepino[4,5-b]indol,
(5aR,10bS)-3-Ethyl-9-[(3,4-difluorphenyl)sulfonyl]-1,2,3,4,5,5a,6,10b-octahydroazepino[4,5-b]indol,
(5aR,10bS)-3-Ethyl-9-[(3,5-difluorphenyl)sulfonyl]-1,2,3,4,5,5a,6,10b-octahydroazepino[4,5-b]indol,
(5aR,10bS)-3-Ethyl-9-[(4-methylphenyl)sulfonyl]-1,2,3,4,5,5a,6,10b-octahydroazepino[4,5-b]indol,
(5aR,10bS)-3-Ethyl-9-[(3-methylphenyl)sulfonyl]-1,2,3,4,5,5a,6,10b-octahydroazepino[4,5-b]indol,
(5aR,10bS)-3-Ethyl-9-[(4-methoxyphenyl)sulfonyl]-1,2,3,4,5,5a,6,10b-octahydroazepino[4,5-b]indol,
(5aR,10bS)-3-Ethyl-9-[(3-methoxyphenyl)sulfonyl]-1,2,3,4,5,5a,6,10b-octahydroazepino[4,5-b]indol,
(5aR,10bS-3-Ethyl-9-[[(4-trifluormethyl)phenyl]sulfonyl]-1,2,3,4,5,5a,6,10b-octahydroazepino[4,5-b]indol,
(5aR,10bS)-1-Methyl-9-(phenylsulfonyl)-1,2,3,4,5,5a,6,10b-octahydroazepino[4,5-b]indol,
(5aR,10bS)-2-Methyl-9-(phenylsulfonyl)-1,2,3,4,5,5a,6,10b-octahydroazepino[4,5-b]indol,
(5aR,10bS)-4-Methyl-9-(phenylsulfonyl)-1,2,3,4,5,5a,6,10b-octahydroazepino[4,5-b]indol,
(5aR,10bS)-5-Methyl-9-(phenylsulfonyl)-1,2,3,4,5,5a,6,10b-octahydroazepino[4,5-b]indol,
(5aR,10bS)-9-[(4-Fluorphenyl)sulfonyl]-1-methyl-1,2,3,4,5,5a,6,10b-octahydroazepino[4,5-b]indol,
(5aR,10bS)-9-[(4-Fluorphenyl)sulfonyl]-2-methyl-1,2,3,4,5,5a,6,10b-octahydroazepino[4,5-b]indol,
(5aR,10bS)-9-[(4-Fluorphenyl)sulfonyl]-4-methyl-1,2,3,4,5,5a,6,10b-octahydroazepino[4,5-b]indol,
(5aR,10bS)-9-[(4-Fluorphenyl)sulfonyl]-5-methyl-1,2,3,4,5,5a,6,10b-octahydroazepino[4,5-b]indol,
(5aR,10bS)-9-[(3-Fluorphenyl)sulfonyl]-1-methyl-1,2,3,4,5,5a,6,10b-octahydroazepino[4,5-b]indol,
(5aR,10bS)-9-[(3-Fluorphenyl)sulfonyl]-2-methyl-1,2,3,4,5,5a,6,10b-octahydroazepino[4,5-b]indol,
(5aR,10bS)-9-[(3-Fluorphenyl)sulfonyl]-4-methyl-1,2,3,4,5,5a,6,10b-octahydroazepino[4,5-b]indol,
(5aR,10bS)-9-[(3-Fluorphenyl)sulfonyl]-5-methyl-1,2,3,4,5,5a,6,10b-octahydroazepino[4,5-b]indol,
(5aR,10bS)-1,5-Dimethyl-9-(phenylsulfonyl)-1,2,3,4,5,5a,6,10b-octahydroazepino[4,5-b]indol,
(5aR,10bS)-2,4-Dimethyl-9-(phenylsulfonyl)-1,2,3,4,5,5a,6,10b-octahydroazepino[4,5-b]indol,
(5aR,10bS)-9-[(4-Fluorphenyl)sulfonyl]-1,5-dimethyl-1,2,3,4,5,5a,6,10b-octahydroazepino[4,5-b]indol,
(5aR,10bS)-9-[(4-Fluorphenyl)sulfonyl]-2,4-dimethyl-1,2,3,4,5,5a,6,10b-octahydroazepino[4,5-b]indol,
(5aR,10bS)-9-[(3-Fluorphenyl)sulfonyl]-1,5-dimethyl-1,2,3,4,5,5a,6,10b-octahydroazepino[4,5-b]indol,
(5aR,10bS)-9-[(3-Fluorphenyl)sulfonyl]-2,4-dimethyl-1,2,3,4,5,5a,6,10b-octahydroazepino[4,5-b]indol,
(5aS,10bR)-9-[(4-Fluorphenyl)sulfonyl]-6-methyl-1,2,3,4,5,5a,6,10b-octahydroazepino[4,5-b]indol,
(5aS,10bR)-9-[(3-Fluorphenyl)sulfonyl]-6-methyl-1,2,3,4,5,5a,6,10b-octahydroazepino[4,5-b]indol,
(5aS,10bR)-9-[(3,4-Difluorphenyl)sulfonyl]-6-methyl-1,2,3,4,5,5a,6,10b-octahydroazepino[4,5-b]indol,
(5aS,10bR)-9-[(3,5-Difluorphenyl)sulfonyl]-6-methyl-1,2,3,4,5,5a,6,10b-octahydroazepino[4,5-b]indol,
(5aS,10bR)-6-Methyl-9-[(4-methylphenyl)sulfonyl]-1,2,3,4,5,5a,6,10b-octahydroazepino[4,5-b]indol,
(5aS,10bR)-6-Methyl-9-[(3-methylphenyl)sulfonyl]-1,2,3,4,5,5a,6,10b-octahydroazepino[4,5-b]indol,
(5aS,10bR)-9-[(4-Methoxyphenyl)sulfonyl]-6-methyl-1,2,3,4,5,5a,6,10b-octahydroazepino[4,5-b]indol,
(5aS,10bR)-9-[(3-Methoxyphenyl)sulfonyl]-6-methyl-1,2,3,4,5,5a,6,10b-octahydroazepino[4,5-b]indol,
(5aS,10bR)-6-Methyl-9-[[(4-trifluormethyl)phenyl]sulfonyl]-1,2,3,4,5,5a,6,10b-octahydroazepino[4,5-b]indol,
(5aS,10bR)-3,6-Dimethyl-9-(phenylsulfonyl)-1,2,3,4,5,5a,6,10b-octahydroazepino[4,5-b]indol,
(5aS,10bR)-9-[(4-Fluorphenyl)sulfonyl]-3,6-dimethyl-1,2,3,4,5,5a,6,10b-octahydroazepino[4,5-b]indol,
(5aS,10bR)-9-[(3-Fluorphenyl)sulfonyl]-3,6-dimethyl-1,2,3,4,5,5a,6,10b-octahydroazepino[4,5-b]indol,
(5aS,10bR)-9-[(3,4-Difluorphenyl)sulfonyl]-3,6-dimethyl-1,2,3,4,5,5a,6,10b-octahydroazepino[4,5-b]indol,
(5aS,10bR)-9-[(3,5-Difluorphenyl)sulfonyl]-3,6-dimethyl-1,2,3,4,5,5a,6,10b-octahydroazepino[4,5-b]indol,
(5aS,10bR)-3,6-Dimethyl-9-[(4-methylphenyl)sulfonyl]-1,2,3,4,5,5a,6,10b-octahydroazepino[4,5-b]indol,
(5aS,10bR)-3,6-Dimethyl-9-[(3-methylphenyl)sulfonyl]-1,2,3,4,5,5a,6,10b-octahydroazepino[4,5-b]indol,
(5aS,10bR)-9-[(4-Methoxyphenyl)sulfonyl]-3,6-dimethyl-1,2,3,4,5,5a,6,10b-octahydroazepino[4,5-b]indol,
(5aS,10bR)-9-[(3-Methoxyphenyl)sulfonyl]-3,6-dimethyl-1,2,3,4,5,5a,6,10b-octahydroazepino[4,5-b]indol,
(5aS,10bR)-3,6-Dimethyl-9-[[(4-trifluormethyl)phenyl]sulfonyl]-1,2,3,4,5,5a,6,10b-octahydroazepino[4,5-b]indol,
(5aS,10bR)-3-Ethyl-6-methyl-9-(phenylsulfonyl)-1,2,3,4,5,5a,6,10b-octahydroazepino[4,5-b]indol,
(5aS,10bR)-3-Ethyl-9-[(4-fluorphenyl)sulfonyl]-6-methyl-1,2,3,4,5,5a,6,10b-octahydroazepino[4,5-b]indol,
(5aS,10bR)-3-Ethyl-9-[(3-fluorphenyl)sulfonyl]-6-methyl-1,2,3,4,5,5a,6,10b-octahydroazepino[4,5-b]indol,
(5aS,10bR)-3-Ethyl-9-[(3,4-difluorphenyl)sulfonyl]-6-methyl-1,2,3,4,5,5a,6,10b-octahydroazepino[4,5-b]indol,
(5aS,10bR)-3-Ethyl-9-[(3,5-difluorphenyl)sulfonyl]-6-methyl-1,2,3,4,5,5a,6,10b-octahydroazepino[4,5-b]indol,
(5aS,10bR)-3-Ethyl-6-methyl-9-[(4-methylphenyl)sulfonyl]-1,2,3,4,5,5a,6,10b-octahydroazepino[4,5-b]indol,
(5aS,10bR)-3-Ethyl-6-methyl-9-[(3-methylphenyl)sulfonyl]-1,2,3,4,5,5a,6,10b-octahydroazepino[4,5-b]indol,
(5aS,10bR)-3-Ethyl-9-[(4-methoxyphenyl)sulfonyl]-6-methyl-1,2,3,4,5,5a,6,10b-octahydroazepino[4,5-b]indol,
(5aS,10bR)-3-Ethyl-9-[(3-methoxyphenyl)sulfonyl]-6-methyl-1,2,3,4,5,5a,6,10b-octahydroazepino[4,5-b]indol,
(5aS,10bR)-3-Ethyl-6-methyl-9-[[(4-trifluormethyl)phenyl]sulfonyl]-1,2,3,4,5,5a,6,10b-octahydroazepino[4,5-b]indol,
(5aS,10bR)-1,6-Dimethyl-9-(phenylsulfonyl)-1,2,3,4,5,5a,6,10b-octahydroazepino[4,5-b]indol,
(5aS,10bR)-2,6-Dimethyl-9-(phenylsulfonyl)-1,2,3,4,5,5a,6,10b-octahydroazepino[4,5-b]indol,
(5aS,10bR)-4,6-Dimethyl-9-(phenylsulfonyl)-1,2,3,4,5,5a,6,10b-octahydroazepino[4,5-b]indol,
(5aS,10bR)-5,6-Dimethyl-9-(phenylsulfonyl)-1,2,3,4,5,5a,6,10b-octahydroazepino[4,5-b]indol,
(5aS,10bR)-9-[(4-Fluorphenyl)sulfonyl]-1,6-dimethyl-1,2,3,4,5,5a,6,10b-octahydroazepino[4,5-b]indol,
(5aS,10bR)-9-[(4-Fluorphenyl)sulfonyl]-2,6-dimethyl-1,2,3,4,5,5a,6,10b-octahydroazepino[4,5-b]indol,
(5aS,10bR)-9-[(4-Fluorphenyl)sulfonyl]-4,6-dimethyl-1,2,3,4,5,5a,6,10b-octahydroazepino[4,5-b]indol,
(5aS,10bR)-9-[(4-Fluorphenyl)sulfonyl]-5,6-dimethyl-1,2,3,4,5,5a,6,10b-octahydroazepino[4,5-b]indol,
(5aS,10bR)-9-[(3-Fluorphenyl)sulfonyl]-1,6-dimethyl-1,2,3,4,5,5a,6,10b-octahydroazepino[4,5-b]indol,
(5aS,10bR)-9-[(3-Fluorphenyl)sulfonyl]-2,6-dimethyl-1,2,3,4,5,5a,6,10b-octahydroazepino[4,5-b]indol,
(5aS,10bR)-9-[(3-Fluorphenyl)sulfonyl]-4,6-dimethyl-1,2,3,4,5,5a,6,10b-octahydroazepino[4,5-b]indol,
(5aS,10bR)-9-[(3-Fluorphenyl)sulfonyl]-5,6-dimethyl-1,2,3,4,5,5a,6,10b-octahydroazepino[4,5-b]indol,
(5aS,10bR)-1,5,6-Trimethyl-9-(phenylsulfonyl)-1,2,3,4,5,5a,6,10b-octahydroazepino[4,5-b]indol,
(5aS,10bR)-2,4,6-Trimethyl-9-(phenylsulfonyl)-1,2,3,4,5,5a,6,10b-octahydroazepino[4,5-b]indol,
(5aS,10bR)-9-[(4-Fluorphenyl)sulfonyl]-1,5,6-trimethyl-1,2,3,4,5,5a,6,10b-octahydroazepino[4,5-b]indol,
(5aS,10bR)-9-[(4-Fluorphenyl)sulfonyl]-2,4,6-trimethyl-1,2,3,4,5,5a,6,10b-octahydroazepino[4,5-b]indol,
(5aS,10bR)-9-[(3-Fluorphenyl)sulfonyl]-1,5,6-trimethyl-1,2,3,4,5,5a,6,10b-octahydroazepino[4,5-b]indol,
(5aS,10bR)-9-[(3-Fluorphenyl)sulfonyl]-2,4,6-trimethyl-1,2,3,4,5,5a,6,10b-octahydroazepino[4,5-b]indol,
(5aS,10bR)-6-Methyl-9-(phenylsulfonyl)-1,2,3,4,5,5a,6,10b-octahydroazepino[4,5-b]indol,
(5aS,10bR)-9-[(4-Fluorphenyl)sulfonyl]-1,2,3,4,5,5a,6,10b-octahydroazepino[4,5-b]indol,
(5aS,10bR)-9-[(3-Fluorphenyl)sulfonyl]-1,2,3,4,5,5a,6,10b-octahydroazepino[4,5-b]indol,
(5aS,10bR)-9-[(3,4-Difluorphenyl)sulfonyl]-1,2,3,4,5,5a,6,10b-octahydroazepino[4,5-b]indol,
(5aS,10bR)-9-[(3,5-Difluorphenyl)sulfonyl]-1,2,3,4,5,5a,6,10b-octahydroazepino[4,5-b]indol,
(5aS,10bR)-9-[(4-Methylphenyl)sulfonyl]-1,2,3,4,5,5a,6,10b-octahydroazepino[4,5-b]indol,
(5aS,10bR)-9-[(3-Methylphenyl)sulfonyl]-1,2,3,4,5,5a,6,10b-octahydroazepino[4,5-b]indol,
(5aS,10bR)-9-[(4-Methoxyphenyl)sulfonyl]-1,2,3,4,5,5a,6,10b-octahydroazepino[4,5-b]indol,
(5aS,10bR)-9-[(3-Methoxyphenyl)sulfonyl]-1,2,3,4,5,5a,6,10b-octahydroazepino[4,5-b]indol,
(5aS,10bR)-9-[[(4-Trifluormethyl)phenyl]sulfonyl]-1,2,3,4,5,5a,6,10b-octahydroazepino[4,5-b]indol,
(5aS,10bR)-3-Methyl-9-(phenylsulfonyl)-1,2,3,4,5,5a,6,10b-octahydroazepino[4,5-b]indol,
(5aS,10bR)-9-[(4-Fluorphenyl)sulfonyl]-3-methyl-1,2,3,4,5,5a,6,10b-octahydroazepino[4,5-b]indol,
(5aS,10bR)-9-[(3-Fluorphenyl)sulfonyl]-3-methyl-1,2,3,4,5,5a,6,10b-octahydroazepino[4,5-b]indol,
(5aS,10bR)-9-[(3,4-Difluorphenyl)sulfonyl]-3-methyl-1,2,3,4,5,5a,6,10b-octahydroazepino[4,5-b]indol,
(5aS,10bR)-9-[(3,5-Difluorphenyl)sulfonyl]-3-methyl-1,2,3,4,5,5a,6,10b-octahydroazepino[4,5-b]indol,
(5aS,10bR)-3-Methyl-9-[(4-methylphenyl)sulfonyl]-1,2,3,4,5,5a,6,10b-octahydroazepino[4,5-b]indol,
(5aS,10bR)-3-Methyl-9-[(3-methylphenyl)sulfonyl]-1,2,3,4,5,5a,6,10b-octahydroazepino[4,5-b]indol,
(5aS,10bR)-9-[(4-Methoxyphenyl)sulfonyl]-3-methyl-1,2,3,4,5,5a,6,10b-octahydroazepino[4,5-b]indol,
(5aS,10bR)-9-[(3-Methoxyphenyl)sulfonyl]-3-methyl-1,2,3,4,5,5a,6,10b-octahydroazepino[4,5-b]indol,
(5aS,10bR)-3-Methyl-9-[[(4-trifluormethyl)phenyl]sulfonyl]-1,2,3,4,5,5a,6,10b-octahydroazepino[4,5-b]indol,
(5aS,10bR)-3-Ethyl-9-(phenylsulfonyl)-1,2,3,4,5,5a,6,10b-octahydroazepino[4,5-b]indol,
(5aS,10bR)-3-Ethyl-9-[(4-fluorphenyl)sulfonyl]-1,2,3,4,5,5a,6,10b-octahydroazepino[4,5-b]indol,
(5aS,10bR)-3-Ethyl-9-[(3-fluorphenyl)sulfonyl]-1,2,3,4,5,5a,6,10b-octahydroazepino[4,5-b]indol,
(5aS,10bR)-3-Ethyl-9-[(3,4-difluorphenyl)sulfonyl]-1,2,3,4,5,5a,6,10b-octahydroazepino[4,5-b]indol,
(5aS,10bR)-3-Ethyl-9-[(3,5-difluorphenyl)sulfonyl]-1,2,3,4,5,5a,6,10b-octahydroazepino[4,5-b]indol,
(5aS,10bR)-3-Ethyl-9-[(4-methylphenyl)sulfonyl]-1,2,3,4,5,5a,6,10b-octahydroazepino[4,5-b]indol,
(5aS,10bR)-3-Ethyl-9-[(3-methylphenyl)sulfonyl]-1,2,3,4,5,5a,6,10b-octahydroazepino[4,5-b]indol,
(5aS,10bR)-3-Ethyl-9-[(4-methoxyphenyl)sulfonyl]-1,2,3,4,5,5a,6,10b-octahydroazepino[4,5-b]indol,
(5aS,10bR)-3-Ethyl-9-[(3-methoxyphenyl)sulfonyl]-1,2,3,4,5,5a,6,10b-octahydroazepino[4,5-b]indol,
(5aS,10bR-3-Ethyl-9-[[(4-trifluormethyl)phenyl]sulfonyl]-1,2,3,4,5,5a,6,10b-octahydroazepino[4,5-b]indol,
(5aS,10bR)-1-Methyl-9-(phenylsulfonyl)-1,2,3,4,5,5a,6,10b-octahydroazepino[4,5-b]indol,
(5aS,10bR)-2-Methyl-9-(phenylsulfonyl)-1,2,3,4,5,5a,6,10b-octahydroazepino[4,5-b]indol,
(5aS,10bR)-4-Methyl-9-(phenylsulfonyl)-1,2,3,4,5,5a,6,10b-octahydroazepino[4,5-b]indol,
(5aS,10bR)-5-Methyl-9-(phenylsulfonyl)-1,2,3,4,5,5a,6,10b-octahydroazepino[4,5-b]indol,
(5aS,10bR)-9-[(4-Fluorphenyl)sulfonyl]-1-methyl-1,2,3,4,5,5a,6,10b-octahydroazepino[4,5-b]indol,
(5aS,10bR)-9-[(4-Fluorphenyl)sulfonyl]-2-methyl-1,2,3,4,5,5a,6,10b-octahydroazepino[4,5-b]indol,
(5aS,10bR)-9-[(4-Fluorphenyl)sulfonyl]-4-methyl-1,2,3,4,5,5a,6,10b-octahydroazepino[4,5-b]indol,
(5aS,10bR)-9-[(4-Fluorphenyl)sulfonyl]-5-methyl-1,2,3,4,5,5a,6,10b-octahydroazepino[4,5-b]indol,
(5aS,10bR)-9-[(3-Fluorphenyl)sulfonyl]-1-methyl-1,2,3,4,5,5a,6,10b-octahydroazepino[4,5-b]indol,
(5aS,10bR)-9-[(3-Fluorphenyl)sulfonyl]-2-methyl-1,2,3,4,5,5a,6,10b-octahydroazepino[4,5-b]indol,
(5aS,10bR)-9-[(3-Fluorphenyl)sulfonyl]-4-methyl-1,2,3,4,5,5a,6,10b-octahydroazepino[4,5-b]indol,
(5aS,10bR)-9-[(3-Fluorphenyl)sulfonyl]-5-methyl-1,2,3,4,5,5a,6,10b-octahydroazepino[4,5-b]indol,
(5aS,10bR)-1,5-Dimethyl-9-(phenylsulfonyl)-1,2,3,4,5,5a,6,10b-octahydroazepino[4,5-b]indol,
(5aS,10bR)-2,4-Dimethyl-9-(phenylsulfonyl)-1,2,3,4,5,5a,6,10b-octahydroazepino[4,5-b]indol,
(5aS,10bR)-9-[(4-Fluorphenyl)sulfonyl]-1,5-dimethyl-1,2,3,4,5,5a,6,10b-octahydroazepino[4,5-b]indol,
(5aS,10bR)-9-[(4-Fluorphenyl)sulfonyl]-2,4-dimethyl-1,2,3,4,5,5a,6,10b-octahydroazepino[4,5-b]indol,
(5aS,10bR)-9-[(3-Fluorphenyl)sulfonyl]-1,5-dimethyl-1,2,3,4,5,5a,6,10b-octahydroazepino[4,5-b]indol,
(5aS,10bR)-9-[(3-Fluorphenyl)sulfonyl]-2,4-dimethyl-1,2,3,4,5,5a,6,10b-octahydroazepino[4,5-b]indol,
(5aR,10bR)-9-[(4-Fluorphenyl)sulfonyl]-6-methyl-1,2,3,4,5,5a,6,10b-octahydroazepino[4,5-b]indol,
(5aR,10bR)-9-[(3-Fluorphenyl)sulfonyl]-6-methyl-1,2,3,4,5,5a,6,10b-octahydroazepino[4,5-b]indol,
(5aR,10bR)-9-[(3,4-Difluorphenyl)sulfonyl]-6-methyl-1,2,3,4,5,5a,6,10b-octahydroazepino[4,5-b]indol,
(5aR,10bR)-9-[(3,5-Difluorphenyl)sulfonyl]-6-methyl-1,2,3,4,5,5a,6,10b-octahydroazepino[4,5-b]indol,
(5aR,10bR)-6-Methyl-9-[(4-methylphenyl)sulfonyl]-1,2,3,4,5,5a,6,10b-octahydroazepino[4,5-b]indol,
(5aR,10bR)-6-Methyl-9-[(3-methylphenyl)sulfonyl]-1,2,3,4,5,5a,6,10b-octahydroazepino[4,5-b]indol,
(5aR,10bR)-9-[(4-Methoxyphenyl)sulfonyl]-6-methyl-1,2,3,4,5,5a,6,10b-octahydroazepino[4,5-b]indol,
(5aR,10bR)-9-[(3-Methoxyphenyl)sulfonyl]-6-methyl-1,2,3,4,5,5a,6,10b-octahydroazepino[4,5-b]indol,
(5aR,10bR)-6-Methyl-9-[[(4-trifluormethyl)phenyl)sulfonyl]-1,2,3,4,5,5a,6,10b-octahydroazepino[4,5-b]indol,
(5aR,10bR)-3,6-Dimethyl-9-(phenylsulfonyl)-1,2,3,4,5,5a,6,10b-octahydroazepino[4,5-b]indol,
(5aR,10bR)-9-[(4-Fluorphenyl)sulfonyl]-3,6-dimethyl-1,2,3,4,5,5a,6,10b-octahydroazepino[4,5-b]indol,
(5aR,10bR)-9-[(3-Fluorphenyl)sulfonyl]-3,6-dimethyl-1,2,3,4,5,5a,6,10b-octahydroazepino[4,5-b]indol,
(5aR,10bR)-9-[(3,4-Difluorphenyl)sulfonyl]-3,6-dimethyl-1,2,3,4,5,5a,6,10b-octahydroazepino[4,5-b]indol,
(5aR,10bR)-9-[(3,5-Difluorphenyl)sulfonyl]-3,6-dimethyl-1,2,3,4,5,5a,6,10b-octahydroazepino[4,5-b]indol,
(5aR,10bR)-3,6-Dimethyl-9-[(4-methylphenyl)sulfonyl]-1,2,3,4,5,5a,6,10b-octahydroazepino[4,5-b]indol,
(5aR,10bR)-3,6-Dimethyl-9-[(3-methylphenyl)sulfonyl]-1,2,3,4,5,5a,6,10b-octahydroazepino[4,5-b]indol,
(5aR,10bR)-9-[(4-Methoxyphenyl)sulfonyl]-3,6-dimethyl-1,2,3,4,5,5a,6,10b-octahydroazepino[4,5-b]indol,
(5aR,10bR)-9-[(3-Methoxyphenyl)sulfonyl]-3,6-dimethyl-1,2,3,4,5,5a,6,10b-octahydroazepino[4,5-b]indol,
(5aR,10bR)-3,6-Dimethyl-9-[[(4-trifluormethyl)phenyl]sulfonyl]-1,2,3,4,5,5a,6,10b-octahydroazepino[4,5-b]indol,
(5aR,10bR)-3-Ethyl-6-methyl-9-(phenylsulfonyl)-1,2,3,4,5,5a,6,10b-octahydroazepino[4,5-b]indol,
(5aR,10bR)-3-Ethyl-9-[(4-fluorphenyl)sulfonyl]-6-methyl-1,2,3,4,5,5a,6,10b-octahydroazepino[4,5-b]indol,
(5aR,10bR)-3-Ethyl-9-[(3-fluorphenyl)sulfonyl]-6-methyl-1,2,3,4,5,5a,6,10b-octahydroazepino[4,5-b]indol,
(5aR,10bR)-3-Ethyl-9-[(3,4-difluorphenyl)sulfonyl]-6-methyl-1,2,3,4,5,5a,6,10b-octahydroazepino[4,5-b]indol,
(5aR,10bR)-3-Ethyl-9-[(3,5-difluorphenyl)sulfonyl]-6-methyl-1,2,3,4,5,5a,6,10b-octahydroazepino[4,5-b]indol,
(5aR,10bR)-3-Ethyl-6-methyl-9-[(4-methylphenyl)sulfonyl]-1,2,3,4,5,5a,6,10b-octahydroazepino[4,5-b]indol,
(5aR,10bR)-3-Ethyl-6-methyl-9-[(3-methylphenyl)sulfonyl]-1,2,3,4,5,5a,6,10b-octahydroazepino[4,5-b]indol,
(5aR,10bR)-3-Ethyl-9-[(4-methoxyphenyl)sulfonyl]-6-methyl-1,2,3,4,5,5a,6,10b-octahydroazepino[4,5-b]indol,
(5aR,10bR)-3-Ethyl-9-[(3-methoxyphenyl)sulfonyl]-6-methyl-1,2,3,4,5,5a,6,10b-octahydroazepino[4,5-b]indol,
(5aR,10bR)-3-Ethyl-6-methyl-9-[[(4-trifluormethyl)phenyl]sulfonyl]-1,2,3,4,5,5a,6,10b-octahydroazepino[4,5-b]indol,
(5aR,10bR)-1,6-Dimethyl-9-(phenylsulfonyl)-1,2,3,4,5,5a,6,10b-octahydroazepino[4,5-b]indol,
(5aR,10bR)-2,6-Dimethyl-9-(phenylsulfonyl)-1,2,3,4,5,5a,6,10b-octahydroazepino[4,5-b]indol,
(5aR,10bR)-4,6-Dimethyl-9-(phenylsulfonyl)-1,2,3,4,5,5a,6,10b-octahydroazepino[4,5-b]indol,
(5aR,10bR)-5,6-Dimethyl-9-(phenylsulfonyl)-1,2,3,4,5,5a,6,10b-octahydroazepino[4,5-b]indol,
(5aR,10bR)-9-[(4-Fluorphenyl)sulfonyl]-1,6-dimethyl-1,2,3,4,5,5a,6,10b-octahydroazepino[4,5-b]indol,
(5aR,10bR)-9-[(4-Fluorphenyl)sulfonyl]-2,6-dimethyl-1,2,3,4,5,5a,6,10b-octahydroazepino[4,5-b]indol,
(5aR,10bR)-9-[(4-Fluorphenyl)sulfonyl]-4,6-dimethyl-1,2,3,4,5,5a,6,10b-octahydroazepino[4,5-b]indol,
(5aR,10bR)-9-[(4-Fluorphenyl)sulfonyl]-5,6-dimethyl-1,2,3,4,5,5a,6,10b-octahydroazepino[4,5-b]indol,
(5aR,10bR)-9-[(3-Fluorphenyl)sulfonyl]-1,6-dimethyl-1,2,3,4,5,5a,6,10b-octahydroazepino[4,5-b]indol,
(5aR,10bR)-9-[(3-Fluorphenyl)sulfonyl]-2,6-dimethyl-1,2,3,4,5,5a,6,10b-octahydroazepino[4,5-b]indol,
(5aR,10bR)-9-[(3-Fluorphenyl)sulfonyl]-4,6-dimethyl-1,2,3,4,5,5a,6,10b-octahydroazepino[4,5-b]indol,
(5aR,10bR)-9-[(3-Fluorphenyl)sulfonyl]-5,6-dimethyl-1,2,3,4,5,5a,6,10b-octahydroazepino[4,5-b]indol,
(5aR,10bR)-1,5,6-Trimethyl-9-(phenylsulfonyl)-1,2,3,4,5,5a,6,10b-octahydroazepino[4,5-b]indol,
(5aR,10bR)-2,4,6-Trimethyl-9-(phenylsulfonyl)-1,2,3,4,5,5a,6,10b-octahydroazepino[4,5-b]indol,
(5aR,10bR)-9-[(4-Fluorphenyl)sulfonyl]-1,5,6-trimethyl-1,2,3,4,5,5a,6,10b-octahydroazepino[4,5-b]indol,
(5aR,10bR)-9-[(4-Fluorphenyl)sulfonyl]-2,4,6-trimethyl-1,2,3,4,5,5a,6,10b-octahydroazepino[4,5-b]indol,
(5aR,10bR)-9-[(3-Fluorphenyl)sulfonyl]-1,5,6-trimethyl-1,2,3,4,5,5a,6,10b-octahydroazepino[4,5-b]indol,
(5aR,10bR)-9-[(3-Fluorphenyl)sulfonyl]-2,4,6-trimethyl-1,2,3,4,5,5a,6,10b-octahydroazepino[4,5-b]indol,
(5aR,10bR)-6-Methyl-9-(phenylsulfonyl)-1,2,3,4,5,5a,6,10b-octahydroazepino[4,5-b]indol,
(5aR,10bR)-9-[(4-Fluorphenyl)sulfonyl]-1,2,3,4,5,5a,6,10b-octahydroazepino[4,5-b]indol,
(5aR,10bR)-9-[(3-Fluorphenyl)sulfonyl]-1,2,3,4,5,5a,6,10b-octahydroazepino[4,5-b]indol,
(5aR,10bR)-9-[(3,4-Difluorphenyl)sulfonyl]-1,2,3,4,5,5a,6,10b-octahydroazepino[4,5-b]indol,
(5aR,10bR)-9-[(3,5-Difluorphenyl)sulfonyl]-1,2,3,4,5,5a,6,10b-octahydroazepino[4,5-b]indol,
(5aR,10bR)-9-[(4-Methylphenyl)sulfonyl]-1,2,3,4,5,5a,6,10b-octahydroazepino[4,5-b]indol,
(5aR,10bR)-9-[(3-Methylphenyl)sulfonyl]-1,2,3,4,5,5a,6,10b-octahydroazepino[4,5-b]indol,
(5aR,10bR)-9-[(4-Methoxyphenyl)sulfonyl]-1,2,3,4,5,5a,6,10b-octahydroazepino[4,5-b]indol,
(5aR,10bR)-9-[(3-Methoxyphenyl)sulfonyl]-1,2,3,4,5,5a,6,10b-octahydroazepino[4,5-b]indol,
(5aR,10bR)-9-[[(4-Trifluormethyl)phenyl]sulfonyl]-1,2,3,4,5,5a,6,10b-octahydroazepino[4,5-b]indol,
(5aR,10bR)-3-Methyl-9-(phenylsulfonyl)-1,2,3,4,5,5a,6,10b-octahydroazepino[4,5-b]indol,
(5aR,10bR)-9-[(4-Fluorphenyl)sulfonyl]-3-methyl-1,2,3,4,5,5a,6,10b-octahydroazepino[4,5-b]indol,
(5aR,10bR)-9-[(3-Fluorphenyl)sulfonyl]-3-methyl-1,2,3,4,5,5a,6,10b-octahydroazepino[4,5-b]indol,
(5aR,10bR)-9-[(3,4-Difluorphenyl)sulfonyl]-3-methyl-1,2,3,4,5,5a,6,10b-octahydroazepino[4,5-b]indol,
(5aR,10bR)-9-[(3,5-Difluorphenyl)sulfonyl]-3-methyl-1,2,3,4,5,5a,6,10b-octahydroazepino[4,5-b]indol,
(5aR,10bR)-3-Methyl-9-[(4-methylphenyl)sulfonyl]-1,2,3,4,5,5a,6,10b-octahydroazepino[4,5-b]indol,
(5aR,10bR)-3-Methyl-9-[(3-methylphenyl)sulfonyl]-1,2,3,4,5,5a,6,10b-octahydroazepino[4,5-b]indol,
(5aR,10bR)-9-[(4-Methoxyphenyl)sulfonyl]-3-methyl-1,2,3,4,5,5a,6,10b-octahydroazepino[4,5-b]indol,
(5aR,10bR)-9-[(3-Methoxyphenyl)sulfonyl]-3-methyl-1,2,3,4,5,5a,6,10b-octahydroazepino[4,5-b]indol,
(5aR,10bR)-3-Methyl-9-[[(4-trifluormethyl)phenyl]sulfonyl]-1,2,3,4,5,5a,6,10b-octahydroazepino[4,5-b]indol,
(5aR,10bR)-3-Ethyl-9-(phenylsulfonyl)-1,2,3,4,5,5a,6,10b-octahydroazepino[4,5-b]indol,
(5aR,10bR)-3-Ethyl-9-[(4-fluorphenyl)sulfonyl]-1,2,3,4,5,5a,6,10b-octahydroazepino[4,5-b]indol,
(5aR,10bR)-3-Ethyl-9-[(3-fluorphenyl)sulfonyl]-1,2,3,4,5,5a,6,10b-octahydroazepino[4,5-b]indol,
(5aR,10bR)-3-Ethyl-9-[(3,4-difluorphenyl)sulfonyl]-1,2,3,4,5,5a,6,10b-octahydroazepino[4,5-b]indol,
(5aR,10bR)-3-Ethyl-9-[(3,5-difluorphenyl)sulfonyl]-1,2,3,4,5,5a,6,10b-octahydroazepino[4,5-b]indol,
(5aR,10bR)-3-Ethyl-9-[(4-methylphenyl)sulfonyl]-1,2,3,4,5,5a,6,10b-octahydroazepino[4,5-b]indol,
(5aR,10bR)-3-Ethyl-9-[(3-methylphenyl)sulfonyl]-1,2,3,4,5,5a,6,10b-octahydroazepino[4,5-b]indol,
(5aR,10bR)-3-Ethyl-9-[(4-methoxyphenyl)sulfonyl]-1,2,3,4,5,5a,6,10b-octahydroazepino[4,5-b]indol,
(5aR,10bR)-3-Ethyl-9-[(3-methoxyphenyl)sulfonyl]-1,2,3,4,5,5a,6,10b-octahydroazepino[4,5-b]indol,
(5aR,10bR-3-Ethyl-9-[[(4-trifluormethyl)phenyl]sulfonyl]-1,2,3,4,5,5a,6,10b-octahydroazepino[4,5-b]indol,
(5aR,10bR)-1-Methyl-9-(phenylsulfonyl)-1,2,3,4,5,5a,6,10b-octahydroazepino[4,5-b]indol,
(5aR,10bR)-2-Methyl-9-(phenylsulfonyl)-1,2,3,4,5,5a,6,10b-octahydroazepino[4,5-b]indol,
(5aR,10bR)-4-Methyl-9-(phenylsulfonyl)-1,2,3,4,5,5a,6,10b-octahydroazepino[4,5-b]indol,
(5aR,10bR)-5-Methyl-9-(phenylsulfonyl)-1,2,3,4,5,5a,6,10b-octahydroazepino[4,5-b]indol,
(5aR,10bR)-9-[(4-Fluorphenyl)sulfonyl]-1-methyl-1,2,3,4,5,5a,6,10b-octahydroazepino[4,5-b]indol,
(5aR,10bR)-9-[(4-Fluorphenyl)sulfonyl]-2-methyl-1,2,3,4,5,5a,6,10b-octahydroazepino[4,5-b]indol,
(5aR,10bR)-9-[(4-Fluorphenyl)sulfonyl]-4-methyl-1,2,3,4,5,5a,6,10b-octahydroazepino[4,5-b]indol,
(5aR,10bR)-9-[(4-Fluorphenyl)sulfonyl]-5-methyl-1,2,3,4,5,5a,6,10b-octahydroazepino[4,5-b]indol,
(5aR,10bR)-9-[(3-Fluorphenyl)sulfonyl]-1-methyl-1,2,3,4,5,5a,6,10b-octahydroazepino[4,5-b]indol,
(5aR,10bR)-9-[(3-Fluorphenyl)sulfonyl]-2-methyl-1,2,3,4,5,5a,6,10b-octahydroazepino[4,5-b]indol,
(5aR,10bR)-9-[(3-Fluorphenyl)sulfonyl]-4-methyl-1,2,3,4,5,5a,6,10b-octahydroazepino[4,5-b]indol,
(5aR,10bR)-9-[(3-Fluorphenyl)sulfonyl]-5-methyl-1,2,3,4,5,5a,6,10b-octahydroazepino[4,5-b]indol,
(5aR,10bR)-1,5-Dimethyl-9-(phenylsulfonyl)-1,2,3,4,5,5a,6,10b-octahydroazepino[4,5-b]indol,
(5aR,10bR)-2,4-Dimethyl-9-(phenylsulfonyl)-1,2,3,4,5,5a,6,10b-octahydroazepino[4,5-b]indol,
(5aR,10bR)-9-[(4-Fluorphenyl)sulfonyl)]-1,5-dimethyl-1,2,3,4,5,5a,6,10b-octahydroazepino[4,5-b]indol,
(5aR,10bR)-9-[(4-Fluorphenyl)sulfonyl)]-2,4-dimethyl-1,2,3,4,5,5a,6,10b-octahydroazepino[4,5-b]indol,
(5aR,10bR)-9-[(3-Fluorphenyl)sulfonyl)]-1,5-dimethyl-1,2,3,4,5,5a,6,10b-octahydroazepino[4,5-b]indol,
(5aR,10bR)-9-[(3-Fluorphenyl)sulfonyl)]-2,4-dimethyl-1,2,3,4,5,5a,6,10b-octahydroazepino[4,5-b]indol.

8. Verbindung nach Anspruch 1, die eine Isotopmarkierung umfasst.

9. Verbindung nach Anspruch 8, die markiertes 6-Methyl-1,2,3,4,5,5a,6,10b-octahydroazepino[4,5-b]indol-9-ylphenylsulfon oder 6-Methyl-1,2,3,4,5,5a,6,10b-octahydroazepino[4,5-b]indol-9-yl-4-fluorphenylsulfon oder ein pharmazeutisch akzeptables Salz derselben ist.

10. Verbindung nach Anspruch 8, wobei das Isotop aus ³H, ¹¹C, ¹⁴C, ¹³N, ¹⁵O, ¹⁸F, ^{99m}Tc, ¹²³I und ¹²⁵I ausgewählt ist.

11. Verbindung nach Anspruch 10, wobei das Isotop aus ¹¹C, ¹³N, ¹⁵O und ¹⁸F ausgewählt ist.

12. Verbindung nach den Ansprüchen 9 und 11 zur Verwendung bei der Durchführung von Positronemissionstomographie.

13. Verbindung nach Anspruch 9, die 6-Methyl-1,2,3,4,5,5a,6,10b-octahydroazepino[4,5-b]indol-9-yl-4-fluorphenylsulfon oder ein pharmazeutisch akzeptables Salz desselben ist, die ¹⁹F umfasst.

14. Verwendung einer Verbindung nach Anspruch 1 zur Herstellung eines Medikaments zur Behandlung einer Erkrankung oder Störung bei einem Säuger, wobei der 5-HT-Rezeptor beteiligt ist und eine Modulation der 5-HT-Funktion gewünscht wird.

15. Verwendung nach Anspruch 14, wobei die Erkrankung oder Störung Angst, Depression, Schizophrenie, eine stressbedingte Erkrankung, eine Panikstörung, eine Phobie, obsessiv-kompulsive Störung, posttraumatisches Stresssyndrom, Immunsystemdepression, eine Psychose, Paraphrenie, Manie, eine Krampfstörung, eine Persönlichkeitsstörung, Migränekopfschmerz, Drogensucht, Alkoholismus, Fettsucht, eine Essstörung oder eine Schlafstörung ist.

16. Verwendung nach Anspruch 15, wobei die Erkrankung oder Störung Angst, Fettsucht, Depression oder eine stressbedingte Erkrankung ist.

17. Verwendung nach Anspruch 14, wobei die Erkrankung oder Störung psychotische, affektive, vegetative oder psychomotorische Symptome von Schizophrenie oder die extrapyramidal-motorischen Nebenwirkungen anderer Antipsychotika umfasst.

18. Verwendung nach Anspruch 14, wobei die Erkrankung oder Störung Fettsucht, Depression, Schizophrenie, eine schizoide Störung, schizoaffektive Psychose, ein paranoides Syndrom, eine stressbedingte Erkrankung (beispielsweise generalisierte Angststörung), eine Panikstörung, Phobie, obsessiv-kompulsive Störung, posttraumatisches Stresssyndrom, Immunsystemdepression, ein stressinduziertes Problem mit dem uropoetischen, gastrointestinalen oder kardiovaskulären System (beispielsweise Stressinkontinenz), eine neurodegenerative Störung, Autismus, durch eine Chemotherapie induziertes Erbrechen, Hypertonie, Migränekopfschmerz, Clusterkopfschmerz, sexuelle Dysfunktion, eine Suchtstörung, ein Entzugssyndrom, eine Anpassungsstörung, eine altersbedingte Lern- und mentale Störung, Anorexia nervosa, Apathie, Aufmerksamkeitsdefizitsstörung aufgrund allgemeiner medizinischer Zustände, Aufmerksamkeitsdefizit-Hyperaktivitätsstörung, eine Verhaltensstörung (einschließlich von Agitatio bei Zuständen in Verbindung mit verminderter Wahrnehmung, beispielsweise Demenz, Geistesschwäche oder Delirium), eine bipolare Störung, Bulimia nervosa, chronisches Erschöpfungssyndrom, Anpassungsstörung im Sozialverhalten, Cyclothymie, Dysthymie, Fibromyalgie oder eine andere somatoforme Störung, generalisierte Angststörung, eine Inhalationsstörung, Intoxikationsstörung, Bewegungsstörung (beispielsweise Chorea Huntington oder Dyskinesia tardive), Oppositional-Defiant-Störung, periphere Neuropathie, posttraumatische Stressstörung, prämenstrulle Dysphorie, eine psychotische Störung (Störungen kurzer und langer Dauer, eine psychotische Störung aufgrund eines medizinischen Zustands, psychotische Störung NOS), eine affektive Psychose (generalisierte depressive oder bipolare Störung mit psychotischen Merkmalen), saisonal bedingte affektive Psychose, Schlafstörung, spezifische Entwicklungsstörung, Agitationsstörung, Stressinkontinenz, wie Dranginkontinenz oder Mischinkontinenz, selektive Serotoninwiederaufnahmehemmung(SSRI)-"Poop-Out"-Syndrom oder Tourette-Syndrom ist.

## Revendications

1. Composé de formule I 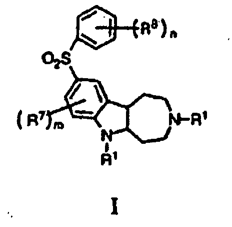 dans laquelle chaque R¹ est indépendamment
a) H,
b) un groupe alkyle en C₁ à C₄,
c) un groupe alkyle en C₁ à C₄ substitué par un groupe phényle, le groupe phényle étant facultativement substitué par un ou deux R², ou
d) un groupe phényle, facultativement substitué par un ou deux R² ;
R² est
a) un halogène,
b) OR³,
c) CF₃,
d) C (=O) -NR⁴R⁵,
e) NH-SO₂-R⁶,
f) NR⁴R⁵,
g) NR⁴-C(=O)-R⁴,
h) SO₂-NR⁴R⁵,
i) CN, ou
j) NO₂ ;
R³ est H, un groupe alkyle en C₁ à C₄ ou phényle ;
R⁴ et R⁵ sont indépendamment H, un groupe alkyle en C₁ à C₄, ou bien R⁴ et R⁵, pris ensemble avec l'atome d'azote auquel ils sont liés, forment un cycle choisi dans la classe formée par les groupes 1-pyrrolidinyle, 1-pipérazinyle et 1-morpholinyle ;
R⁶ est H ou un groupe alkyle en C₁ à C₄ ;
R⁷ est
a) H, ou
b) un halogène,
c) OR³,
d) CF₃,
e) C (=O)-NR⁴R⁵,
f) NH-SO₂-R⁶,
g) NR⁴R⁵,
h) NR⁴-C(=O)-R⁴,
i) SO₂-NR⁴R⁵,
j) CN, ou
k) NO₂ ;
R⁸ est
a) H,
b) F,
c) Cl,
d) un groupe alkyle en C₁ à C₄,
e) un groupe alkoxy en C₁ à C₃,
f) CF₃,
g) un groupe alkyle en C₁ à C₄ substitué par un groupe phényle, le groupe phényle étant facultativement substitué par un ou deux R²,
h) un groupe phényle, facultativement substitué par un ou deux R²,
i) OR³,
j) CO-NR⁴R⁵,
k) NR⁴R⁵,
l) NH-SO₂-R⁶, ou
m) NH-CO-R⁴ ;
à chaque occurrence, un groupe alkyle ou alkoxy est facultativement substitué par OH, un halogène ou NH₂ ; m est 1 à 2 ; et n est 1 à 3 ;
ou un sel pharmaceutiquement acceptable de ce composé.

2. Composé selon la revendication 1, dans lequel chaque R¹ est indépendamment H, un groupe méthyle ou éthyle.

3. Composé selon la revendication 1, dans lequel R⁷ est l'hydrogène.

4. Composé selon la revendication 1, dans lequel R⁸ est l'hydrogène.

5. Composé selon la revendication 1, dans lequel R⁸ est F, OCH₃ ou CF₃.

6. Composé selon la revendication 1, qui est
(a) le chlorhydrate de (R,R)-6-méthyl-1,2,3,4,5,5a,6,10b-octahydroazépino[4,5-b]indole-9-yl-phényl-sulfone,
(b) le chlorhydrate de (S,S)-6-méthyl-1,2,3,4,5,5a,6,10b-octahydroazépino[4,5-b]indole-9-yl-phényl-sulfone,
(c) le chlorhydrate de (R,S)-6-méthyl-1,2,3,4,5,5a,6,10b-octahydroazépino[4,5-b]indole-9-yl-phényl-sulfone, ou
(d) le chlorhydrate de (S,R)-6-méthyl-1,2,3,4,5,5a,6,10b-octahydroazépino[4,5-b]indole-9-yl-phényl-sulfone.

7. Composé choisi parmi les suivants :
(5aS,10bS)-9-[(4-fluorophényl)sulfonyl]-6-méthyl-1,2,3,4,5,5a,6,10b-octahydroazépino[4,5-b]indole ;
(5aS,10bS)-9-[(3-fluorophényl)sulfonyl]-6-méthyl-1,2,3,4,5,5a,6,10b-octahydroazépino[4,5-b]indole ;
(5aS,10bS)-9-[(3,4-difluorophényl)sulfonyl]-6-méthyl-1,2,3,4,5,5a,6,10b-octahydroazépino[4,5-b]indole ;
(5aS,10bS)-9-[(3,5-difluorophényl)sulfonyl]-6-méthyl-1,2,3,4,5,5a,6,10b-octahydroazépino[4,5-b]indole ;
(5aS,10bS)-6-méthyl-9-[(4-méthylphényl)sulfonyl]-1,2,3,4,5,5a,6,10b-octahydroazépino[4,5-b]indole ;
(5aS,10bS)-6-méthyl-9-[(3-méthylphényl)sulfonyl]-1,2,3,4,5,5a,6,10b-octahydroazépino[4,5-b]indole ;
(5aS,10bS)-9-[(4-méthoxyphényl)sulfonyl]-6-méthyl-1,2,3,4,5,5a,6,10b-octahydroazépino[4,5-b]indole ;
(5aS,10bS)-9-[(3-méthoxyphényl)sulfonyl]-6-méthyl-1,2,3,4,5,5a,6,10b-octahydroazépino[4,5-b]indole ;
(5aS,10bS)-6-méthyl-9-[[(4-trifluorométhyl)phényl]sulfonyl]-1,2,3,4,5,5a,6,10b-octahydroazépino[4,5-b]indole ;
(5aS,10bS)-3,6-diméthyl-9-(phénylsulfonyl)-1,2,3,4,5,5a,6,10b-octahydroazépino[4,5-b]indole ;
(5aS,10bS)-9-[(4-fluorophényl)sulfonyl]-3,6-diméthyl-1,2,3,4,5,5a,6,10b-octahydroazépino[4,5-b]indole ;
(5aS,10bS)-9-[(3-fluorophényl)sulfonyl]-3,6-diméthyl-1,2,3,4,5,5a,6,10b-octahydroazépino[4,5-b]indole ;
(5aS,10bS)-9-[(3,4-difluorophényl)sulfonyl]-3,6-diméthyl-1,2,3,4,5,5a,6,10b-octahydroazépino[4,5-b]indole ;
(5aS,10bS)-9-[(3,5-difluorophényl)sulfonyl]-3,6-diméthyl-1,2,3,4,5,5a,6,10b-octahydroazépino[4,5-b]indole ;
(5aS,10bS)-3,6-diméthyl-9-[(4-méthylphényl)sulfonyl]-1,2,3,4,5,5a,6,10b-octahydroazépino[4,5-b]indole ;
(5aS,10bS)-3,6-diméthyl-9-[(3-méthylphényl)sulfonyl]-1,2,3,4,5,5a,6,10b-octahydroazépino[4,5-b]indole ;
(5aS,10bS)-9-[(4-méthoxyphényl)sulfonyl]-3,6-diméthyl-1,2,3,4,5,5a,6,10b-octahydroazépino[4,5-b]indole ;
(5aS,10bS)-9-[(3-méthoxyphényl)sulfonyl]-3,6-diméthyl-1,2,3,4,5,5a,6,10b-octahydroazépino[4,5-b]indole ;
(5aS,10bS)-3,6-diméthyl-9-[[(4-trifluorométhyl)phényl]-sulfonyl]-1,2,3,4,5,5a,6,10b-octahydroazépino[4,5-b]indole ;
(5aS,10bS)-3-éthyl-6-méthyl-9-(phénylsulfonyl)-1,2,3,4,5,5a,6,10b-octahydroazépino[4,5-b]indole ;
(5aS,10bS)-3-éthyl-9-[(4-fluorophényl)sulfonyl]-6-méthyl-1,2,3,4,5,5a,6,10b-octahydroazépino[4,5-b]indole ;
(5aS,10bS)-3-éthyl-9-[(3-fluorophényl)sulfonyl]-6-méthyl-1,2,3,4,5,5a,6,10b-octahydroazépino[4,5-b]indole ;
(5aS,10bS)-3-éthyl-9-[(3,4-difluorophényl)sulfonyl]-6-méthyl-1,2,3,4,5,5a,6,10b-octahydroazépino[4,5-b]indole ;
(5aS,10bS)-3-éthyl-9-[(3,5-difluorophényl)sulfonyl]-6-méthyl-1,2,3,4,5,5a,6,10b-octahydroazépino[4,5-b]indole ;
(5aS,10bS)-3-éthyl-6-méthyl-9-[(4-méthylphényl)sulfonyl]-1,2,3,4,5,5a,6,10b-octahydroazépino[4,5-b]indole ;
(5aS,10bS)-3-éthyl-6-méthyl-9-[(3-méthylphényl)sulfonyl]-1,2,3,4,5,5a,6,10b-octahydroazépino[4,5-b]indole ;
(5aS,10bS)-3-éthyl-9-[(4-méthoxyphényl)sulfonyl]-6-méthyl-1,2,3,4,5,5a,6,10b-octahydroazépino[4,5-b]indole ;
(5aS,10bS)-3-éthyl-9-[(3-méthoxyphényl)sulfonyl]-6-méthyl-1,2,3,4,5,5a,6,10b-octahydroazépino[4,5-b]indole ;
(5aS,10bS)-3-éthyl-6-méthyl-9-[[(4-trifluorométhyl)phényl]-sulfonyl]-1,2,3,4,5,5a,6,10b-octahydroazépino[4,5-b]indole ;
(5aS,10bS)-1,6-diméthyl-9-(phénylsulfonyl)-1,2,3,4,5,5a,6,10b-octahydroazépino[4,5-b]indole ;
(5aS,10bS)-2,6-diméthyl-9-(phénylsulfonyl)-1,2,3,4,5,5a,6,10b-octahydroazépino[4,5-b]indole ;
(5aS,10bS)-4,6-diméthyl-9-(phénylsulfonyl)-1,2,3,4,5,5a,6,10b-octahydroazépino[4,5-b]indole ;
(5aS,10bS)-5,6-diméthyl-9-(phénylsulfonyl)-1,2,3,4,5,5a,6,10b-octahydroazépino[4,5-b]indole ;
(5aS,10bS)-9-[(4-fluorophényl)sulfonyl]-1,6-diméthyl-1,2,3,4,5,5a,6,10b-octahydroazépino[4,5-b]indole ;
(5aS,10bS)-9-[(4-fluorophényl)sulfonyl]-2,6-diméthyl-1,2,3,4,5,5a,6,10b-octahydroazépino[4,5-b]indole ;
(5aS,10bS)-9-[(4-fluorophényl)sulfonyl]-4,6-diméthyl-1,2,3,4,5,5a,6,10b-octahydroazépino[4,5-b]indole ;
(5aS,10bS)-9-[(4-fluorophényl)sulfonyl]-5,6-diméthyl-1,2,3,4,5,5a,6,10b-octahydroazépino[4,5-b]indole ;
(5aS,10bS)-9-[(3-fluorophényl)sulfonyl]-1,6-diméthyl-1,2,3,4,5,5a,6,10b-octahydroazépino[4,5-b]indole ;
(5aS,10bS)-9-[(3-fluorophényl)sulfonyl]-2,6-diméthyl-1,2,3,4,5,5a,6,10b-octahydroazépino[4,5-b]indole ;
(5aS,10bS)-9-[(3-fluorophényl)sulfonyl]-4,6-diméthyl-1,2,3,4,5,5a,6,10b-octahydroazépino[4,5-b]indole ;
(5aS,10bS)-9-[(3-fluorophényl)sulfonyl]-5,6-diméthyl-1,2,3,4,5,5a,6,10b-octahydroazépino[4,5-b]indole ;
(5aS,10bS)-1,5,6-triméthyl-9-(phénylsulfonyl)-1,2,3,4,5,5a,6,10b-octahydroazépino[4,5-b]indole ;
(5aS,10bS)-2,4,6-triméthyl-9-(phénylsulfonyl)-1,2,3,4,5,5a,6,10b-octahydroazépino[4,5-b]indole ;
(5aS,10bS)-9-[(4-fluorophényl)sulfonyl]-1,5,6-triméthyl-1,2,3,4,5,5a,6,10b-octahydroazépino[4,5-b]indole ;
(5aS,10bS)-9-[(4-fluorophényl)sulfonyl]-2,4,6-triméthyl-1,2,3,4,5,5a,6,10b-octahydroazépino[4,5-b]indole ;
(5aS,10bS)-9-[(3-fluorophényl)sulfonyl]-1,5,6-triméthyl-1,2,3,4,5,5a,6,10b-octahydroazépino[4,5-b]indole ;
(5aS,10bS)-9-[(3-fluorophényl)sulfonyl]-2,4,6-triméthyl-1,2,3,4,5,5a,6,10b-octahydroazépino[4,5-b]indole ;
(5aS,10bS)-6-méthyl-9-(phénylsulfonyl)-1,2,3,4,5,5a,6,10b-octahydroazépino[4,5-b]indole ;
(5aS,10bS)-9-[(4-fluorophényl)sulfonyl]-1,2,3,4,5,5a,6,10b-octahydroazépino[4,5-b]indole ;
(5aS,10bS)-9-[(3-fluorophényl)sulfonyl]-1,2,3,4,5,5a,6,10b-octahydroazépino[4,5-b]indole ;
(5aS,10bS)-9-[(3,4-difluorophényl)sulfonyl]-1,2,3,4,5,5a,6,10b-octahydroazépino[4,5-b]indole ;
(5aS,10bS)-9-[(3,5-difluorophényl)sulfonyl]-1,2,3,4,5,5a,6,10b-octahydroazépino[4,5-b]indole ;
(5aS,10bS)-9-[(4-méthylphényl)sulfonyl]-1,2,3,4,5,5a,6,10b-octahydroazépino[4,5-b]indole ;
(5aS,10bS)-9-[(3-méthylphényl)sulfonyl]-1,2,3,4,5,5a,6,10b-octahydroazépino[4,5-b]indole ;
(5aS,10bS)-9-[(4-méthoxyphényl)sulfonyl]-1,2,3,4,5,5a,6,10b-octahydroazépino[4,5-b]indole ;
(5aS,10bS)-9-[(3-méthoxyphényl)sulfonyl]-1,2,3,4,5,5a,6,10b-octahydroazépino[4,5-b]indole ;
(5aS,10bS)-9- [[(4-trifluorométhyl)phényl]sulfonyl]-1,2,3,4,5,5a,6,10b-octahydroazépino[4,5-b]indole ;
(5aS,10bS)-3-méthyl-9-(phénylsulfonyl)-1,2,3,4,5,5a,6,10b-octahydroazépino[4,5-b]indole ;
(5aS,10bS)-9-[(4-fluorophényl)sulfonyl]-3-méthyl-1,2,3,4,5,5a,6,10b-octahydroazépino[4,5-b]indole ;
(5aS,10bS)-9-[(3-fluorophényl)sulfonyl]-3-méthyl-1,2,3,4,5,5a,6,10b-octahydroazépino[4,5-b]indole ;
(5aS,10bS)-9-[(3,4-difluorophényl)sulfonyl]-3-méthyl-1,2,3,4,5,5a,6,10b-octahydroazépino[4,5-b]indole ;
(5aS,10bS)-9-[(3,5-difluorophényl)sulfonyl]-3-méthyl-1,2,3,4,5,5a,6,10b-octahydroazépino[4,5-b]indole ;
(5aS,10bS)-3-méthyl-9-[(4-méthylphényl)sulfonyl]-1,2,3,4,5,5a,6,10b-octahydroazépino[4,5-b]indole ;
(5aS,10bS)-3-méthyl-9-[(3-méthylphényl)sulfonyl]-1,2,3,4,5,5a,6,10b-octahydroazépino[4,5-b]indole ;
(5aS,10bS)-9-[(4-méthoxyphényl)sulfonyl]-3-méthyl-1,2,3,4,5,5a,6,10b-octahydroazépino[4,5-b]indole ;
(5aS,10bS)-9-[(3-méthoxyphényl)sulfonyl]-3-méthyl-1,2,3,4,5,5a,6,10b-octahydroazépino[4,5-b]indole ;
(5aS,10bS)-3-méthyl-9-[[(4-trifluorométhyl)phényl]sulfonyl]-1,2,3,4,5,5a,6,10b-octahydroazépino[4,5-b]indole ;
(5aS,10bS)-3-éthyl-9-(phénylsulfonyl)-1,2,3,4,5,5a,6,10b-octahydroazépino[4,5-b]indole ;
(5aS,10bS)-3-éthyl-9-[(4-fluorophényl)sulfonyl]-1,2,3,4,5,5a,6,10b-octahydroazépino[4,5-b]indole ;
(5aS,10bS)-3-éthyl-9-[(3-fluorophényl)sulfonyl]-1,2,3,4,5,5a,6,10b-octahydroazépino[4,5-b]indole ;
(5aS,10bS)-3-éthyl-9-[(3,4-difluorophényl)sulfonyl]-1,2,3,4,5,5a,6,10b-octahydroazépino[4,5-b]indole ;
(5aS,10bS)-3-éthyl-9-[(3,5-difluorophényl)sulfonyl]-1,2,3,4,5,5a,6,10b-octahydroazépino[4,5-b]indole ;
(5aS,10bS)-3-éthyl-9-[(4-méthylphényl)sulfonyl]-1,2,3,4,5,5a,6,10b-octahydroazépino[4,5-b]indole ;
(5aS,10bS)-3-éthyl-9-[(3-méthylphényl)sulfonyl]-1,2,3,4,5,5a,6,10b-octahydroazépino[4,5-b]indole ;
(5aS,10bS)-3-éthyl-9-[(4-méthoxyphényl)sulfonyl]-1,2,3,4,5,5a,6,10b-octahydroazépino[4,5-b]indole ;
(5aS,10bS)-3-éthyl-9-[(3-méthoxyphényl)sulfonyl]-1,2,3,4,5,5a,6,10b-octahydroazépino[4,5-b]indole ;
(5aS,10bS)-3-éthyl-9-[[(4-trifluorométhyl)phényl]sulfonyl]-1,2,3,4,5,5a,6,10b-octahydroazépino[4,5-b]indole ;
(5aS,10bS)-1-méthyl-9-(phénylsulfonyl)-1,2,3,4,5,5a,6,10b-octahydroazépino[4,5-b]indole ;
(5aS,10bS)-2-méthyl-9-(phénylsulfonyl)-1,2,3,4,5,5a,6,10b-octahydroazépino[4,5-b]indole ;
(5aS,10bS)-4-méthyl-9-(phénylsulfonyl)-1,2,3,4,5,5a,6,10b-octahydroazépino[4,5-b]indole ;
(5aS,10bS)-5-méthyl-9-(phénylsulfonyl)-1,2,3,4,5,5a,6,10b-octahydroazépino[4,5-b]indole ;
(5aS,10bS)-9-[(4-fluorophényl)sulfonyl]-1-méthyl-1,2,3,4,5,5a,6,10b-octahydroazépino[4,5-b]indole ;
(5aS,10bS)-9-[(4-fluorophényl)sulfonyl]-2-méthyl-1,2,3,4,5,5a,6,10b-octahydroazépino[4,5-b]indole ;
(5aS,10bS)-9-[(4-fluorophényl)sulfonyl]-4-méthyl-1,2,3,4,5,5a,6,10b-octahydroazépino[4,5-b]indole ;
(5aS,10bS)-9-[(4-fluorophényl)sulfonyl]-5-méthyl-1,2,3,4,5,5a,6,10b-octahydroazépino[4,5-b]indole ;
(5aS,10bS)-9-[(3-fluorophényl)sulfonyl]-1-méthyl-1,2,3,4,5,5a,6,10b-octahydroazépino[4,5-b]indole ;
(5aS,10bS)-9-[(3-fluorophényl)sulfonyl]-2-méthyl-1,2,3,4,5,5a,6,10b-octahydroazépino[4,5-b]indole ;
(5aS,10bS)-9-[(3-fluorophényl)sulfonyl]-4-méthyl-1,2,3,4,5,5a,6,10b-octahydroazépino[4,5-b]indole ;
(5aS,10bS)-9-[(3-fluorophényl)sulfonyl]-5-méthyl-1,2,3,4,5,5a,6,10b-octahydroazépino[4,5-b]indole ;
(5aS,10bS)-1,5-diméthyl-9-(phénylsulfonyl)-1,2,3,4,5,5a,6,10b-octahydroazépino[4,5-b]indole ;
(5aS,10bS)-2,4-diméthyl-9-(phénylsulfonyl)-1,2,3,4,5,5a,6,10b-octahydroazépino[4,5-b]indole ;
(5aS,10bS)-9-[(4-fluorophényl)sulfonyl]-1,5-diméthyl-1,2,3,4,5,5a,6,10b-octahydroazépino[4,5-b]indole ;
(5aS,10bS)-9-[(4-fluorophényl)sulfonyl]-2,4-diméthyl-1,2,3,4,5,5a,6,10b-octahydroazépino[4,5-b]indole ;
(5aS,10bS)-9-[(3-fluorophényl)sulfonyl]-1,5-diméthyl-1,2,3,4,5,5a,6,10b-octahydroazépino[4,5-b]indole ;
(5aS,10bS)-9-[(3-fluorophényl)sulfonyl]-2,4-diméthyl-1,2,3,4,5,5a,6,10b-octahydroazépino[4,5-b]indole ;
(5aR,10bS)-9-[(4-fluorophényl)sulfonyl]-6-méthyl-1,2,3,4,5,5a,6,10b-octahydroazépino[4,5-b]indole ;
(5aR,10bS)-9-[(3-fluorophényl)sulfonyl]-6-méthyl-1,2,3,4,5,5a,6,10b-octahydroazépino[4,5-b]indole ;
(5aR,10bS)-9-[(3,4-difluorophényl)sulfonyl]-6-méthyl-1,2,3,4,5,5a,6,10b-octahydroazépino[4,5-b]indole ;
(5aR,10bS)-9-[(3,5-difluorophényl)sulfonyl]-6-méthyl-1,2,3,4,5,5a,6,10b-octahydroazépino[4,5-b]indole ;
(5aR,10bS)-6-méthyl-9-[(4-méthylphényl)sulfonyl]-1,2,3,4,5,5a,6,10b-octahydroazépino[4,5-b]indole ;
(5aR,10bS)-6-méthyl-9-[(3-méthylphényl)sulfonyl]-1,2,3,4,5,5a,6,10b-octahydroazépino[4,5-b]indole ;
(5aR,10bS)-9-[(4-méthoxyphényl)sulfonyl]-6-méthyl-1,2,3,4,5,5a,6,10b-octahydroazépino[4,5-b]indole ;
(5aR,10bS)-9-[(3-méthoxyphényl)sulfonyl]-6-méthyl-1,2,3,4,5,5a,6,10b-octahydroazépino[4,5-b]indole ;
(5aR,10bS)-6-méthyl-9-[[(4-trifluorométhyl)phényl]sulfonyl]-1,2,3,4,5,5a,6,10b-octahydroazépino[4,5-b]indole ;
(5aR,10bS)-3,6-diméthyl-9-(phénylsulfonyl)-1,2,3,4,5,5a,6,10b-octahydroazépino[4,5-b]indole ;
(5aR,10bS)-9-[(4-fluorophényl)sulfonyl]-3,6-diméthyl-1,2,3,4,5,5a,6,10b-octahydroazépino[4,5-b]indole ;
(5aR,10bS)-9-[(3-fluorophényl)sulfonyl]-3,6-diméthyl-1,2,3,4,5,5a,6,10b-octahydroazépino[4,5-b]indole ;
(5aR,10bS)-9-[(3,4-difluorophényl)sulfonyl]-3,6-diméthyl-1,2,3,4,5,5a,6,10b-octahydroazépino[4,5-b]indole ;
(5aR,10bS)-9-[(3,5-difluorophényl)sulfonyl]-3,6-diméthyl-1,2,3,4,5,5a,6,10b-octahydroazépino[4,5-b]indole ;
(5aR,10bS)-3,6-diméthyl-9-[(4-méthylphényl)sulfonyl]-1,2,3,4,5,5a,6,10b-octahydroazépino[4,5-b]indole ;
(5aR,10bS)-3,6-diméthyl-9-[(3-méthylphényl)sulfonyl]-1,2,3,4,5,5a,6,10b-octahydroazépino[4,5-b]indole ;
(5aR,10bS)-9-[(4-méthoxyphényl)sulfonyl]-3,6-diméthyl-1,2,3,4,5,5a,6,10b-octahydroazépino[4,5-b]indole ;
(5aR,10bS)-9-[(3-méthoxyphényl)sulfonyl]-3,6-diméthyl-1,2,3,4,5,5a,6,10b-octahydroazépino[4,5-b]indole ;
(5aR,10bS)-3,6-diméthyl-9-[[(4-trifluorométhyl)phényl]-sulfonyl]-1,2,3,4,5,5a,6,10b-octahydroazépino[4,5-b]indole ;
(5aR,10bS)-3-éthyl-6-méthyl-9-(phénylsulfonyl)-1,2,3,4,5,5a,6,10b-octahydroazépino[4,5-b]indole ;
(5aR,10bS)-3-éthyl-9-[(4-fluorophényl)sulfonyl]-6-méthyl-1,2,3,4,5,5a,6,10b-octahydroazépino[4,5-b]indole ;
(5aR,10bS)-3-éthyl-9-[(3-fluorophényl)sulfonyl]-6-méthyl-1,2,3,4,5,5a,6,10b-octahydroazépino[4,5-b]indole ;
(5aR,10bS)-3-éthyl-9-[(3,4-difluorophényl)sulfonyl]-6-méthyl-1,2,3,4,5,5a,6,10b-octahydroazépino[4,5-b]indole ;
(5aR,10bS)-3-éthyl-9-[(3,5-difluorophényl)sulfonyl]-6-méthyl-1,2,3,4,5,5a,6,10b-octahydroazépino[4,5-b]indole ;
(5aR,10bS)-3-éthyl-6-méthyl-9-[(4-méthylphényl)sulfonyl]-1,2,3,4,5,5a,6,10b-octahydroazépino[4,5-b]indole ;
(5aR,10bS)-3-éthyl-6-méthyl-9-[(3-méthylphényl)sulfonyl]-1,2,3,4,5,5a,6,10b-octahydroazépino[4,5-b]indole ;
(5aR,10bS)-3-éthyl-9-[(4-méthoxyphényl)sulfonyl]-6-méthyl-1,2,3,4,5,5a,6,10b-octahydroazépino[4,5-b]indole ;
(5aR,10bS)-3-éthyl-9-[(3-méthoxyphényl)sulfonyl]-6-méthyl-1,2,3,4,5,5a,6,10b-octahydroazépino[4,5-b]indole ;
(5aR,10bS)-3-éthyl-6-méthyl-9-[[(4-trifluorométhyl)phényl]-sulfonyl]-1,2,3,4,5,5a,6,10b-octahydroazépino[4,5-b]indole ;
(5aR,10bS)-1,6-diméthyl-9-(phénylsulfonyl)-1,2,3,4,5,5a,6,10b-octahydroazépino[4,5-b]indole ;
(5aR,10bS)-2,6-diméthyl-9-(phénylsulfonyl)-1,2,3,4,5,5a,6,10b-octahydroazépino[4,5-b]indole ;
(5aR,10bS)-4,6-diméthyl-9-(phénylsulfonyl)-1,2,3,4,5,5a,6,10b-octahydroazépino[4,5-b]indole ;
(5aR,10bS)-5,6-diméthyl-9-(phénylsulfonyl)-1,2,3,4,5,5a,6,10b-octahydroazépino[4,5-b]indole ;
(5aR,10bS)-9-[(4-fluorophényl)sulfonyl]-1,6-diméthyl-1,2,3,4,5,5a,6,10b-octahydroazépino[4,5-b]indole ;
(5aR,10bS)-9-[(4-fluorophényl)sulfonyl]-2,6-diméthyl-1,2,3,4,5,5a,6,10b-octahydroazépino[4,5-b]indole ;
(5aR,10bS)-9-[(4-fluorophényl)sulfonyl]-4,6-diméthyl-1,2,3,4,5,5a,6,10b-octahydroazépino[4,5-b]indole ;
(5aR,10bS)-9-[(4-fluorophényl)sulfonyl]-5,6-diméthyl-1,2,3,4,5,5a,6,10b-octahydroazépino[4,5-b]indole ;
(5aR,10bS)-9-[(3-fluorophényl)sulfonyl]-1,6-diméthyl-1,2,3,4,5,5a,6,10b-octahydroazépino[4,5-b]indole ;
(5aR,10bS)-9-[(3-fluorophényl)sulfonyl]-2,6-diméthyl-1,2,3,4,5,5a,6,10b-octahydroazépino[4,5-b]indole ;
(5aR,10bS)-9-[(3-fluorophényl)sulfonyl]-4,6-diméthyl-1,2,3,4,5,5a,6,10b-octahydroazépino[4,5-b]indole ;
(5aR,10bS)-9-[(3-fluorophényl)sulfonyl]-5,6-diméthyl-1,2,3,4,5,5a,6,10b-octahydroazépino[4,5-b]indole ;
(5aR,10bS)-1,5,6-triméthyl-9-(phénylsulfonyl)-1,2,3,4,5,5a,6,10b-octahydroazépino[4,5-b]indole ;
(5aR,10bs)-2,4,6-triméthyl-9-(phénylsulfonyl)-1,2,3,4,5,5a,6,10b-octahydroazépino[4,5-b]indole ;
(5aR,10bS)-9-[(4-fluorophényl)sulfonyl]-1,5,6-triméthyl-1,2,3,4,5,5a,6,10b-octahydroazépino[4,5-b]indole ;
(5aR,10bs)-9-[(4-fluorophényl)sulfonyl]-2,4,6-triméthyl-1,2,3,4,5,5a,6,10b-octahydroazépino[4,5-b]indole ;
(5aR,10bS)-9-[(3-fluorophényl)sulfonyl]-1,5,6-triméthyl-1,2,3,4,5,5a,6,10b-octahydroazépino[4,5-b]indole ;
(5aR,10bs)-9-[(3-fluorophényl)sulfonyl]-2,4,6-triméthyl-1,2,3,4,5,5a,6,10b-octahydroazépino[4,5-b]indole ;
(5aR,10bs)-6-méthyl-9-(phénylsulfonyl)-1,2,3,4,5,5a,6,10b-octahydroazépino[4,5-b]indole ;
(5aR,10bs)-9-[(4-fluorophényl)sulfonyl]-1,2,3,4,5,5a,6,10b-octahydroazépino[4,5-b]indole ;
(5aR,10bs)-9-[(3-fluorophényl)sulfonyl]-1,2,3,4,5,5a,6,10b-octahydroazépino[4,5-b]indole ;
(5aR,10bS)-9-[(3,4-difluorophényl)sulfonyl]-1,2,3,4,5,5a,6,10b-octahydroazépino[4,5-b]indole ;
(5aR,10bS)-9-[(3,5-difluorophényl)sulfonyl]-1,2,3,4,5,5a,6,10b-octahydroazépino[4,5-b]indole ;
(5aR,10bS)-9-[(4-méthylphényl)sulfonyl]-1,2,3,4,5,5a,6,10b-octahydroazépino[4,5-b]indole ;
(5aR,10bs)-9-[(3-méthylphényl)sulfonyl]-1,2,3,4,5,5a,6,10b-octahydroazépino[4,5-b]indole ;
(5aR,10bs)-9-[(4-méthoxyphényl)sulfonyl]-1,2,3,4,5,5a,6,10b-octa-hydroazépino[4,5-b]indole ;
(5aR,10bS)-9-[(3-méthoxyphényl)sulfonyl]-1,2,3,4,5,5a,6,10b-octahydroazépino[4,5-b]indole ;
(5aR,10bS)-9-[[(4-trifluorométhyl)phényl]sulfonyl]-1,2,3,4,5,5a,6,10b-octahydroazépino[4,5-b]indole ;
(5aR,10bs)-3-méthyl-9-(phénylsulfonyl)-1,2,3,4,5,5a,6,10b-octahydroazépino[4,5-b]indole ;
(5aR,10bS)-9-[(4-fluorophényl)sulfonyl]-3-méthyl-1,2,3,4,5,5a,6,10b-octahydroazépino[4,5-b]indole ;
(5aR,10bS)-9-[(3-fluorophényl)sulfonyl]-3-méthyl-1,2,3,4,5,5a,6,10b-octahydroazépino[4,5-b]indole ;
(5aR,10bS)-9- [(3,4-difluorophényl)sulfonyl]-3-méthyl-1,2,3,4,5,5a,6,10b-octahydroazépino[4,5-b]indole ;
(5aR,10bS)-9-[(3,5-difluorophényl)sulfonyl]-3-méthyl-1,2,3,4,5,5a,6,10b-octahydroazépino[4,5-b]indole ;
(5aR,10bS)-3-méthyl-9-[(4-méthylphényl)sulfonyl]-1,2,3,4,5,5a,6,10b-octahydroazépino[4,5-b]indole ;
(5aR,10bS)-3-méthyl-9-[(3-méthylphényl)sulfonyl]-1,2,3,4,5,5a,6,10b-octahydroazépino[4,5-b]indole ;
(5aR,10bS)-9-[(4-méthoxyphényl)sulfonyl]-3-méthyl-1,2,3,4,5,5a,6,10b-octahydroazépino[4,5-b]indole ;
(5aR,10bS)-9-[(3-méthoxyphényl)sulfonyl]-3-méthyl-1,2,3,4,5,5a,6,10b-octahydroazépino[4,5-b]indole ;
(5aR,10bS)-3-méthyl-9-[[(4-trifluorométhyl)phényl]sulfonyl]-1,2,3,4,5,5a,6,10b-octahydroazépino[4,5-b]indole ;
(5aR,10bS)-3-éthyl-9-(phénylsulfonyl)-1,2,3,4,5,5a,6,10b-octahydroazépino[4,5-b]indole ;
(5aR,10bS)-3-éthyl-9-[(4-fluorophényl)sulfonyl]-1,2,3,4,5,5a,6,10b-octahydroazépino[4,5-b]indole ;
(5aR,10bS)-3-éthyl-9-[(3-fluorophényl)sulfonyl]-1,2,3,4,5,5a,6,10b-octahydroazépino[4,5-b]indole ;
(5aR,10bS)-3-éthyl-9-[(3,4-difluorophényl)sulfonyl]-1,2,3,4,5,5a,6,10b-octahydroazépino[4,5-b]indole ;
(5aR,10bS)-3-éthyl-9-[(3,5-difluorophényl)sulfonyl]-1,2,3,4,5,5a,6,10b-octahydroazépino[4,5-b]indole ;
(5aR,10bS)-3-éthyl-9-[(4-méthylphényl)sulfonyl]-1,2,3,4,5,5a,6,10b-octahydroazépino[4,5-b]indole ;
(5aR,10bS)-3-éthyl-9-[(3-méthylphényl)sulfonyl]-1,2,3,4,5,5a,6,10b-octahydroazépino[4,5-b]indole ;
(5aR,10bS)-3-éthyl-9-[(4-méthoxyphényl)sulfonyl]-1,2,3,4,5,5a,6,10b-octahydroazépino[4,5-b]indole ;
(5aR,10bS)-3-éthyl-9-[(3-méthoxyphényl)sulfonyl]-1,2,3,4,5,5a,6,10b-octahydroazépino[4,5-b]indole ;
(5aR,10bS)-3-éthyl-9-[[(4-trifluorométhyl)phényl]sulfonyl]-1,2,3,4,5,5a,6,10b-octahydroazépino[4,5-b]indole ;
(5aR,10bS)-1-méthyl-9-(phénylsulfonyl)-1,2,3,4,5,5a,6,10b-octahydroazépino[4,5-b]indole ;
(5aR,10bS)-2-méthyl-9-(phénylsulfonyl)-1,2,3,4,5,5a,6,10b-octahydroazépino[4,5-b]indole ;
(5aR,10bS)-4-méthyl-9-(phénylsulfonyl)-1,2,3,4,5,5a,6,10b-octahydroazépino[4,5-b]indole ;
(5aR,10bS)-5-méthyl-9-(phénylsulfonyl)-1,2,3,4,5,5a,6,10b-octahydroazépino[4,5-b]indole ;
(5aR,10bS)-9-[(4-fluorophényl)sulfonyl]-1-méthyl-1,2,3,4,5,5a,6,10b-octahydroazépino[4,5-b]indole ;
(5aR,10bS)-9-[(4-fluorophényl)sulfonyl]-2-méthyl-1,2,3,4,5,5a,6,10b-octahydroazépino[4,5-b]indole ;
(5aR,10bS)-9-[(4-fluorophényl)sulfonyl]-4-méthyl-1,2,3,4,5,5a,6,10b-octahydroazépino[4,5-b]indole ;
(5aR,10bS)-9-[(4-fluorophényl)sulfonyl]-5-méthyl-1,2,3,4,5,5a,6,10b-octahydroazépino[4,5-b]indole ;
(5aR,10bS)-9-[(3-fluorophényl)sulfonyl]-1-méthyl-1,2,3,4,5,5a,6,10b-octahydroazépino[4,5-b]indole ;
(5aR,10bS)-9-[(3-fluorophényl)sulfonyl]-2-méthyl-1,2,3,4,5,5a,6,10b-octahydroazépino[4,5-b]indole ;
(5aR,10bS)-9-[(3-fluorophényl)sulfonyl]-4-méthyl-1,2,3,4,5,5a,6,10b-octahydroazépino[4,5-b]indole ;
(5aR,10bS)-9-[(3-fluorophényl)sulfonyl]-5-méthyl-1,2,3,4,5,5a,6,10b-octahydroazépino[4,5-b]indole ;
(5aR,10bS)-1,5-diméthyl-9-(phénylsulfonyl)-1,2,3,4,5,5a,6,10b-octahydroazépino[4,5-b]indole ;
(5aR,10bS)-2,4-diméthyl-9-(phénylsulfonyl)-1,2,3,4,5,5a,6,10b-octahydroazépino[4,5-b]indole ;
(5aR,10bS)-9-[(4-fluorophényl)sulfonyl]-1,5-diméthyl-1,2,3,4,5,5a,6,10b-octahydroazépino[4,5-b]indole ;
(5aR,10bS)-9-[(4-fluorophényl)sulfonyl]-2,4-diméthyl-1,2,3,4,5,5a,6,10b-octahydroazépino[4,5-b]indole ;
(5aR,10bS)-9-[(3-fluorophényl)sulfonyl]-1,5-diméthyl-1,2,3,4,5,5a,6,10b-octahydroazépino[4,5-b]indole ;
(5aR,10bS)-9-[(3-fluorophényl)sulfonyl]-2,4-diméthyl-1,2,3,4,5,5a,6,10b-octahydroazépino[4,5-b]indole ;
(5aS,10bR)-9-[(4-fluorophényl)sulfonyl]-6-méthyl-1,2,3,4,5,5a,6,10b-octahydroazépino[4,5-b]indole ;
(5aS,10bR)-9-[(3-fluorophényl)sulfonyl]-6-méthyl-1,2,3,4,5,5a,6,10b-octahydroazépino[4,5-b]indole ;
(5aS,10bR)-9-[(3,4-difluorophényl)sulfonyl]-6-méthyl-1,2,3,4,5,5a,6,10b-octahydroazépino[4,5-b]indole ;
(5aS,10bR)-9-[(3,5-difluorophényl)sulfonyl]-6-méthyl-1,2,3,4,5,5a,6,10b-octahydroazépino[4,5-b]indole ;
(5aS,10bR)-6-méthyl-9-[(4-méthylphényl)sulfonyl]-1,2,3,4,5,5a,6,10b-octahydroazépino[4,5-b]indole ;
(5aS,10bR)-6-méthyl-9-[(3-méthylphényl)sulfonyl]-1,2,3,4,5,5a,6,10b-octahydroazépino[4,5-b]indole ;
(5aS,10bR)-9-[(4-méthoxyphényl)sulfonyl]-6-méthyl-1,2,3,4,5,5a,6,10b-octahydroazépino[4,5-b]indole ;
(5aS,10bR)-9-[(3-méthoxyphényl)sulfonyl]-6-méthyl-1,2,3,4,5,5a,6,10b-octahydroazépino[4,5-b]indole ;
(5aS,10bR)-6-méthyl-9-[[(4-trifluorométhyl)phényl]sulfonyl]-1,2,3,4,5,5a,6,10b-octahydroazépino[4,5-b]indole ;
(5aS,10bR)-3,6-diméthyl-9-(phénylsulfonyl)-1,2,3,4,5,5a,6,10b-octahydroazépino[4,5-b]indole ;
(5aS,10bR)-9-[(4-fluorophényl)sulfonyl]-3,6-diméthyl-1,2,3,4,5,5a,6,10b-octahydroazépino[4,5-b]indole ;
(5aS,10bR)-9-[(3-fluorophényl) sulfonyl]-3,6-diméthyl-1,2,3,4,5,5a,6,10b-octahydroazépino[4,5-b]indole ;
(5aS,10bR)-9-[(3,4-difluorophényl)sulfonyl]-3,6-diméthyl-1,2,3,4,5,5a,6,10b-octahydroazépino[4,5-b]indole ;
(5aS,10bR)-9-[(3,5-difluorophényl)sulfonyl]-3,6-diméthyl-1,2,3,4,5,5a,6,10b-octahydroazépino[4,5-b]indole ;
(5aS,10bR)-3,6-diméthyl-9-[(4-méthylphényl)sulfonyl]-1,2,3,4,5,5a,6,10b-octahydroazépino[4,5-b]indole ;
(5aS,10bR)-3,6-diméthyl-9-[(3-méthylphényl)sulfonyl]-1,2,3,4,5,5a,6,10b-octahydroazépino[4,5-b]indole ;
(5aS,10bR)-9-[(4-méthoxyphényl)sulfonyl]-3,6-diméthyl-1,2,3,4,5,5a,6,10b-octahydroazépino[4,5-b]indole ;
(5aS,10bR)-9-[(3-méthoxyphényl)sulfonyl]-3,6-diméthyl-1,2,3,4,5,5a,6,10b-octahydroazépino[4,5-b]indole ;
(5aS,10bR)-3,6-diméthyl-9-[[(4-trifluorométhyl)phényl]-sulfonyl]-1,2,3,4,5,5a,6,10b-octahydroazépino[4,5-b]indole ;
(5aS,10bR)-3-éthyl-6-méthyl-9-(phénylsulfonyl)-1,2,3,4,5,5a,6,10b-octahydroazépino[4,5-b]indole ;
(5aS,10bR)-3-éthyl-9-[(4-fluorophényl)sulfonyl]-6-méthyl-1,2,3,4,5,5a,6,10b-octahydroazépino[4,5-b]indole ;
(5aS,10bR)-3-éthyl-9-[(3-fluorophényl)sulfonyl]-6-méthyl-1,2,3,4,5,5a,6,10b-octahydroazépino[4,5-b]indole ;
(5aS,10bR)-3-éthyl-9-[(3,4-difluorophényl)sulfonyl]-6-méthyl-1,2,3,4,5,5a,6,10b-octahydroazépino[4,5-b]indole ;
(5aS,10bR)-3-éthyl-9-[(3,5-difluorophényl)sulfonyl]-6-méthyl-1,2,3,4,5,5a,6,10b-octahydroazépino[4,5-b]indole ;
(5aS,10bR)-3-éthyl-6-méthyl-9-[(4-méthylphényl)sulfonyl]-1,2,3,4,5,5a,6,10b-octahydroazépino[4,5-b]indole ;
(5aS,10bR)-3-éthyl-6-méthyl-9-[(3-méthylphényl)sulfonyl]-1,2,3,4,5,5a,6,10b-octahydroazépino[4,5-b]indole ;
(5aS,10bR)-3-éthyl-9-[(4-méthoxyphényl)sulfonyl]-6-méthyl-1,2,3,4,5,5a,6,10b-octahydroazépino[4,5-b]indole ;
(5aS,10bR)-3-éthyl-9-[(3-méthoxyphényl)sulfonyl]-6-méthyl-1,2,3,4,5,5a,6,10b-octahydroazépino[4,5-b]indole ;
(5aS,10bR)-3-éthyl-6-méthyl-9-[[(4-trifluorométhyl)phényl]-sulfonyl]-1,2,3,4,5,5a,6,10b-octahydroazépino[4,5-b]indole ;
(5aS,10bR)-1,6-diméthyl-9-(phénylsulfonyl)-1,2,3,4,5,5a,6,10b-octahydroazépino[4,5-b]indole ;
(5aS,10bR)-2,6-diméthyl-9-(phénylsulfonyl)-1,2,3,4,5,5a,6,10b-octahydroazépino[4,5-b]indole ;
(5aS,10bR)-4,6-diméthyl-9-(phénylsulfonyl)-1,2,3,4,5,5a,6,10b-octahydroazépino[4,5-b]indole ;
(5aS,10bR)-5,6-diméthyl-9-(phénylsulfonyl)-1,2,3,4,5,5a,6,10b-octahydroazépino[4,5-b]indole ;
(5aS,10bR)-9-[(4-fluorophényl)sulfonyl]-1,6-diméthyl-1,2,3,4,5,5a,6,10b-octahydroazépino[4,5-b]indole ;
(5aS,10bR)-9-[(4-fluorophényl)sulfonyl]-2,6-diméthyl-1,2,3,4,5,5a,6,10b-octahydroazépino[4,5-b]indole ;
(5aS,10bR)-9-[(4-fluorophényl)sulfonyl]-4,6-diméthyl-1,2,3,4,5,5a,6,10b-octahydroazépino[4,5-b]indole ;
(5aS,10bR)-9-[(4-fluorophényl)sulfonyl]-5,6-diméthyl-1,2,3,4,5,5a,6,10b-octahydroazépino[4,5-b]indole ;
(5aS,10bR)-9-[(3-fluorophényl)sulfonyl]-1,6-diméthyl-1,2,3,4,5,5a,6,10b-octahydroazépino[4,5-b]indole ;
(5aS,10bR)-9-[(3-fluorophényl)sulfonyl]-2,6-diméthyl-1,2,3,4,5,5a,6,10b-octahydroazépino[4,5-b]indole ;
(5aS,10bR)-9-[(3-fluorophényl)sulfonyl]-4,6-diméthyl-1,2,3,4,5,5a,6,10b-octahydroazépino[4,5-b]indole ;
(5aS,10bR)-9-[(3-fluorophényl)sulfonyl]-5,6-diméthyl-1,2,3,4,5,5a,6,10b-octahydroazépino[4,5-b]indole ;
(5aS,10bR)-1,5,6-triméthyl-9-(phénylsulfonyl)-1,2,3,4,5,5a,6,10b-octahydroazépino[4,5-b]indole ;
(5aS,10bR)-2,4,6-triméthyl-9-(phénylsulfonyl)-1,2,3,4,5,5a,6,10b-octahydroazépino[4,5-b]indole ;
(5aS,10bR)-9-[(4-fluorophényl)sulfonyl]-1,5,6-triméthyl-1,2,3,4,5,5a,6,10b-octahydroazépino[4,5-b]indole ;
(5aS,10bR)-9-[(4-fluorophényl)sulfonyl]-2,4,6-triméthyl-1,2,3,4,5,5a,6,10b-octahydroazépino[4,5-b]indole ;
(5aS,10bR)-9-[(3-fluorophényl)sulfonyl]-1,5,6-triméthyl-1,2,3,4,5,5a,6,10b-octahydroazépino[4,5-b]indole ;
(5aS,10bR)-9-[(3-fluorophényl)sulfonyl]-2,4,6-triméthyl-1,2,3,4,5,5a,6,10b-octahydroazépino[4,5-b]indole ;
(5aS,10bR)-6-méthyl-9-(phénylsulfonyl)-1,2,3,4,5,5a,6,10b-octahydroazépino[4,5-b]indole ;
(5aS,10bR)-9-[(4-fluorophényl)sulfonyl]-1,2,3,4,5,5a,6,10b-octahydroazépino[4,5-b]indole ;
(5aS,10bR)-9-[(3-fluorophényl)sulfonyl]-1,2,3,4,5,5a,6,10b-octahydroazépino[4,5-b]indole ;
(5aS,10bR)-9-[(3,4-difluorophényl)sulfonyl]-1,2,3,4,5,5a,6,10b-octahydroazépino[4,5-b]indole ;
(5aS,10bR)-9-[(3,5-difluorophényl)sulfonyl]-1,2,3,4,5,5a,6,10b-octahydroazépino[4,5-b]indole ;
(5aS,10bR)-9-[(4-méthylphényl)sulfonyl]-1,2,3,4,5,5a,6,10b-octahydroazépino[4,5-b]indole ;
(5aS,10bR)-9-[(3-méthylphényl)sulfonyl]-1,2,3,4,5,5a,6,10b-octahydroazépino[4,5-b]indole ;
(5aS,10bR)-9-[(4-méthoxyphényl)sulfonyl]-1,2,3,4,5,5a,6,10b-octahydroazépino[4,5-b]indole ;
(5aS,10bR)-9-[(3-méthoxyphényl)sulfonyl]-1,2,3,4,5,5a,6,10b-octahydroazépino[4,5-b]indole ;
(5aS,10bR)-9-[[(4-trifluorométhyl)phényl]sulfonyl]-1,2,3,4,5,5a,6,10b-octahydroazépino[4,5-b]indole ;
(5aS,10bR)-3-méthyl-9-(phénylsulfonyl)-1,2,3,4,5,5a,6,10b-octahydroazépino[4,5-b]indole ;
(5aS,10bR)-9-[(4-fluorophényl)sulfonyl]-3-méthyl-1,2,3,4,5,5a,6,10b-octahydroazépino[4,5-b]indole ;
(5aS,10bR)-9-[(3-fluorophényl)sulfonyl]-3-méthyl-1,2,3,4,5,5a,6,10b-octahydroazépino[4,5-b]indole ;
(5aS,10bR)-9-[(3,4-difluorophényl)sulfonyl]-3-méthyl-1,2,3,4,5,5a,6,10b-octahydroazépino[4,5-b]indole ;
(5aS,10bR)-9-[(3,5-difluorophényl)sulfonyl]-3-méthyl-1,2,3,4,5,5a,6,10b-octahydroazépino[4,5-b]indole ;
(5aS,10bR)-3-méthyl-9-[(4-méthylphényl)sulfonyl]-1,2,3,4,5,5a,6,10b-octahydraazépino[4,5-b]indole ;
(5aS,10bR)-3-méthyl-9-[(3-méthylphényl)sulfonyl]-1,2,3,4,5,5a,6,10b-octahydroazépino[4,5-b]indole ;
(5aS,10bR)-9-[(4-méthoxyphényl)sulfonyl]-3-méthyl-1,2,3,4,5,5a,6,10b-octahydroazépino[4,5-b]indole ;
(5aS,10bR)-9-[(3-méthoxyphényl)sulfonyl]-3-méthyl-1,2,3,4,5,5a,6,10b-octahydroazépino[4,5-b]indole ;
(5aS,10bR)-3-méthyl-9-[[(4-trifluorométhyl)phényl]sulfonyl]-1,2,3,4,5,5a,6,10b-octahydroazépino[4,5-b]indole ;
(5aS,10bR)-3-éthyl-9-(phénylsulfonyl)-1,2,3,4,5,5a,6,10b-octahydroazépino[4,5-b]indole ;
(5aS,10bR)-3-éthyl-9-[(4-fluorophényl)sulfonyl]-1,2,3,4,5,5a,6,10b-octahydroazépino[4,5-b]indole ;
(5aS,10bR)-3-éthyl-9-[(3-fluorophényl)sulfonyl]-1,2,3,4,5,5a,6,10b-octahydroazépino[4,5-b]indole ;
(5aS,10bR)-3-éthyl-9-[(3,4-difluorophényl)sulfonyl]-1,2,3,4,5,5a,6,10b-octahydroazépino[4,5-b]indole ;
(5aS,10bR)-3-éthyl-9-[(3,5-difluorophényl)sulfonyl]-1,2,3,4,5,5a,6,10b-octahydroazépino[4,5-b]indole ;
(5aS,10bR)-3-éthyl-9-[(4-méthylphényl)sulfonyl]-1,2,3,4,5,5a,6,10b-octahydroazépino[4,5-b]indole ;
(5aS,10bR)-3-éthyl-9-[(3-méthylphényl)sulfonyl]-1,2,3,4,5,5a,6,10b-octahydroazépino[4,5-b]indole ;
(5aS,10bR)-3-éthyl-9-[(4-méthoxyphényl)sulfonyl]-1,2,3,4,5,5a,6,10b-octahydroazépino[4,5-b]indole ;
(5aS,10bR)-3-éthyl-9-[(3-méthoxyphényl)sulfonyl]-1,2,3,4,5,5a,6,10b-octahydroazépino[4,5-b]indole ;
(5aS,10bR)-3-éthyl-9-[[(4-trifluorométhyl)phényl]sulfonyl]-1,2,3,4,5,5a,6,10b-octahydroazépino[4,5-b]indole ;
(5aS,10bR)-1-méthyl-9-(phénylsulfonyl)-1,2,3,4,5,5a,6,10b-octahydroazépino[4,5-b]indole ;
(5aS,10bR)-2-méthyl-9-(phénylsulfonyl)-1,2,3,4,5,5a,6,10b-octahydroazépino[4,5-b]indole ;
(5aS,10bR)-4-méthyl-9-(phénylsulfonyl)-1,2,3,4,5,5a,6,10b-octahydroazépino[4,5-b]indole ;
(5aS,10bR)-5-méthyl-9-(phénylsulfonyl)-1,2,3,4,5,5a,6,10b-octahydroazépino[4,5-b]indole ;
(5aS,10bR)-9-[(4-fluorophényl)sulfonyl]-1-méthyl-1,2,3,4,5,5a,6,10b-octahydroazépino[4,5-b]indole ;
(5aS,10bR)-9-[(4-fluorophényl)sulfonyl]-2-méthyl-1,2,3,4,5,5a,6,10b-octahydroazépino[4,5-b]indole ;
(5aS,10bR)-9-[(4-fluorophényl)sulfonyl]-4-méthyl-1,2,3,4,5,5a,6,10b-octahydroazépino[4,5-b]indole ;
(5aS,10bR)-9-[(4-fluorophényl)sulfonyl]-5-méthyl-1,2,3,4,5,5a,6,10b-octahydroazépino[4,5-b]indole ;
(5aS,10bR)-9-[(3-fluorophényl)sulfonyl]-1-méthyl-1,2,3,4,5,5a,6,10b-octahydroazépino[4,5-b]indole ;
(5aS,10bR)-9-[(3-fluorophényl)sulfonyl]-2-méthyl-1,2,3,4,5,5a,6,10b-octahydroazépino[4,5-b]indole ;
(5aS,10bR)-9-[(3-fluorophényl)sulfonyl]-4-méthyl-1,2,3,4,5,5a,6,10b-octahydroazépino[4,5-b]indole ;
(5aS,10bR)-9-[(3-fluorophényl)sulfonyl]-5-méthyl-1,2,3,4,5,5a,6,10b-octahydroazépino[4,5-b]indole ;
(5aS,10bR)-1,5-diméthyl-9-(phénylsulfonyl)-1,2,3,4,5,5a,6,10b-octahydroazépino[4,5-b]indole ;
(5aS,10bR)-2,4-diméthyl-9-(phénylsulfonyl)-1,2,3,4,5,5a,6,10b-octahydroazépino[4,5-b]indole ;
(5aS,10bR)-9-[(4-fluorophényl)sulfonyl]-1,5-diméthyl-1,2,3,4,5,5a,6,10b-octahydroazépino[4,5-b]indole ;
(5aS,10bR)-9-[(4-fluorophényl)sulfonyl]-2,4-diméthyl-1,2,3,4,5,5a,6,10b-octahydroazépino[4,5-b]indole ;
(5aS,10bR)-9-[(3-fluorophényl)sulfonyl]-1,5-diméthyl-1,2,3,4,5,5a,6,10b-octahydroazépino[4,5-b]indole ;
(5aS,10bR)-9-[(3-fluorophényl)sulfonyl]-2,4-diméthyl-1,2,3,4,5,5a,6,10b-octahydroazépino[4,5-b]indole ;
(5aR,10bR)-9-[(4-fluorophényl)sulfonyl]-6-méthyl-1,2,3,4,5,5a,6,10b-octahydroazépino[4,5-b]indole ;
(5aR,10bR)-9-[(3-fluorophényl)sulfonyl]-6-méthyl-1,2,3,4,5,5a,6,10b-octahydroazépino[4,5-b]indole ;
(5aR,10bR)-9-[(3,4-difluorophényl)sulfonyl]-6-méthyl-1,2,3,4,5,5a,6,10b-octahydroazépino[4,5-b]indole ;
(5aR,10bR)-9-[(3,5-difluorophényl)sulfonyl]-6-méthyl-1,2,3,4,5,5a,6,10b-octahydroazépino[4,5-b]indole ;
(5aR,10bR)-6-méthyl-9-[(4-méthylphényl)sulfonyl]-1,2,3,4,5,5a,6,10b-octahydroazépino[4,5-b]indole ;
(5aR,10bR)-6-méthyl-9-[(3-méthylphényl)sulfonyl]-1,2,3,4,5,5a,6,10b-octahydroazépino[4,5-b]indole ;
(5aR,10bR)-9-[(4-méthoxyphényl)sulfonyl]-6-méthyl-1,2,3,4,5,5a,6,10b-octahydroazépino[4,5-b]indole ;
(5aR,10bR)-9-[(3-méthoxyphényl)sulfonyl]-6-méthyl-1,2,3,4,5,5a,6,10b-octahydroazépino[4,5-b]indole ;
(5aR,10bR)-6-méthyl-9-[[(4-trifluorométhyl)phényl]sulfonyl]-1,2,3,4,5,5a,6,10b-octahydroazépino[4,5-b]indole ;
(5aR,10bR)-3,6-diméthyl-9-(phénylsulfonyl)-1,2,3,4,5,5a,6,10b-octahydroazépino[4,5-b]indole ;
(5aR,10bR)-9-[(4-fluorophényl)sulfonyl]-3,6-diméthyl-1,2,3,4,5,5a,6,10b-octahydroazépino[4,5-b]indole ;
(5aR,10bR)-9-[(3-fluorophényl)sulfonyl]-3,6-diméthyl-1,2,3,4,5,5a,6,10b-octahydroazépino[4,5-b]indole ;
(5aR,10bR)-9-[(3,4-difluorophényl)sulfonyl]-3,6-diméthyl-1,2,3,4,5,5a,6,10b-octahydroazépino[4,5-b]indole ;
(5aR,10bR)-9-[(3,5-difluorophényl)sulfonyl]-3,6-diméthyl-1,2,3,4,5,5a,6,10b-octahydroazépino[4,5-b]indole ;
(5aR,10bR)-3,6-diméthyl-9-[(4-méthylphényl)sulfonyl]-1,2,3,4,5,5a,6,10b-octahydroazépino[4,5-b]indole ;
(5aR,10bR)-3,6-diméthyl-9-[(3-méthylphényl)sulfonyl]-1,2,3,4,5,5a,6,10b-octahydroazépino[4,5-b]indole ;
(5aR,10bR)-9-[(4-méthoxyphényl)sulfonyl]-3,6-diméthyl-1,2,3,4,5,5a,6,10b-octahydroazépino[4,5-b]indole ;
(5aR,10bR)-9-[(3-méthoxyphényl)sulfonyl]-3,6-diméthyl-1,2,3,4,5,5a,6,10b-octahydroazépino[4,5-b]indole ;
(5aR,10bR)-3,6-diméthyl-9-[[(4-trifluorométhyl)phényl]-sulfonyl]-1,2,3,4,5,5a,6,10b-octahydroazépino[4,5-b]indole ;
(5aR,10bR)-3-éthyl-6-méthyl-9-(phénylsulfonyl)-1,2,3,4,5,5a,6,10b-octahydroazépino[4,5-b]indole ;
(5aR,10bR)-3-éthyl-9-[(4-fluorophényl)sulfonyl]-6-méthyl-1,2,3,4,5,5a,6,10b-octahydroazépino[4,5-b]indole ;
(5aR,10bR)-3-éthyl-9-[(3-fluorophényl)sulfonyl]-6-méthyl-1,2,3,4,5,5a,6,10b-octahydroazépino[4,5-b]indole ;
(5aR,10bR)-3-éthyl-9-[(3,4-difluorophényl)sulfonyl]-6-méthyl-1,2,3,4,5,5a,6,10b-octahydroazépino[4,5-b]indole ;
(5aR,10bR)-3-éthyl-9-[(3,5-difluorophényl)sulfonyl]-6-méthyl-1,2,3,4,5,5a,6,10b-octahydroazépino[4,5-b]indole ;
(5aR,10bR)-3-éthyl-6-méthyl-9-[(4-méthylphényl)sulfonyl]-1,2,3,4,5,5a,6,10b-octahydroazépino[4,5-b]indole ;
(5aR,10bR)-3-éthyl-6-méthyl-9-[(3-méthylphényl)sulfonyl]-1,2,3,4,5,5a,6,10b-octahydroazépino[4,5-b]indole ;
(5aR,10bR)-3-éthyl-9-[(4-méthoxyphényl)sulfonyl]-6-méthyl-1,2,3,4,5,5a,6,10b-octahydroazépino[4,5-b]indole ;
(5aR,10bR)-3-éthyl-9-[(3-méthoxyphényl)sulfonyl]-6-méthyl-1,2,3,4,5,5a,6,10b-octahydroazépino[4,5-b]indole ;
(5aR,10bR)-3-éthyl-6-méthyl-9-[[(4-trifluorométhyl)phényl]-sulfonyl]-1,2,3,4,5,5a,6,10b-octahydroazépino[4,5-b]indole ;
(5aR,10bR)-1,6-diméthyl-9-(phénylsulfonyl)-1,2,3,4,5,5a,6,10b-octahydroazépino[4,5-b]indole ;
(5aR,10bR)-2,6-diméthyl-9-(phénylsulfonyl)-1,2,3,4,5,5a,6,10b-octahydroazépino[4,5-b]indole ;
(5aR,10bR)-4,6-diméthyl-9-(phénylsulfonyl)-1,2,3,4,5,5a,6,10b-octahydroazépino[4,5-b]indole ;
(5aR,10bR)-5,6-diméthyl-9-(phénylsulfonyl)-1,2,3,4,5,5a,6,10b-octahydroazépino[4,5-b]indole ;
(5aR,10bR)-9-[(4-fluorophényl)sulfonyl]-1,6-diméthyl-1,2,3,4,5,5a,6,10b-octahydroazépino[4,5-b]indole ;
(5aR,10bR)-9-[(4-fluorophényl)sulfonyl]-2,6-diméthyl-1,2,3,4,5,5a,6,10b-octahydroazépino[4,5-b]indole ;
(5aR,10bR)-9-[(4-fluorophényl)sulfonyl]-4,6-diméthyl-1,2,3,4,5,5a,6,10b-octahydroazépino[4,5-b]indole ;
(5aR,10bR)-9-[(4-fluorophényl)sulfonyl]-5,6-diméthyl-1,2,3,4,5,5a,6,10b-octahydroazépino[4,5-b]indole ;
(5aR,10bR)-9-[(3-fluorophényl)sulfonyl]-1,6-diméthyl-1,2,3,4,5,5a,6,10b-octahydroazépino[4,5-b]indole ;
(5aR,10bR)-9-[(3-fluorophényl)sulfonyl]-2,6-diméthyl-1,2,3,4,5,5a,6,10b-octahydroazépino[4,5-b]indole ;
(5aR,10bR)-9-[(3-fluorophényl)sulfonyl]-4,6-diméthyl-1,2,3,4,5,5a,6,10b-octahydroazépino[4,5-b]indole ;
(5aR,10bR)-9-[(3-fluorophényl)sulfonyl]-5,6-diméthyl-1,2,3,4,5,5a,6,10b-octahydroazépino[4,5-b]indole ;
(5aR,10bR)-1,5,6-triméthyl-9-(phénylsulfonyl)-1,2,3,4,5,5a,6,10b-octahydroazépino[4,5-b]indole ;
(5aR,10bR)-2,4,6-triméthyl-9-(phénylsulfonyl)-1,2,3,4,5,5a,6,10b-octahydroazépino[4,5-b]indole ;
(5aR,10bR)-9-[(4-fluorophényl)sulfonyl]-1,5,6-triméthyl-1,2,3,4,5,5a,6,10b-octahydroazépino[4,5-b]indole ;
(5aR,10bR)-9-[(4-fluorophényl)sulfonyl]-2,4,6-triméthyl-1,2,3,4,5,5a,6,10b-octahydroazépino[4,5-b]indole ;
(5aR,10bR)-9-[(3-fluorophényl)sulfonyl]-1,5,6-triméthyl-1,2,3,4,5,5a,6,10b-octahydroazépino[4,5-b]indole ;
(5aR,10bR)-9-[(3-fluorophényl)sulfonyl]-2,4,6-triméthyl-1,2,3,4,5,5a,6,10b-octahydroazépino[4,5-b]indole ;
(5aR,10bR)-6-méthyl-9-(phénylsulfonyl)-1,2,3,4,5,5a,6,10b-octahydroazépino[4,5-b]indole ;
(5aR,10bR)-9-[(4-fluorophényl)sulfonyl]-1,2,3,4,5,5a,6,10b-octahydroazépino[4,5-b]indole ;
(5aR,10bR)-9-[(3-fluorophényl)sulfonyl]-1,2,3,4,5,5a,6,10b-octahydroazépino[4,5-b]indole ;
(5aR,10bR)-9-[(3,4-difluorophényl)sulfonyl]-1,2,3,4,5,5a,6,10b-octahydroazépino[4,5-b]indole ;
(5aR,10bR)-9-[(3,5-difluorophényl)sulfonyl]-1,2,3,4,5,5a,6,10b-octahydroazépino[4,5-b]indole ;
(5aR,10bR)-9-[(4-méthylphényl)sulfonyl]-1,2,3,4,5,5a,6,10b-octahydroazépino[4,5-b]indole ;
(5aR,10bR)-9-[(3-méthylphényl)sulfonyl]-1,2,3,4,5,5a,6,10b-octahydroazépino[4,5-b]indole ;
(5aR,10bR)-9-[(4-méthoxyphényl)sulfonyl]-1,2,3,4,5,5a,6,10b-octahydroazépino[4,5-b]indole ;
(5aR,10bR)-9-[(3-méthoxyphényl)sulfonyl]-1,2,3,4,5,5a,6,10b-octahydroazépino[4,5-b]indole ;
(5aR,10bR)-9-[[(4-trifluorométhyl)phényl]sulfonyl]-1,2,3,4,5,5a,6,10b-octahydroazépino[4,5-b]indole ;
(5aR,10bR)-3-méthyl-9-(phénylsulfonyl)-1,2,3,4,5,5a,6,10b-octahydroazépino[4,5-b]indole ;
(5aR,10bR)-9-[(4-fluorophényl)sulfonyl]-3-méthyl-1,2,3,4,5,5a,6,10b-octahydroazépino[4,5-b]indole ;
(5aR,10bR)-9-[(3-fluorophényl)sulfonyl]-3-méthyl-1,2,3,4,5,5a,6,10b-octahydroazépino[4,5-b]indole ;
(5aR,10bR)-9-[(3,4-difluorophényl)sulfonyl]-3-méthyl-1,2,3,4,5,5a,6,10b-octahydroazépino[4,5-b]indole ;
(5aR,10bR)-9-[(3,5-difluorophényl)sulfonyl]-3-méthyl-1,2,3,4,5,5a,6,10b-octahydroazépino[4,5-b]indole ;
(5aR,10bR)-3-méthyl-9-[(4-méthylphényl)sulfonyl]-1,2,3,4,5,5a,6,10b-octahydroazépino[4,5-b]indole ;
(5aR,10bR)-3-méthyl-9-[(3-méthylphényl)sulfonyl]-1,2,3,4,5,5a,6,10b-octahydroazépino[4,5-b]indole ;
(5aR,10bR)-9-[(4-méthoxyphényl)sulfonyl]-3-méthyl-1,2,3,4,5,5a,6,10b-octahydroazépino[4,5-b]indole ;
(5aR,10bR)-9-[(3-méthoxyphényl)sulfonyl]-3-méthyl-1,2,3,4,5,5a,6,10b-octahydroazépino[4,5-b]indole ;
(5aR,10bR)-3-méthyl-9-[[(4-trifluorométhyl)phényl]sulfonyl]-1,2,3,4,5,5a,6,10b-octahydroazépino[4,5-b]indole ;
(5aR,10bR)-3-éthyl-9-(phénylsulfonyl)-1,2,3,4,5,5a,6,10b-octahydroazépino[4,5-b]indole ;
(5aR,10bR)-3-éthyl-9-[(4-fluorophényl)sulfonyl]-1,2,3,4,5,5a,6,10b-octahydroazépino[4,5-b]indole ;
(5aR,10bR)-3-éthyl-9-[(3-fluorophényl)sulfonyl]-1,2,3,4,5,5a,6,10b-octahydroazépino[4,5-b]indole ;
(5aR,10bR)-3-éthyl-9-[(3,4-difluorophényl)sulfonyl]-1,2,3,4,5,5a,6,10b-octahydroazépino[4,5-b]indole ;
(5aR,10bR)-3-éthyl-9-[(3,5-difluorophényl)sulfonyl]-1,2,3,4,5,5a,6,10b-octahydroazépino[4,5-b]indole ;
(5aR,10bR)-3-éthyl-9-[(4-méthylphényl)sulfonyl]-1,2,3,4,5,5a,6,10b-octahydroazépino[4,5-b]indole ;
(5aR,10bR)-3-éthyl-9-[(3-méthylphényl)sulfonyl]-1,2,3,4,5,5a,6,10b-octahydroazépino[4,5-b]indole ;
(5aR,10bR)-3-éthyl-9-[(4-méthoxyphényl)sulfonyl]-1,2,3,4,5,5a,6,10b-octahydroazépino[4,5-b]indole ;
(5aR,10bR)-3-éthyl-9-[(3-méthoxyphényl)sulfonyl]-1,2,3,4,5,5a,6,10b-octahydroazépino[4,5-b]indole ;
(5aR,10bR)-3-éthyl-9-[[(4-trifluorométhyl)phényl]sulfonyl]-1,2,3,4,5,5a,6,10b-octahydroazépino[4,5-b]indole ;
(5aR,10bR)-1-méthyl-9-(phénylsulfonyl)-1,2,3,4,5,5a,6,10b-octahydroazépino[4,5-b]indole ;
(5aR,10bR)-2-méthyl-9-(phénylsulfonyl)-1,2,3,4,5,5a,6,10b-octahydroazépino[4,5-b]indole ;
(5aR,10bR)-4-méthyl-9-(phénylsulfonyl)-1,2,3,4,5,5a,6,10b-octahydroazépino[4,5-b]indole ;
(5aR,10bR)-5-méthyl-9-(phénylsulfonyl)-1,2,3,4,5,5a,6,10b-octahydroazépino[4,5-b]indole ;
(5aR,10bR)-9-[(4-fluorophényl)sulfonyl]-1-méthyl-1,2,3,4,5,5a,6,10b-octahydroazépino[4,5-b]indole ;
(5aR,10bR)-9-[(4-fluorophényl)sulfonyl]-2-méthyl-1,2,3,4,5,5a,6,10b-octahydroazépino[4,5-b]indole ;
(5aR,10bR)-9-[(4-fluorophényl)sulfonyl]-4-méthyl-1,2,3,4,5,5a,6,10b-octahydroazépino[4,5-b]indole ;
(5aR,10bR)-9-[(4-fluorophényl)sulfonyl]-5-méthyl-1,2,3,4,5,5a,6,10b-octahydroazépino[4,5-b]indole ;
(5aR,10bR)-9-[(3-fluorophényl)sulfonyl]-1-méthyl-1,2,3,4,5,5a,6,10b-octahydroazépino[4,5-b]indole ;
(5aR,10bR)-9-[(3-fluorophényl)sulfonyl]-2-méthyl-1,2,3,4,5,5a,6,10b-octahydroazépino[4,5-b]indole ;
(5aR,10bR)-9-[(3-fluorophényl)sulfonyl]-4-méthyl-1,2,3,4,5,5a,6,10b-octahydroazépino[4,5-b]indole ;
(5aR,10bR)-9-[(3-fluorophényl)sulfonyl]-5-méthyl-1,2,3,4,5,5a,6,10b-octahydroazépino[4,5-b]indole ;
(5aR,10bR)-1,5-diméthyl-9-(phénylsulfonyl)-1,2,3,4,5,5a,6,10b-octahydroazépino[4,5-b]indole ;
(5aR,10bR)-2,4-diméthyl-9-(phénylsulfonyl)-1,2,3,4,5,5a,6,10b-octahydroazépino[4,5-b]indole ;
(5aR,10bR)-9-[(4-fluorophényl)sulfonyl]-1,5-diméthyl-1,2,3,4,5,5a,6,10b-octahydroazépino[4,5-b]indole ;
(5aR,10bR)-9-[(4-fluorophényl)sulfonyl]-2,4-diméthyl-1,2,3,4,5,5a,6,10b-octahydroazépino[4,5-b]indole ;
(5aR,10bR)-9-[(3-fluorophényl)sulfonyl]-1,5-diméthyl-1,2,3,4,5,5a,6,10b-octahydroazépino[4,5-b]indole ; et
(5aR,10bR)-9-[(3-fluorophényl)sulfonyl]-2,4-diméthyl-1,2,3,4,5,5a,6,10b-octahydroazépino[4,5-b]indole ;.

8. Composé selon la revendication 1, qui comprend un marqueur isotopique.

9. Composé selon la revendication 8, qui est la 6-méthyl-1,2,3,4,5,5a,6,10b-octahydroazépino[4,5-b]indole-9-yl-phényl-sulfone ou la 6-méthyl-1,2,3,4,5,5a,6,10b-octahydroazépino[4,5-b]indole-9-yl-4-fluorophényl-sulfone marquée, ou un de leurs sels pharmaceutiquement acceptables.

10. Composé selon la revendication 8, dans lequel l'isotope est choisi parmi ³H, ¹¹C, ¹⁴C, ¹³N, ¹⁵O, ¹⁸F, ^{99m}Tc, ¹²³I et ¹²⁵I.

11. Composé selon la revendication 10, dans lequel l'isotope est choisi parmi ¹¹C, ¹³N, ¹⁵O et ¹⁸F.

12. Composé selon les revendications 9 et 11, à utiliser dans l'exécution d'une tomographie par émission de positrons.

13. Composé selon la revendication 9, qui est la 6-méthyl-1,2,3,4,5,5a,6,10b-octahydroazépino[4,5-b]indole-9-yl-4-fluorophényl-sulfone ou un de ses sels pharmaceutiquement acceptables, qui contient ¹⁹F.

14. Utilisation d'un composé selon la revendication 1, pour la production d'un médicament destiné au traitement chez un mammifère d'une maladie ou d'une affection où le récepteur de 5-HT est impliqué et une modulation de la fonction de 5-HT est souhaitée.

15. Utilisation selon la revendication 14, dans laquelle la maladie ou l'affection est l'anxiété, la dépression, la schizophrénie, une affection associée au stress, un trouble panique, une phobie, un trouble obsessionnel compulsif, un syndrome de stress post-traumatique, une dépression du système immunitaire, une psychose, une paraphrénie, une manie, un trouble convulsif, un trouble de la personnalité, la céphalée migraineuse, la toxicomanie, l'alcoolisme, l'obésité, un trouble de l'alimentation ou un trouble du sommeil.

16. Utilisation selon la revendication 15, dans laquelle la maladie ou l'affection est l'anxiété, l'obésité, la dépression ou une affection associée au stress.

17. Utilisation selon la revendication 14, dans laquelle la maladie ou l'affection comporte des symptômes psychotiques, affectifs, végétatifs ou psychomoteurs de la schizophrénie, ou les effets secondaires moteurs extrapyramidaux d'autres médicaments antipsychotiques.

18. Utilisation selon la revendication 14, dans laquelle la maladie ou l'affection est l'obésité, la dépression, la schizophrénie, un trouble schizophréniforme, un trouble schizoaffectif, un trouble délirant, une affection associée au stress (par exemple un trouble d'anxiété générale), un trouble panique, une phobie, un trouble obsessionnel compulsif, un syndrome de stress post-traumatique, une dépression du système immunitaire, un problème induit par le stress concernant le système urinaire, gastro-intestinal ou cardio-vasculaire (par exemple l'incontinence urinaire à l'effort), un trouble neurodégénératif, l'autisme, le vomissement provoqué par la chimiothérapie, l'hypertension, la céphalée migraineuse, la céphalée vasculaire de Horton, un dysfonctionnement sexuel, une toxicomanie, un syndrome de manque, un trouble d'adaptation, un trouble de l'apprentissage et mental associé à l'âge, l'anorexie mentale, l'apathie, un problème de déficit d'attention dû à des conditions médicales générales, un trouble d'hyperactivité avec déficit d'attention, un trouble du comportement (y compris de l'agitation dans des conditions associées à une cognition réduite, par exemple en cas de démence, arriération mentale ou délire), un trouble bipolaire, la boulimie nerveuse, le syndrome de fatigue chronique, un trouble de la conduite, un trouble cyclothymique, un trouble dysthymique, la fibromyalgie ou autre trouble somatoforme, un trouble d'anxiété généralisée, un trouble de l'inhalation, un trouble d'intoxication, un trouble du mouvement (par exemple la chorée de Huntington ou la dyskinésie tardive), le trouble oppositionnel avec provocation, la neuropathie périphérique, un trouble de stress post-traumatique, le trouble dysphorique prémenstruel, un trouble psychotique (troubles de brève et longue durée, trouble psychotique dû à une condition médicale, trouble psychotique sans autre spécification), un trouble de l'humeur (trouble bipolaire ou dépressif majeur à caractères psychotiques), le trouble affectif saisonnier, un trouble du sommeil, un trouble du développement spécifique, un trouble d'agitation, une incontinence urinaire à l'effort telle que l'incontinence impérieuse ou mixte, le syndrome d'épuisement de l'inhibition sélective de la recapture de sérotonine (SSRI) ou le syndrome de Tourette.
